(19) Europäisches Patentamt
European Patent Office
Office européen des brevets

(11) EP 2 978 767 B1

(12) # FASCICULE DE BREVET EUROPEEN

(45) Date de publication et mention de la délivrance du brevet:
**03.10.2018 Bulletin 2018/40**

(21) Numéro de dépôt: **14721429.0**

(22) Date de dépôt: **31.03.2014**

(51) Int Cl.:
***C07D 498/04*** (2006.01) ***A61K 8/49*** (2006.01)
***A61Q 5/10*** (2006.01)

(86) Numéro de dépôt international:
**PCT/FR2014/000068**

(87) Numéro de publication internationale:
**WO 2014/154957 (02.10.2014 Gazette 2014/40)**

(54) **PROCEDE DE COLORATION UTILISANT UN PRECURSEUR DE COLORATION CAPILLAIRE ISSU D'IRIDOIDE, COMPOSITION, PRECURSEUR ET DISPOSITIF LE COMPRENANT**

FÄRBUNGSVERFAHREN UNTER VERWENDUNG EINES HAARFÄRBEVERFAHREN VORLÄUFER AUS IRIDOIDEN, ZUSAMMENSETZUNG, VORLÄUFER UND VORRICHTUNG DAMIT

DYEING METHOD USING A HAIR-DYEING PRECURSOR OBTAINED FROM IRIDOIDS, COMPOSITION, PRECURSOR AND DEVICE INCLUDING SAME

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **29.03.2013 FR 1352904**

(43) Date de publication de la demande:
**03.02.2016 Bulletin 2016/05**

(73) Titulaire: **L'OREAL**
**75008 Paris (FR)**

(72) Inventeur: **DAVID, Hervé**
**F-94210 La Varenne Saint Hilaire (FR)**

(74) Mandataire: **Rivière, François Armand**
**L'Oréal**
**Service DIPI**
**9 Rue Pierre Dreyfus**
**92110 Clichy (FR)**

(56) Documents cités:
**EP-A2- 0 251 063** **EP-A2- 0 440 494**
**FR-A1- 2 957 796** **FR-A1- 2 957 797**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 978 767 B1

## Description

La présente invention a pour objet un procédé de coloration mettant en oeuvre une composition comprenant au moins un précurseur de coloration issu d'iridoïde(s) non glycosylé(s), au moins une amine, au moins un composé carbonylé et au moins un agent oxydant chimique, une composition comprenant au moins un précurseur de coloration, un dispositif comprenant lesdits précurseurs, les précurseurs de colorations et une procédé de préparation desdits précurseurs.

Depuis quelques années, on observe un intérêt grandissant pour les composés naturels utilisables en tant que colorant capillaire.

Par exemple, dans la demande EP 440 494, il est décrit un procédé de coloration capillaire mettant en oeuvre une composition comprenant au moins un composé de type (seco)iridoïde-glycoside ou (séco)iridoïde non glycosylé (encore appelé aglycon), extrait de plantes comme les Rubiaceae, Euphorbiaceae, Valerianaceae, Cornaceae, Gentianaceae, Caprifoliaceae, Oleaceae, Ericaceae, Loganiaceae, etc.

Il est notamment connu que les iridoïdes glycosidiques et séco-iridoïdes glycosidiques contenus dans les plantes, à commencer par le géniposide présent dans le genre Gardenia (famille des Rubiaceae) ou l'aucubine présente notamment dans Aucuba japonica (famille des Cornaceae), réagissent avec les amines primaires en présence de β-glucosidases, pour se développer chromatiquement et former des colorants qui ont la vertu d'être bien tolérés par l'organisme et qui sont du reste exploités comme colorants alimentaires et pharmaceutiques (voir par exemple Tetrahedron Letters, pp. 2347-2350 (1969)).

Il existe des applications cosmétiques utilisant ce type d'actifs ou d'extraits naturels, mais qui ne sont pas totalement satisfaisantes. Elles peuvent nécessiter des applications répétées sur plusieurs jours ou plusieurs semaines, du fait d'une faible réactivité des précurseurs iridoïdiques avec les constituants du milieu de teinture.

Le problème rencontré avec de telles colorations est qu'elles sont peu intenses ou nécessitent l'application répétée sur plusieurs jours ou plusieurs semaines de la composition pour obtenir une coloration satisfaisante.

De plus, on peut difficilement atteindre des nuances naturelles, ce qui représente un avantage recherché. Et quand de telles nuances sont atteintes, il n'est pas rare d'observer un virage de couleur important au cours du temps.

La présente invention a donc pour but de remédier aux inconvénients décrits ci-dessus.

En effet, il a été découvert de manière surprenante que l'on pouvait améliorer de manière importante la montée de colorant et la vitesse de coloration à partir de composés de type iridoïde et de ses dérivés, et d'une façon générale tout extrait naturel en contenant, en associant à ce composé, une amine et un troisième composé carbonylé particulier, dans des conditions de pH définies.

On a également constaté que la structure des fibres traitées n'était pas altérée par la coloration selon l'invention.

La présente invention a donc pour premier objet un procédé de coloration des fibres kératiniques de préférence humaines, tels que les cheveux, comprenant l'application sur lesdites fibres :

i) d'au moins une composition **(A)** comprenant au moins un précurseur de coloration biomimétique, composé de formule (**I**) suivante :

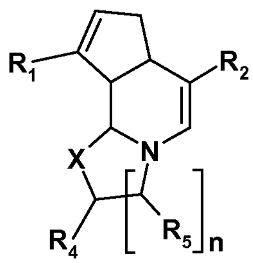

formule **(I)**

composé de formule (**I**) ainsi que ses isomères optiques ou géométriques, ses tautomères, ses sels d'acide ou de base, minéral ou organique, leurs solvates tels que les hydrates ;

formule (**I**) dans laquelle :

- $\mathbf{R_1}$ représente i) un groupe $(C_1-C_6)$alkyle, linéaire ou ramifié, tel que méthyle $-CH_3$, ii) $-(C_1-C_6)$alk-O-H de préférence $-CH_2$-O-H avec $(C_1-C_6)$alk représente un groupe alkylène, linéaire ou ramifié en $C_1-C_6$ ;
- $\mathbf{R_2}$ représente un atome i) d'hydrogène, ii) un groupe -C(O)-R avec R représente un atome d'hydrogène ou un groupe $(C_1-C_4)$alkyle tel que $-C(O)-CH_3$, iii) carboxy -C(O)-OH, iv) $-C(O)-O-(C_1-C_6)$alkyl, v) (di)$(C_1-C_6)$(alkyl)aminocarbonyle tel que $-C(O)-NH_2$ ou $-C(O)-N(H)-(C_1-C_6)$alkyl ; de préférence $-C(O)-O-(C_1-C_6)$alkyl ;
- $\mathbf{R_4}$ représente un atome d'hydrogène, ii) un radical alkyle en $C_1-C_4$ linéaire ou ramifié, iii) un radical benzyle ;

en particulier $R_4$ et $R_5$ sont identiques, de préférence $R_4$ et $R_5$ eprésentent un atome d'hydrogène;

- **$R_5$** identiques ou différents représentent un atome d'hydrogène, ii) un groupe alkyle, linéaire ou ramifié, en $C_1$-$C_6$ éventuellement substitué par un groupe hydroxyle, iii) un radical benzyle, iv) un radical hydroxycarbonyle -C(O)-OH, v) un radical -C(O)-O-($C_1$-$C_{12}$)alkyl, en particulier -C(O)-O-($C_1$-$C_6$)alkyl ;
- **X** représente un atome d'oxygène ;
- **n** vaut 1 ou 2 ; de préférence n vaut 2.

ii) d'au moins une composition **(B)** comprenant au moins un agent oxydant chimique ; les compositions **(A)** et **(B)** pouvant être appliquées simultanément, ou séquentiellement par l'application d'abord de **(A),** puis par l'application de **(B).** De préférence la composition **(A)** est d'abord appliquée puis est appliqué sur les fibres kératiniques la composition **(B),** plus particulièrement la composition **(B)** étant appliquée après un temps de pause d'au moins 5 minutes après application de la composition **(A).**

Un autre objet de l'invention concerne une composition comprenant, dans un milieu cosmétiquement acceptable :

- un ou plusieurs composés de formule **(I)** tel que défini ci après ;
- éventuellement une ou plusieurs amines de formule **(V)** ou de formule **(Va)** à **(Vi')** telles que définies ci-après ;
- éventuellement un ou plusieurs polymères aminés ;
- éventuellement un ou plusieurs composés (thio)carbonylés de formule **(i), (i'), (ii), (a), (b), (c),** et **(d)** tels que définis ci après ; et
- éventuellement un ou plusieurs agents oxydants chimiques tels que définis ci après.

Un autre objet de l'invention concerne un dispositif ou kit, à plusieurs compartiments comprenant :

- dans un premier compartiment au moins un composé de formule **(I)** tel que défini précédemment, de préférence se présentant sous forme de poudre, de préférence anhydre ;
- dans un deuxième compartiment au moins un composé nucléophile choisi parmi les amines primaires naturelles ou non ou polyamine ; de préférence sous forme liquide ; et éventuellement au moins un polymère aminé tel que défini précédemment ;
- dans un troisième compartiment au moins un composé électrophile choisi parmi les composés (thio)carbonylés tels que définis précédemment ; et
- dans un quatrième compartiment au moins un agent oxydant chimique.

Un autre objet de l'invention concerne les composés de formule (**I**) tels que définis précédemment tels que définis ci après.

Un autre objet concerne le procédé de synthèse chimique des composés de de formule (**I**).

La composition et le procédé de coloration selon l'invention permettent d'obtenir des colorations variées, ne dégradant pas les cheveux, tenaces notamment vis-à-vis des shampooings.

Par ailleurs les précurseurs de coloration de formule **(I)**, tels que définis ci aprés sont incolores ou faiblement colorés. Ils se révèlent avec l'utilisation d'amine de formule **(V)** telle que définie ci après, d'un composé (thio)carbonylé de formule **(i), (i'), (ii), (a), (b), (c),** et **(d)** tel que défini ci après et d'un agent oxydant en post-traitement. Il a été possible de réaliser de la coloration « *propre* » en utilisant le procédé de l'invention, ce qui permet d'éviter pour l'utilisateur de tacher les textiles, tissus etc lors de la mise oeuvre dudit procédé de coloration et ce à partir de dérivés issus de produits naturels. Lesdits précurseurs peuvent réagir sur tête à un pH compris entre 7 et 9,5 en présence d'un composé carbonylé et éventuellement d'une amine. La synthèse desdits précurseurs est possible même en milieu tamponné ou à pH neutre.

A l'issue d'un temps de pause supérieur à 5 minutes, l'application d'un agent oxydant chimique (eau oxygénée faiblement dosée par exemple) lors d'une étape de post traitement permet de révéler la couleur sur tête, et permet d'éviter d'altérer les fibres kératiniques.

D'autres caractéristiques et avantages de l'invention apparaîtront plus clairement à la lecture de la description et des exemples qui suivent.

Il est à noter, à moins d'une autre indication, que les bornes des domaines de valeurs donnés dans la description, sont comprises dans les domaines

Les fibres kératiniques humaines traitées par le procédé selon l'invention sont de préférence les cheveux.

Au sens de la présente invention, et à moins qu'une indication différente ne soit donnée :

- le terme « *au moins un », « un ou plusieurs* » est considéré comme synonyme ;
- un radical « *aryle* » représente un groupement carboné mono ou polycyclique, condensé ou non, comprenant

de 6 à 22 atomes de carbone, et dont au moins un cycle est aromatique ; préférentiellement le radical aryle est un phényle, biphényle, naphtyle, indényle, anthracényle, ou tétrahydronaphtyle ;

- un « *radical alkyle* » est un radical hydrocarboné en $C_1$-$C_{12}$, linéaire ou ramifié, de préférence en $C_1$-$C_6$ ;
- un « *radical alkoxy* » est un radical alkyle-oxy pour lequel le radical alkyle est un radical hydrocarboné, linéaire ou ramifié, en $C_1$-$C_{16}$ préférentiellement en $C_1$-$C_8$ ;
- par « *sel d'acide organique ou minéral* » on entend plus particulièrement les sels choisis parmi un sel dérivé i) d'acide chlorhydrique HCl, ii) d'acide bromhydrique HBr, iii) d'acide sulfurique $H_2SO_4$, iv) d'acides alkylsulfoniques : Alk-S(O)$_2$OH tels que d'acide méthylsulfonique et d'acide éthylsulfonique ; v) d'acides arylsulfoniques : Ar-S(O)$_2$OH tel que d'acide benzène sulfonique et d'acide toluène sulfonique ; vi) d'acide citrique ; vii) d'acide succinique ; viii) d'acide tartrique ; ix) d'acide lactique, x) d'acides alkoxysulfiniques : Alk-OS(O)OH tels que d'acide méthoxysulfinique et d'acide éthoxysulfinique ; xi) d'acides aryloxysulfiniques tels que d'acide toluèneoxysulfinique et d'acide phénoxysulfinique ; xii) d'acide phosphorique $H_3PO_4$; xiii) d'acide acétique $CH_3C(O)OH$ ; xiv) d'acide triflique $CF_3SO_3H$ et xv) d'acide tétrafluoroborique $HBF_4$; les sels d'acide minéral sont plus particulièrement les chlorhydrates, les bromhydrates, les sulfates, les phosphates; les sels d'acide organique sont plus particulièrement les citrates, les succinates, les tartrates, les lactates, les tosylates, les benzènesulfonates, et les acétates.
- par « *sel de base organique ou minéral* » on entend les sels choisis parmi un sel dérivé d'agent alcalinisant minéral, ou organique et particulièrement choisis parmi l'ammoniaque, les carbonates ou bicarbonates alcalins tels que les carbonates ou bicarbonates de sodium ou de potassium, les hydroxydes de sodium ou de potassium, les amines organiques choisis parmi les alcanolamines, les éthylènediamines oxyéthylénées et/ou oxypropylénées, les acides aminés et les composés de formule (III) ou leurs mélanges :

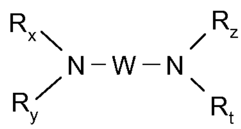
(III)

formule (III) dans laquelle W est un radical divalent alkylène en $C_1$-$C_6$ éventuellement substitué par un ou plusieurs groupements hydroxyle ou un radical alkyle en $C_1$-$C_6$, et/ou éventuellement interrompu par un ou plusieurs hétéroatomes tel que O, ou $NR_u$; $R_x$, $R_y$, $R_z$, $R_t$, et $R_u$, identiques ou différents, représentent un atome d'hydrogène, un radical alkyle en $C_1$-$C_6$ ou hydroxyalkyle en $C_1$-$C_6$, aminoalkyle en $C_1$-$C_6$;

- les solvates sont plus particulièrement des hydrates.

*Précurseurs de coloration de formule (**I'**)* dérivé d'iridoide

Le procédé de coloration des fibres kératiniques et la composition de l'invention mettent en oeuvre un ou plusieurs précurseur(s) de coloration de formule **(I)** tel(s) que défini(s) précédemment.
Selon une variante de l'invention, les composés de formule **(I)** sont tels que n vaut 1.
Selon une autre variante intéressante de l'invention les composés de formule **(I)** sont tels que n vaut 2.
Plus particulièrement les composés de formule **(I)** sont choisis parmi

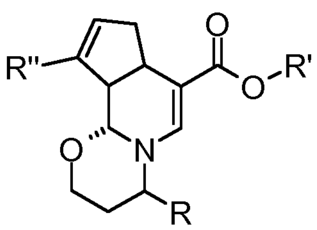
(A)

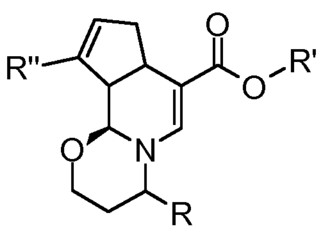
(B)

et

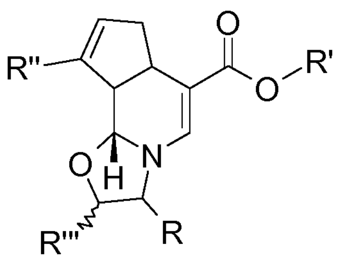
(C)

ainsi que leurs isomères optiques ou géométriques, leurs tautomères, leurs sels d'acide ou de base, minéral ou organique, leurs solvates tels que les hydrates ; Formules **(A), (B)** ou **(C)** dans lesquelles :

- **R** représente un atome d'hydrogène ou un groupe carboxy ;
- **R'** représente un atome d'hydrogène ou un groupe ($C_1$-$C_6$)alkyle ;

- **R"** représente un groupe ($C_1$-$C_6$)alkyle éventuellement substitué par au moins un groupe hydroxy ; et
- **R'''** représente un atome d'hydrogène ou un groupe ($C_1$-$C_6$)alkyle.

De préférence les composés de formule (**I**) sont choisis parmi :

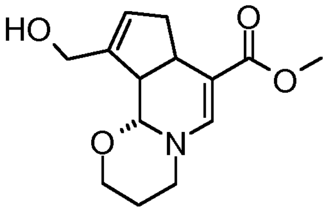

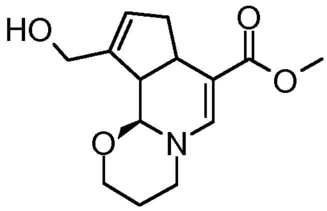

et

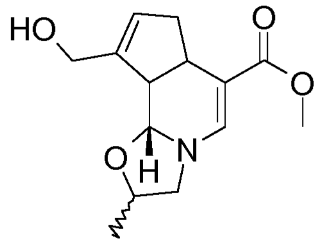

.

*Procédé de préparation des composés de formule (I)*

Particulièrement, le ou lesdits précurseur(s) de formule (**I**) sont biomimétiques.

Le ou lesdits précurseur(s) de formule (**I**) sont obtenus par réaction entre :

- au moins un iridoide non glycosylé de formule **(II)** ou **(IV)** tels que définis ci après, plus particulièrement i'iridoïde non glycosylé est de formule **(II)** ou **(II')** tels que définis ci après ;
- au moins un composé nucléophile choisi parmi les amines de formule **(V)** telles que définies ci après, ou de formule **(Va)** à **(Vi')** telles que définies ci après, de préférence ladite amine est un amino alcool ou aminothiol, et en particulier un β- ou γ- aminoalcool ou aminothiol, plus particulièrement un amino alcool ou aminothiol, et en particulier un β- ou γ- aminoalcool ou aminothiol de formule **(Vf)** ou **(Vf')** tels que définis ci après, et
- et éventuellement au moins un composé électrophile choisi parmi les composés (thio)carbonylés de formule **(i), (i'), (ii), (a), (b), (c),** et **(d)** tels que définis ci après, et de préférence de formule **(i), (i'), (ii), (a)** et **(d),** plus préférentiellement **(i), (i'), (a)** et **(d)** comme suit :

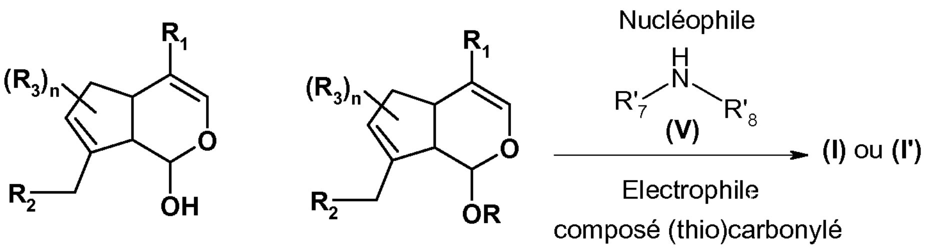


**(II)** **(IV)** **(i), (i'), (ii), (a), (b), (c)** ou **(d)**

L'iridoïde non glycosylé étant de préférence choisi parmi la génipine et/ou l'acide géniposique, ou tout extrait contenant au moins un de ces produits et en particulier l'extrait de *Gardenia jasminoïdes.*

Selon un mode de réalisation particulier du procédé de préparation de (**I**), les composés de départ **(II)** ou **(IV)** tels que définis ci après, sont mis en solution à un pH inférieur ou égal à 7 à l'aide d'une solution tampon, et sont ajoutés un composé nucléophile choisi parmi les amines de formule **(V)** telles que définies ci après, ou de formule **(Va)** à **(Vi')** telles que définies ci après, de préférence de formule **(Vf)** ou **(Vf')** telles que définies ci-après, et un composé électrophile choisi parmi les composés (thio)carbonylés de formule **(i), (i'), (ii), (a), (b), (c),** et **(d)** tels que définis ci après, de préférence de formule **(i), (i')** ou **(ii),** ces ingrédients étant laissés à une température comprise entre 15 °C et 60 °C de préférence entre la température ambiante (25 °C) et 50 °C tel que 40 °C, pendant une durée comprise entre 1 minute et 24 heures, préférentiellement entre 20 minutes et 6 heures, plus particulièrement entre 1 heure et 4 heures telle que 2 heures. Le produit de réaction peut ensuite être purifié par méthodes classiques connues par l'homme du métier tel que par extraction, dans un solvant organique non miscible à l'eau, tel que le dichlorométhane, l'acétate d'éthyle suivi ou non de chromatographie préparative comme sur colonne de silice avec un éluant de type solvant organique halogéné ou non tel que le dichlorométhane ou l'acétate d'éthyle en mélange ou non avec un solvant protique polaire tel que l'éthanol ou le méthanol.

*i/ iridoïde non glycosylé de formule (II)*

Il est à noter que les iridoides mis en oeuvre dans le procédé selon l'invention, sont « *non glycosylés* » i.e. lesdits iridoides ne portent pas de motifs sucre sur l'atome d'oxygène en position pseudo-anomérique (sur l'atome de carbone en alpha de l'atome d'oxygène intracyclique).

De préférence les iridoïdes non glycosylés de formule (**II**) suivante, ainsi que ses isomères optiques ou géométriques, ses sels d'acide minéral ou organique, ses solvates, ou l'extrait végétal en comprenant :

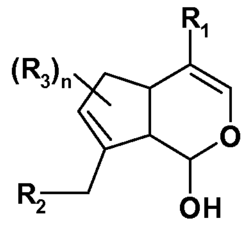

**(II)**

formule (**II**) dans laquelle :

- **$R_1$** représente un atome d'hydrogène, un radical méthyle, un radical hydroxyméthyle, un groupement aldéhyde ; un groupement -C(O)-O-$R_4$ dans lequel $R_4$ représente un atome d'hydrogène ou un radical ($C_1$-$C_6$)alkyle, linéaire ou ramifié de préférence en $C_1$-$C_2$ ; ou un groupement -$CH_2$-glucose ;
- **$R_2$** représente un atome d'hydrogène, un radical hydroxyle, un radical glucose ;
- **$R_3$**, identiques ou non, représentent un atome d'hydrogène, un radical hydroxyle, un radical ($C_1$-$C_4$)alkyloxy ; le nombre de groupement hydroxyle n'étant pas supérieur à 2 ;
- **n** est un entier compris inclusivement entre 1 et 5.

Selon un mode de réalisation particulier de l'invention les extraits naturels contenant les composés de formule (**II**) sont choisis parmi : *Veronica persica; Apodytes dimidiata; Randia canthioides; Tarenna attenuata* ou bien de la famille des iridoïdes déglycosylés de formule (**II**) ou les extraits naturels en contenant choisis parmi *Abelia grandiflora, Adenorandia kalbreyeri, Adina polycephala, Aeginetia indica var. gracilis, Asperula sp, Asystasia bella, Aucuba japonica, Avicennia marina, Bartsia trixago, Buddleja Americana, Buddleja crispa, Buddleja japonica, Canthium schimperianum, Castilleja wightii, Chaenorhinum minus, Clerodendrum serratum, Coprosma sp, Cornus officinalis, Craibiodendron henryi, Cremaspora triflora, Crucianella sp, Daphniphyllum calycinum, Daphniphyllum humile, Daphniphyllum macropodum, Eremostachys glabra, Escallonia sp, Eucommia ulmoides, Feretia apodanthera, Galium humifusum, Galium verum, Gardenia jasminoïdes, Garrya elliptica, Globularia dumulosa, Hedyotis corymbosa, Hygrophila difformis, Ixeris chinensis, Lamiastrum galeobdolon (Lamium galeobdolon), Lamiophlomis rotata (Phlomis rotata), Leonotis nepetaefolia, Linaria sp, Morinda coreia, Mussaenda pubescens, Nepeta cilicia, Nepeta nuda ssp. Albiflora, Odeontites verna, Oldenlandia corymbosa, Paederia scandens, Pedicularis chinensis, Pedicularis condensata, Pedicularis dolichocymba, Penstemon confertus, Penstemon deutus, Penstemon richardsonii, Penstemon serrulatus, Pithecoctenium crucigerum, Plantago alpina, Plantago carinata, Plantago lagopus, Plantago lanceolata, Plantago subulata, Premna barbata, Randia dumetorum, Rhododendron latoucheae, Rothmannia withfieldii, Rubia peregrina, Rubia tinctorum, Saprosma scortechinii, Scrophularia korainensis, Scrophularia lepidota, Scrophularia ningpoensis, Scyphiphora hydrophyllacea, Swida controversa, Syringa vulgaris, Tarenna kotoensis, Tecoma heptaphylla, Thevetia gaumeri, Thevetia peruviana, Verbascum laxum, Verbascum nigrum, Verbascum phlomoides, Verbascum salviifolium, Verbascum sinuatum, Verbascum thapsus, Verbascum undulatum, Veronica derwentiana, Vitex nigrum, Wendlandia formosana,* de préférence *Gardenia jasminoïdes.*

De préférence, les composés de formule (**II**) sont tels que, pris ensembles ou séparement, de préférence pris ensembles :

- **$R_1$** représente un atome d'hydrogène, un radical méthyle, un radical hydroxyméthyle, un groupement aldéhyde ; un groupement hydroxycarbonyle, un groupement méthoxycarbonyle, un groupement éthoxycarbonyle ;
- **$R_2$** représente un atome d'hydrogène, un radical hydroxyle ;
- **$R_3$** identiques ou différents, représentent un atome d'hydrogène, un groupement hydroxyle, un groupement méthoxy, un groupement éthoxy, un groupement n-butyloxy.

Conformément à un mode de réalisation particulièrement avantageux de l'invention, le composé de formule (**II**) est la génipine (avec $R_1$ représentant un groupement méthoxycarbonyle) et/ou l'acide géniposique ou l'un de ses sels (avec $R_1$ représentant un groupement carboxylique sous forme acide ou salifiée).

Les composés de formule **(II)** se trouvent de préférence dans des extraits végétaux provenant des plantes suivantes : *Veronica persica ; Apodytes dimidiata ; Randia canthioides ; Tarenna attenuata.*

A noter que par « *extrait* », on désigne des jus, ou des poudres obtenues par une ou plusieurs opérations de séparation des parties végétales de la plante, d'enrichissement, de concentration et éventuellement de séchage, à partir de substances naturelles végétales.

Ces composés de formule **(II)** sont notamment extraits des végétaux de manière connue en soi.

On pourra plus spécifiquement se reporter au mode opératoire décrit dans la demande internationale WO 2005/105020.

Ainsi, dans le cas des parties aériennes, celles-ci sont lavées si nécessaire, soit broyées, éventuellement sous une forme congelée, soit coupées à température ambiante, puis mises à macération dans un solvant adéquat, en particulier l'éthanol ou l'eau, puis filtrées, concentrées et éventuellement séchées.

Dans le cas plus particulier des fruits, ceux-ci sont éventuellement congelés, lavés à l'eau pour éliminer les impuretés présentes. On peut, éventuellement les stériliser, en particulier avec une solution comprenant de l'éthanol et du chlore.

Pour l'extraction proprement dite, les fruits sont décongelés si nécessaire et pressés par exemple au moyen d'une presse hydraulique spécialement adaptée. Le jus ainsi récupéré est filtré, mis à dégazer en présence d'azote, cette opération évitant l'oxydation de la génipine ou de ses dérivés, en augmentant la quantité de gaz dissout.

Le jus est ensuite conservé dans un emballage hermétique à l'abri de l'air.

Le jus récupéré peut ensuite, le cas échéant, faire l'objet d'une étape de concentration et de séchage.

La teneur en composé de formule **(II)** dans l'extrait sec varie de 0,01 à 30 % en poids.

*ii/ Iridoïdes non glycosylés de formule **(IV)***

selon une autre variante préférée de l'invention les iridoïdes non glycosylés sont de formule **(IV)** suivante,

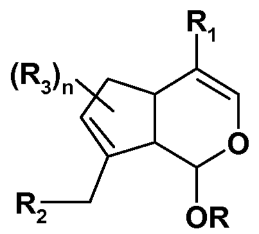

**(IV)**

Formule **(IV)** dans laquelle :

- **$R_1$** représente un radical hydroxyméthyle, un groupement $-C(O)-O-R_4$ dans lequel $R_4$ représente un atome d'hydrogène ou un radical alkyle en $C_1$-$C_2$ ; un radical sucre ;
- **$R_2$** représente un atome d'hydrogène, un radical hydroxyle, un radical sucre ;
- **$R_3$,** identiques ou non, représentent un atome d'hydrogène, un radical hydroxyle, un radical alkyl($C_1$-$C_4$)oxy ; le nombre de groupement hydroxyle n'étant pas supérieur à 2 ;
- **R** représente un radical sucre ;
- **n** est un entier compris entre 1 et 5 ;
- le radical sucre est un dérivé issu d'un aldose ou d'un dérivé d'aldose : ou un extrait végétal comprenant ledit composé de formule (IV), choisi parmi les extraits de plantes suivantes : *Abelia grandiflora, Adenorandia kalbreyeri, Adina polycephala, Aeginetia indica var. gracilis, Asperula sp, Asystasia bella, Aucuba japonica, Avicennia marina, Bartsia trixago, Buddleja Americana, Buddleja crispa, Buddleja japonica, Canthium schimperianum, Castilleja wightii, Chaenorhinum minus, Clerodendrum serratum, Coprosma sp, Cornus officinalis, Craibiodendron henryi, Cremaspora triflora, Crucianella sp, Daphniphyllum calycinum, Daphniphyllum humile, Daphniphyllum macropodum, Eremostachys glabra, Escallonia sp, Eucommia ulmoides, Feretia apodanthera, Galium humifusum, Galium verum, Gardenia jasminoïdes, Garrya elliptica, Globularia dumulosa, Hedyotis corymbosa, Hygrophila difformis, Ixeris chinensis, Lamiastrum galeobdolon (Lamium galeobdolon), Lamiophlomis rotata (Phlomis rotata), Leonotis nepetaefolia, Linaria sp, Morinda coreia, Mussaenda pubescens, Nepeta cilicia, Nepeta nuda ssp. Albiflora, Odeontites*

*verna, Oldenlandia corymbosa, Paederia scandens, Pedicularis chinensis, Pedicularis condensata, Pedicularis dolichocymba, Penstemon confertus, Penstemon deutus, Penstemon richardsonii, Penstemon serrulatus, Pithecoctenium crucigerum, Plantago alpina, Plantago carinata, Plantago lagopus, Plantago lanceolata, Plantago subulata, Premna barbata, Randia dumetorum, Rhododendron latoucheae, Rothmannia withfieldii, Rubia peregrina, Rubia tinctorum, Saprosma scortechinii, Scrophularia korainensis, Scrophularia lepidota, Scrophularia ningpoensis, Scyphiphora hydrophyllacea, Swida controversa, Syringa vulgaris, Tarenna kotoensis, Tecoma heptaphylla, Thevetia gaumeri, Thevetia peruviana, Verbascum laxum, Verbascum nigrum, Verbascum phlomoides, Verbascum salviifolium, Verbascum sinuatum, Verbascum thapsus, Verbascum undulatum, Veronica derwentiana, Vitex nigrum, Wendlandia formosana, de préférence Gardenia jasminoïdes* et leurs mélanges

De préférence, les composés de formule **(IV)** sont tels que, pris ensembles ou séparement :

- **$R_1$** représente un atome d'hydrogène, un radical hydroxyméthyle ; un groupement hydroxycarbonyle, un groupement méthoxycarbonyle, un groupement éthoxycarbonyle ;
- **$R_2$** représente un atome d'hydrogène, un radical hydroxyle ;
- **$R_3$** identiques ou différents, représentent un atome d'hydrogène, un groupement hydroxyle, un groupement méthoxy, un groupement éthoxy, un groupement n-butyloxy ;
- **R** représente un radical issu d'un aldose en $C_6$, comme l'allose, l'altrose, le galactose, le glucose, le gulose, l'idose, le mannose, le talose, et de préférence le glucose. En ce qui concerne les dérivés d'aldose, on préfère les dérivés désoxy, comme le rhamnose, ainsi que les diholosides, en particulier le maltose ; conformément à un mode de réalisation encore plus particulier de l'invention, R représente un radical issu du glucose, du rhamnose, du maltose, et de préférence issu du glucose.

Conformément à un mode de réalisation particulier de l'invention, le composé de formule **(IV)** est le géniposide ($R_1$ représente un groupement methoxycarbonyle) ou l'acide géniposidique ou l'un de ses sels ($R_1$ représente un groupement hydroxycarbonyle sous forme acide ou salifiée).

Les composés de formule **(IV)** se trouvent en général dans des extraits végétaux provenant des plantes suivantes : *Abelia grandiflora, Adenorandia kalbreyeri, Adina polycephala, Aeginetia indica* var. *gracilis, Asperula sp, Asystasia bella, Aucuba japonica, Avicennia marina, Bartsia trixago, Buddleja Americana, Buddleja crispa, Buddleja japonica, Canthium schimperianum, Castilleja wightii, Chaenorhinum minus, Clerodendrum serratum, Coprosma sp, Cornus officinalis, Craibiodendron henryi, Cremaspora triflora, Crucianella sp, Daphniphyllum calycinum, Daphniphyllum humile, Daphniphyllum macropodum, Eremostachys glabra, Escallonia sp, Eucommia ulmoides, Feretia apodanthera, Galium humifusum, Galium verum, Gardenia jasminoïdes, Garrya elliptica, Globularia dumulosa, Hedyotis corymbosa, Hygrophila difformis, Ixeris chinensis, Lamiastrum galeobdolon (Lamium galeobdolon), Lamiophlomis rotata (Phlomis rotata), Leonotis nepetaefolia, Linaria sp, Morinda coreia, Mussaenda pubescens, Nepeta cilicia, Nepeta nuda ssp. Albiflora, Odeontites verna, Oldenlandia corymbosa, Paederia scandens, Pedicularis chinensis, Pedicularis condensata, Pedicularis dolichocymba, Penstemon confertus, Penstemon deutus, Penstemon richardsonii, Penstemon serrulatus, Pithecoctenium crucigerum, Plantago alpina, Plantago carinata, Plantago lagopus, Plantago lanceolata, Plantago subulata, Premna barbata, Randia dumetorum, Rhododendron latoucheae, Rothmannia withfieldii,, Rubia peregrina, Rubia tinctorum, Saprosma scortechinii, Scrophularia korainensis, Scrophularia lepidota, Scrophularia ningpoensis, Scyphiphora hydrophyllacea, Swida controversa, Syringa vulgaris, Tarenna kotoensis, Tecoma heptaphylla, Thevetia gaumeri, Thevetia peruviana, Verbascum laxum, Verbascum nigrum, Verbascum phlomoides, Verbascum salviifolium, Verbascum sinuatum, Verbascum thapsus, Verbascum undulatum, Veronica derwentiana, Vitex nigrum, Wendlandia formosana.*

De préférence, l'extrait végétal mis en oeuvre est le *Gardenia jasminoïdes.*

A noter que par extrait, on désigne des jus, ou des poudres obtenues par une ou plusieurs opérations d'extraction, d'enrichissement, de concentration et éventuellement de séchage, à partir de substances naturelles végétales.

Ces composés de formule **(IV)** sont extraits des végétaux de manière connue en soi.

Ainsi, dans le cas des parties aériennes, celles-ci sont lavées si nécessaire, broyées puis mises à macération dans un solvant adéquat, en particulier l'éthanol ou l'eau, puis filtrées, concentrées et éventuellement séchées.

Dans le cas plus particulier des fruits, ceux-ci sont éventuellement congelés, lavés à l'eau pour éliminer les impuretés présentes. On peut, éventuellement les stériliser, en particulier avec une solution comprenant de l'éthanol et du chlore.

Pour l'extraction proprement dite, les fruits sont décongelés si nécessaire et pressés par exemple au moyen d'une presse hydraulique spécialement adaptée.

Le jus récupéré peut ensuite, le cas échéant, faire l'objet d'une étape de concentration et de séchage.

Dans le cas du *Gardenia Jasminoïdes,* il existe des extraits commerciaux présentant des teneurs variées en composé de formule **(IV).**

La teneur en composé de formule **(IV)** dans l'extrait sec varie de 0,1 à 70 % en poids.

De préférence le composé **(I)** de l'invention est issu de composés de formule **(II)** tel que défini précédemment et en particulier **(II')** ainsi que ses sels d'acide minerai ou organiques, ses isomères optiques et géométriques, ses tautomères et ses solvates :

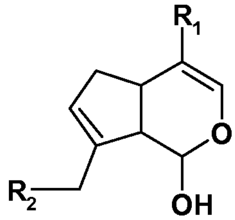
formule **(II')**

formules **(II')** dans laquelle :

- **$R_1$** représente un atome d'hydrogène, un groupement aldéhyde ; un groupement -C(O)-O-$R_4$ dans lequel $R_4$ représente un atome d'hydrogène ou un radical ($C_1$-$C_6$)alkyle, linéaire ou ramifié de préférence en $C_1$-$C_2$ ; de préférence un groupement -C(O)-O-$R_4$ avec $R_4$ tel que défini précédemment ou plus particulièrement avec $R_4$ représentant un atome d'hydrogène, un radical méthyle ;
- **$R_2$** représente un atome d'hydrogène, un radical hydroxyle, un radical glucose ; de préférence un radical hydroxyle.

*Composé nucléophile : les amines*

Selon un mode de réalisation particulier de l'invention le procédé de coloration met également en oeuvre dans la même composition **(A)** ou dans une autre composition au moins un composé nucléophile choisi parmi les amines naturelles ou non.

Conformément à un mode de réalisation de l'invention, une composition mise en oeuvre dans le procédé de coloration peut comprendre au moins une amine primaire, secondaire ou ses sels d'addition, de l'ammoniaque, de l'hydroxylamine, ou leurs mélanges, de préférence au moins une amine primaire ou ses sels d'addition, l'ammoniaque, l'hydroxyamine, ou une polyamine.

D'une manière générale, les sels d'addition de ces composés aminés utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les dodécylbenzènesulfonates, les phosphates et les acétates de préférence les chlorhydrates, les citrates, les succinates, les tartrates, les phosphates, les lactates.

En particulier, la ou les amines primaires ou secondaires, utilisables dans le cadre de l'invention, sont choisies parmi les amines de formule **(V)** qui sera détaillée ci-dessous, les polymères aminés, les bases puriques, ainsi que leurs sels d'addition, et leurs combinaisons.

En particulier, la formule **(V)** est la suivante :

$$R'_7R'_8NH \qquad (V)$$

Formule **(V)** dans laquelle $R'_7$, $R'_8$, représentent indépendamment l'un de l'autre

- un atome d'hydrogène
- un radical hydrocarboné en $C_1$-$C_{20}$, linéaire, ramifié et/ou cyclique, saturé et/ou insaturé, aromatique ou non, pouvant contenir de 1 à 5 doubles liaisons carbone-carbone et/ou éventuellement substitué, éventuellement interrompu par un ou plusieurs hétéroatomes et/ou par un ou plusieurs groupements comprenant au moins un hétéroatome ou groupement comprenant au moins un hétéroatome (de préférence choisi parmi l'oxygène, l'azote, le soufre, C=O, C=S, S(O), $S(O)_2$ ou leurs combinaisons) ; lesdits radicaux $R'_7$ et $R'_8$ hydrocarbonés pouvant éventuellement former avec l'atome d'azote auquel chacun est rattaché, un hétérocycle à 5 ou 7 chaînons, saturé ou insaturé, éventuellement substitué, éventuellement aromatique, éventuellement condensé à un noyau aromatique ou hétéroaromatique à 6 chaînons, comprenant éventuellement un autre hétéroatome identique ou différent de l'azote; le radical hydrocarboné ne comportant pas de fonction nitro, nitroso, peroxo ou diazo.

Les composés de formule **(V)** ne sont avantageusement pas des bases d'oxydation ni des coupleurs d'oxydation, employés dans la coloration des fibres kératiniques.

Parmi les groupements présents comme substituants des groupements hydrocarbonés, hétérocycliques, on

peut citer les groupements :

carboxylique, sulfonique, phosphonique, sous forme acide ou salifiée,
hydroxyle, alcoxy en $C_1$-$C_4$, ($C_1$-$C_8$)alcoxycarbonyle,
($C_1$-$C_4$ )alkylsulfonate, ($C_1$-$C_8$)alkylphosphonate,
trialkyl($C_1$-$C_4$)silyle, trialcoxy($C_1$-$C_4$)silanyle,
amino, (di)($C_1$-$C_4$)alkylamino, tri($C_1$-$C_4$)alkylammonium,
thiol, ($C_1$-$C_4$)alkylthio,
aminosulfonyle, (di-)alkyl($C_1$-$C_4$)aminosulfonyle,
aminocarbonyle, (di-)alkyl($C_1$-$C_4$)aminocarbonyle,
alkyl($C_1$-$C_4$)carbonylamino,
guanidine,
uréido ($R_2$N-C(O)-N(R')-) dans lequel les radicaux R et R', indépendamment les uns des autres, représentent un atome d'hydrogène, un radical alkyle en $C_1$-$C_4$, ($C_1$-$C_4$)alkysulfonylamino ;
phényle, indolyle, pyrolynyle, imidazolyle éventuellement substitué par un ou plusieurs alkyle en $C_1$-$C_2$, hydroxyle.

De préférence, les groupements présents comme substituants sont choisis parmi les groupements carboxylique, sous forme acide ou salifiée ; hydroxyle ; alcoxy en $C_1$-$C_4$, ; ($C_1$-$C_8$)alcoxycarbonyle ; thiol ; ($C_1$-$C_4$)alkylthio ; amino ; mono- et di- ($C_1$-$C_4$)alkylamino ; aminocarbonyle ; mono- et di- ($C_1$-$C_2$)alkylaminocarbonyle ; ($C_1$-$C_4$)alkylcarbonylamino ; phényle, indolyle, pyrrolinyle, imidazolyle éventuellement substitué par un ou plusieurs alkyle en $C_1$-$C_2$, hydroxyle.

En particulier, la ou les amines de formule **(V),** identique(s) ou différente(s), comprennent de une à cinq fonctions amines primaires et/ou secondaires ; le ou les amines ne comportant pas de liaison N-N. Egalement, la ou les amines de formule **(V)** ne comprennent pas plus de deux hétéroatomes liés entre eux.

De préférence, le ou les composés nucléophiles sont des amines de formule **(V),** plus particulièrement choisies parmi les composés de formules **(Va)** à **(Vi)**, **(Vi')** ci-dessous, ainsi que leurs sels d'addition :

◦ **les acides aminés et/ou dérivés de formule générale (Va)** :

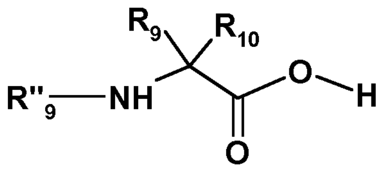

**(Va)**

Formule **(Va)** dans laquelle :

- **$R_9$** représente un atome d'hydrogène un radical alkyle en $C_1$-$C_6$ linéaire ou ramifié, de préférence substitué par un ou plusieurs groupements hydroxyle, hydroxycarbonyle, thiol, ($C_1$-$C_4$)alkylthio, amido, amino, guanidine, un radical phényle, éventuellement substitué par un ou plusieurs hydroxyle, un radical indolyle éventuellement substitué par un ou plusieurs hydroxyle, un radical imidazolyle, un radical pyrrolinyle éventuellement substitué par un groupement alkyle en $C_1$-$C_2$ ; ou un radical phényle non substitué ;
- **R"**$_9$ représente un hydrogène, un radical alkyle en $C_1$-$C_4$, ou un radical phényle non substitué ;
- **$R_{10}$** représente un hydrogène ou un radical alkyle en $C_1$-$C_4$ ;
- **R"**$_9$ et **$R_9$** pouvant former ensemble avec l'atome d'azote auquel ils sont rattachés, un hétérocycle à 5 ou 6 chaînons saturé.

A titre d'exemple de composés de formule **(Va)** et tout particulièrement l'asparagine, la cystéine, la glutamine, l'histidine, la lysine, la méthionine, la phénylalanine, la proline, la pyrrolysine, la sérine, la thréonine, le tryptophane, la tyrosine, et leurs sels d'addition.

Selon une autre variante, les composés de formule **(Va)** sont avanatgeusement choisis parmi l'acide 2-amino-2-méthylpropanoïque ; l'alpha-méthyl-DL-phénylalanine ; la D,L-alpha-(hydroxyméthyl)alanine ; la D,L-alpha-méthyl-métatyrosine ; l'alpha-methyl-D,L-tryptophane; le dichlorhydrate de D,L-alpha-méthylhistidine ; la L-2-méthylsérine ; le

dichlorhydrate de (S)-2-méthylcystéine ; l'acide (S)-2-méthyl-2-pyrrolidine carboxylique.

◦ **les esters issus d'acides aminés et/ou dérivés de formule générale (Vb)** :

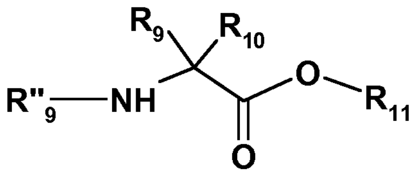

**(Vb)**

formule **(Vb)** dans laquelle :

- **$R_9$** représente un atome d'hydrogène un radical alkyle en $C_1$-$C_6$ linéaire ou ramifié, de préférence substitué par un ou plusieurs groupements hydroxyle, hydroxycarbonyle, ($C_1$-$C_4$)alcoxycarbonyle, thiol, ($C_1$-$C_4$)alkylthio, amido, amino, guanidine, un radical phényle, éventuellement substitué par un ou plusieurs hydroxyle, un radical indolyle éventuellement substitué par un ou plusieurs hydroxyle, un radical imidazolyle, un radical pyrrolinyle éventuellement substitué par un groupement alkyle en $C_1$-$C_2$ ; ou un radical phényle non substitué ;
- **R"**$_9$ représente un hydrogène, un radical alkyle en $C_1$-$C_4$, ou un radical phényle non substitué ;
- **$R_{10}$** représente un hydrogène ou un radical alkyle en $C_1$-$C_4$ ;
- **R"$_9$** et **Rg** pouvant former ensemble avec l'atome d'azote auquel ils sont rattachés, un hétérocycle à 5 ou 6 chaînons saturé ;
- **$R_{11}$** représente :
    - un radical hydrocarboné en $C_1$-$C_{18}$ linéaire ou ramifié, saturé ou insaturé et comprenant éventuellement de une à 5 doubles liaisons carbone-carbone conjuguées ou non, éventuellement substitué comme indiqué précédemment, éventuellement interrompu par un ou plusieurs hétéroatomes et/ou par un ou plusieurs groupements comprenant au moins un hétéroatome, de préférence choisis parmi l'oxygène, l'azote, le soufre, C=O, C=S, S(O), $S(O)_2$ ou leurs combinaisons ; le radical alkyle ne comportant pas de fonction nitro, nitroso, peroxo ou diazo ;
    - un radical benzyle non substitué.

Selon une variante particulière, $R_9$ et $R_{11}$ peuvent éventuellement former un cycle carboné saturé à 5 chaînons.

De préférence $R_{11}$ représente un radical alkyle en $C_1$-$C_{10}$, linéaire ou ramifié éventuellement substitué ; un radical benzyle ; et de façon encore plus préférée, un radical alkyle en $C_1$-$C_4$ linéaire ou ramifié éventuellement substitué par au moins un groupement hydroxyle, de préférence de 1 à 2 groupements hydroxyle ; un radical benzyle.

A titre d'exemples de formule **(Vb)** on peut citer la méthoxytyrosine, le carboxylate d'éthylpipéridine-2 ; l'ester méthylique de la D,L phénylalanine ; le dichlorhydrate de L-cystine diméthylester ; l'ester méthylique de la L-leucine ; l'ester méthylique de l'acide 2-amino-3-methyl-butyrique ; le chlorhydrate de L-phénylalanine éthyl ester ; le chlorhydrate de diéthylester d'acide L-glutamique ; le chlorhydrate de (S) éthyl-2- amino-3- méthylbutanoate ; le chlorhydrate d'ester méthylique de D,L, sérine ; le chlorhydrate d'ester méthylique de tyrosine ; le chlorhydrate d'ester éthylique de L-cystéine ; le chlorhydrate d'ester méthylique de L-histidine ; chlorhydrate d'ester méthylique d'acide (S)pyrrolidine-2 carboxylique ; le bromhydrate de méthyl 2-aminoacetate ; l'éthylglycine ; le chlorhydrate d'ester éthylique de H-DL-alanine ; le chlorhydrate d'ester éthylique de DL tyrosine ; le méthyl-2-(phénylamino)acétate ; l'éthylglutamate ; le chlorhydrate de diester alpha, béta, tertiobutylique d'acide DL aspartique ; le chlorhydrate d'ester éthylique d'acide L-alpha-aminoisocaproïque ; le paratoluène sulfonate de benzylglycinate ; le chlorhydrate d'ester méthylique de DL alanine ; le chlorhydrate d'ester méthylique de 5-hydroxy-DL-tryptophane ; le chlorhydrate d'ester méthylique de DL-thréonine ; l'ester tertiobutylique de DL proline ; le chlorhydrate d'ester méthylique de DL phénylalanine.

◦ **les amides et les thioesters issus d'acides aminés et/ou dérivés de formule générale (Vc)** :

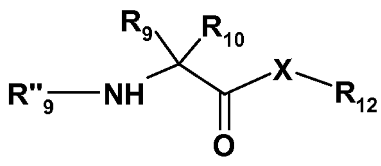

**(Vc)**

Formule **(Vc)** dans laquelle :

- **Rg** représente un atome d'hydrogène, un radical alkyle en $C_1$-$C_6$, linéaire ou ramifié, de préférence substitué par un ou plusieurs groupements hydroxyle, alcoxy($C_1$-$C_4$)carbonyle, hydroxycarbonyle, thiol, ($C_1$-$C_4$)alkylthio, amido, amino, guanidine, un radical phényle, éventuellement substitué par un ou plusieurs hydroxyle, un radical indolyle éventuellement substitué par un ou plusieurs hydroxyle, un radical imidazolyle, un radical pyrrolinyle éventuellement substitué par un groupement alkyle en $C_1$-$C_2$ ;
- **R"**$_9$ représente un hydrogène ou un radical alkyle en $C_1$-$C_4$ éventuellement substitué par un radical hydroxysulfonyle ;
- $\mathbf{R_{10}}$ représente un hydrogène ou un radical alkyle en $C_1$-$C_4$ ;
- **R"**$_9$ et **Rg** pouvant former ensemble avec l'atome d'azote auquel ils sont rattachés, un hétérocycle à 5 chaînons saturé ;
- $\mathbf{R_{12}}$ représente :

  *un atome hydrogène
  * un radical alkyle en $C_1$-$C_6$, de préférence substitué par un ou plusieurs groupements hydroxyle, thiol, ($C_1$-$C_4$)alkylthio, amido, amino, un radical phényle, éventuellement substitué par un ou plusieurs hydroxyle, un radical indolyle éventuellement substitué par un ou plusieurs hydroxyle, un radical imidazolyle, un radical pyrrolinyle éventuellement substitué par un groupement alkyle en $C_1$-$C_2$ ;

- **X** représente un atome de soufre ou d'azote.

Selon une variante particulière, $\mathbf{R_9}$ et $\mathbf{R_{12}}$ peuvent éventuellement former un cycle carboné saturé à 5, 6, 7 chaînons.

Dans le cas ou **X** représente un atome d'azote et $R_{12}$ représente un radical alkyle défini comme précédemment et tout particulièrement un résidu d'acide aminé et/ou leur ester méthylique ou éthylique correspondant choisi parmi l'alanine, l'arginine, l'asparagine, l'aspartate, la cystéine, le glutamate, la glutamine, la glycine, l'histidine, la lysine, la méthionine, la phénylalanine, la proline, la pyrrolysine, la sérine, la thréonine, le tryptophane, la tyrosine, la valine, la leucine, l'isoleucine, le composé de formule **(Vc)** représente un dipeptide, un oligopeptide.

A titre d'exemples de composés de formule **(Vc)** on peut citer le chlorhydrate de 3-amino-dihydrothiophène-2-one ; la thiolactone de DL homocystéine ; la DL-leucyl-DL-alanine, l'aspartame ; la (S)-pyrrolidine 2 carboxamide ; l'acide [N-(-acétamido)] 2-aminoéthane sulfonique ;la DL-Alanyl-DL-phenylalanine; l'acide 2-(2-aminoacetamido)-3-(4-hydroxyphenyl)propanoique ; l'acide 2-(2-aminoacetylamino)-acétique ; le (R)-3-aminoazepane-2-one ; le chlorhydrate de glycinamide ; le chlorhydrate d'amide de L-Leucine ; le 2-aminopropanediamide ; l'acide 2-(2-amino-3-methylbutanamido)-propanoique ; la L-tyrosyl-L-alanine; la L-valyl-L-phenylalanine; le sarcosyl-L-phénylalanine ; le L-tyroyl-béta-alanine ; la glycyl-L-proline ; la glycyl-DL-valine ; la 2-aminomalonamide ; le chlorhydrate de L-methionamide ; la 2-amino-3-methylbutanamide; le chlorhydrate de D-alaninamide ; le bromhydrate de L-tyrosinamide ; l'amide d'acide aspartique ; le chlorhydrate d'amide de L-tyrosine ; le dichlorhydrate d'amide de L-arginine ; le dichlorhydrate d'amide de lysine ; le chlorhydrate d'amide de thréonine ; le chlorhydrate d'amide d'isoleucine ; le dichlorhydrate d'amide d'histidine ; le chlorhydrate d'amide de DL, alanine ; la 2-amino-3-(4-hydroxy-phenyl)-propionamide; le chlorhydrate de DL-triptophanamide ; l'acétate de N-hydroxy-L-arginine (H-ARG-$NH_2$ 2AcOH) ; le chlorhydrate d'amide d'asparagine ; le chlorhydrate de diamide alpha,gamma d'acide L-glutamique ; l'amide de D-phanylalanine ; le chlorhydrate de D-leucinamide ; l'acide L-glutamique alpha-amide; L-methionineamide ; le dichlorhydrate de L-cystine bisamide ; l'acétate de glycinamide ; le dichlorhydrate d'amide de D-lysine ; le glycinamide ; le chlorhydrate de l'ester gamma-méthylique de la L-isoglutamine; le dichlorhydrate d'amide de D-arginine ; le 2 carboxamide de (S) pyrrolidine ; le chlorhydrate de prolyl histamine.

◦ **Des composés aminés de formule générale (Vd)** :

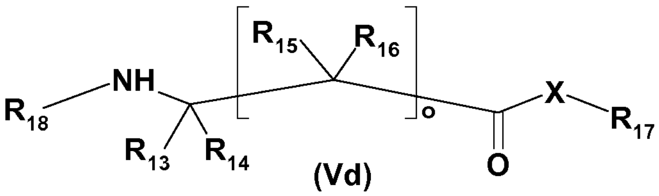

(Vd)

Formule **(Vd)** dans laquelle :

- $\mathbf{R_{13}}$, $\mathbf{R_{14}}$, $\mathbf{R_{15}}$, $\mathbf{R_{16}}$ représentent indépendamment les uns des autres :

  *un atome hydrogène
  *un radical hydrocarboné en $C_1$-$C_{20}$, linéaire, ramifié et/ou cyclique, saturé et/ou insaturé, pouvant contenir de 1 à 5 doubles liaisons carbone-carbone, éventuellement aromatique, éventuellement substitué comme indiqué précédemment, éventuellement interrompu par un ou plusieurs hétéroatomes et/ou par un ou plusieurs groupements comprenant au moins un hétéroatome, de préférence choisis parmi l'oxygène, l'azote, le soufre, C=O, C=S, S(O), $S(O)_2$ ou leurs combinaisons, éventuellement porteurs d'au moins un groupement hydroxyle ou alcoxy en $C_1$-$C_2$, lesdits radicaux alkyle $R_{13}$ et $R_{14}$ ou $R_{14}$ et $R_{15}$ ou $R_{15}$ et $R_{16}$ pouvant éventuellement former avec l'atome de carbone auquel chacun est rattaché, un hétérocycle à 5 ou 7 chaînons, saturé ou insaturé, éventuellement substitué comme indiqué précédemment, éventuellement aromatique, comprenant éventuellement un autre hétéroatome identique ou différent de l'azote; le radical alkyle ne comportant pas de fonction nitro, nitroso, peroxo ou diazo ; plus particulièrement un radical alkyle en $C_1$-$C_{10}$, éventuellement substitué ; et de préférence, un radical alkyle en $C_1$-$C_8$ linéaire ou ramifié éventuellement substitué par au moins un groupement hydroxyle, de préférence de 1 à 2 groupements hydroxyle, un radical hydroxycarbonyle, un radical ureido, un radical alkoxy($C_1$-$C_4$)carbonyle ; un radical phenyle non substitué ;

- **X** représente un atome d'azote, d'oxygène ou de soufre.
- $\mathbf{R_{17}}$ représente :

  *un atome hydrogène
  *un radical hydrocarboné en $C_1$-$C_{18}$ linéaire ou ramifié, saturé ou insaturé et comprenant éventuellement de une à 5 doubles liaisons carbone-carbone conjuguées ou non, éventuellement substitué comme indiqué précédemment, éventuellement interrompu par un ou plusieurs hétéroatomes et/ou par un ou plusieurs groupements comprenant au moins un hétéroatome, de préférence choisis parmi l'oxygène, l'azote, le soufre, C=O, C=S, S(O), $S(O)_2$ ou leurs combinaisons ; le radical alkyle ne comportant pas de fonction nitro, nitroso, peroxo ou diazo ; plus particulièrement $R_{17}$ représente un hydrogène, un radical alkyle en $C_1$-$C_{10}$, linéaire ou ramifié, éventuellement substitué ; et de préférence, un hydrogène, un radical alkyle en $C_1$-$C_4$ linéaire ou ramifié éventuellement substitué par au moins un groupement hydroxyle, de préférence de 1 à 2 groupements hydroxyle ;

- $\mathbf{R_{18}}$ représente :

  *un atome d'hydrogène
  *un radical alkyle en $C_1$-$C_8$ linéaire ou ramifié éventuellement substitué comme indiqué précédemment, éventuellement interrompu par un ou plusieurs hétéroatomes et/ou par un ou plusieurs groupements comprenant au moins un hétéroatome, de préférence choisis parmi l'oxygène, l'azote, le soufre, CO, C=S, SO, $SO_2$ ou leurs combinaisons, éventuellement porteurs d'au moins un groupement hydroxyle ou alcoxy en $C_1$-$C_2$ ; le radical alkyle ne comportant pas de fonction nitro, nitroso, peroxo ou diazo,

- **o** est un entier compris inclusivement entre 0 et 5.

Selon une autre variante de l'invention, les radicaux $R_{16}$ et $R_{17}$ peuvent éventuellement former avec l'atome de carbone pour $R_{16}$ et l'atome X pour le radical $R_{17}$ auquel chacun est rattaché, un hétérocycle à 5 ou 6 chaînons, saturé ou insaturé, éventuellement substitué comme indiqué précédemment, éventuellement aromatique, comprenant éventuellement un autre hétéroatome identique ou différent de l'azote.

Selon une autre variante de l'invention, les radicaux $R_{18}$ et $R_{15}$ peuvent éventuellement former avec l'atome de d'azote pour $R_{18}$ et l'atome de carbone pour le radical $R_{15}$ auquel chacun est rattaché, un hétérocycle à 5 ou 6 chaînons, saturé ou insaturé, éventuellement substitué comme indiqué précédemment, éventuellement aromatique,

comprenant éventuellement un autre hétéroatome identique ou différent de l'azote.

A titre d'exemples de composés de formule (Vd), on peut citer le chlorhydrate d'amide de l'acide L-2-aminohexanoïque ; l'amide de L-phénylalanine ; l'acide (S)(+)aminosuccinique ; l'acide (R)-2-(méthylamino)succinique ; l'éthyl nipecotate ; l'acide carboxylique 3-piperidine ; le 3-phényl-béta-alanine ; l'éthyl-3-aminobutyrate ; la 2-carboyéthylamine ; l'acide DL béta-amino adipique ; le chlorhydrate d'ester éthylique de béta-alanine ; l'ester éthylique d'acide 3-amino-3-ureido-N-butyrique ; le chlorhydrate de diméthyl (S)-aminosuccinate ; le chlorhydrate d'ester méthylique de béta L-alanine ; la 4-carboxyyéthoxypiperidine ; l'acide 4-aminobutyrique ; l'acide DL-béta-amino adipique ; le chlorhydrate de 4-(méthylamino)butyrique ; le chlorhydrate d'éthyl-gamma-aminobutyrate ; l'hexahydronicotinamide ; le 4-carboxamide piperidine ; le 3-carbamoyl-2,2,5,5-tétraméthylpyrrolidine.

◦ **Des composés aminés de formule générale (Ve)** :

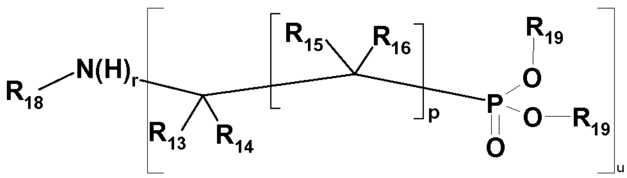

**(Ve)**

Formule **(Ve)** dans laquelle :

- **$R_{13}$, $R_{14}$, $R_{15}$,** $R_{16}$ et **$R_{18}$** ont la même signification que précédemment pour la formule (Vd) ;
- **$R_{19}$** représente :

  *un atome d'hydrogène

  *un radical alkyle en $C_1$-$C_8$ linéaire ou ramifié éventuellement substitué comme indiqué précédemment, éventuellement interrompu par un ou plusieurs hétéroatomes et/ou par un ou plusieurs groupements comprenant au moins un hétéroatome, de préférence choisis parmi l'oxygène, l'azote, le soufre, C=O, C=S, S(O), $S(O)_2$ ou leurs combinaisons, éventuellement porteurs d'au moins un groupement hydroxyle ou alcoxy en $C_1$-$C_2$ ; le radical alkyle ne comportant pas de fonction nitro, nitroso, peroxo ou diazo,

- **p** est un entier compris inclusivement entre 0 et 7 ;
- **u** est un entier égal à 1 ou 2, étant entendu que lorsque u vaut 2 alors le radical $R_{18}$ représente un atome d'hydrogène,
- **r** vaut 0 ou 1, étant étendu que lorsque u vaut 1 alors r vaut 1 et lorsque u vaut 2, alors r vaut 0 ;

Selon une autre variante de l'invention, les radicaux $R_{13}$ et $R_{14}$ peuvent éventuellement former avec l'atome de carbone pour $R_{13}$ et $R_{14}$ auquel ces substituants sont rattachée, un hétérocycle à 5 ou 6 chaînons, saturé.

A titre d'exemples de composés **(Ve)** on peut citer les acides suivants, leurs énantiomères s'ils existent, ainsi que leurs sels, leurs hydrates: (1-aminoéthyl) phosphonique, (aminométhyl)phosphonique, (1-aminoéthyl-1-cyclohexyl)phosphonique, (1-aminopropyl)phosphonique, (1-aminobutyl)-phosphonique, imino-bis(méthylphosphonique), (1-amino-2-méthylpropyl)-phosphonique, (1-amino-2-phényléthyl)phosphonique, (1-amino-1-méthyléthyl)-phosphonique, (1-amino-3-méthylbutyl)phosphonique, 1-amino-benzyl phosphonique, 1-amino hexyl phosphonique, diéthyl(aminoéthyl)phosphonique (en particulier sel oxalate), tétraéthyl(aminométhylène)bisphosphonique (en particulier ses sels), (1-amino-2,2-diméthylpropyl)phosphonique, N-méthyl aminométhyl phosphonique, (1-aminopentyl)phosphonique, (1-amino-2-méthylbutyl)phosphonique, (1-aminooctyl) phosphonique, (1-amino-1-méthylpropyl)phosphonique, (1-amino-1,2-diméthylpropyl) phosphonique, (1-amino-1,3-diméthylbutyl)phosphonique, (1-amino-1-méthylbutyl)phosphonique, (1-amino-1-cyclopentyl)phosphonique, (1-amino-hydroxycarbonyl)propyl phosphonique, (1-amino-1-méthyléthyl)phosphonique, 1-amino-2-méthyl-butyl phosphonique, 1-phosphono-2-phényléthylamine, (aminométhyl) phosphonique, 3-aminopropyl phosphonique, , 2-amino-2-méthyl-4-phosphonobutanoïque et leurs esters éthyliques, (diéthyl(3-aminopropyl)phosphonique (en particulier sel oxalate), , 3-(N-hydroxyamino)propyl phosphonique, , 2-amino-2-méthyl-4-phosphonobutyrique, diéthylester de (3-aminopropyl)phosphonique, 2-amino-4-phosphonobutyrique, 2-aminoéthylphosphonique, 2-amino-3-phosphonopropionique, diéthyl ester de (2-aminoéthyl)phosphonique, diéthyl (2-aminoéthyl)phosphonique, (2-((2-pyrrolidinylcarbonyl)amino)éthyl) phosphonique, le diéthyl ester de (2-amino-1-méthyl-2-phényl)éthyl phosphonique, le diéthyl ester de (2-amino-2-phényl)éthyl phosphonique, e, ou leurs mélanges.

◦ **Des composés aminés de formule générale (Vf) :**

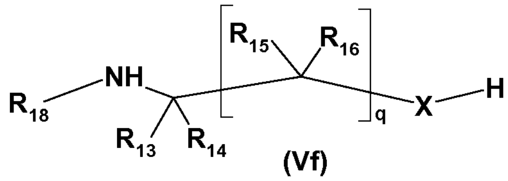

**(Vf)**

Formule **(Vf)** dans laquelle :

- $\mathbf{R_{13}}$, $\mathbf{R_{14}}$, $\mathbf{R_{15}}$, $R_{16}$ et $\mathbf{R_{18}}$ ont la même signification que précédemment ; En outre, les radicaux $\mathbf{R_{13}}$, $\mathbf{R_{14}}$, $\mathbf{R_{15}}$ et $\mathbf{R_{16}}$ indépendamment les uns des autres peuvent aussi représenter un radical hydroxy, un radical $(C_1\text{-}C_4)$alkoxycarbonyle, un radical carboxaldéhyde, un $(C_1\text{-}C_3)$alkoxy ;
- **q** est un entier compris incusivement entre 1 et 18 ;
- **X** représente, un atome d'oxygène, ou de soufre, un groupement méthylène éventuellement substitué par un radical hydroxy ;
- étant entendu que lorsque **X** représente un atome d'oxygène, alors $\mathbf{R_{18}}$ forme un cycle à 5 ou 6 chaînons éventuellement substitué par un ou plusieurs hydroxy(méthyl), de préférence de 1 à 4 groupements hydroxy(méthyle).

Selon une autre variante de l'invention, les radicaux $R_{16}$ et $R_{18}$ ou $R_{13}$ et $R_{18}$ peuvent éventuellement former avec l'atome de carbone pour $R_{16}$ (ou pour $R_{13}$) et l'atome d'azote pour le radical $R_{18}$ auquel chacun est rattaché, un hétérocycle à 5 ou 6 chaînons, saturé ou insaturé, éventuellement substitué comme indiqué précédemment, éventuellement aromatique, comprenant éventuellement un autre hétéroatome identique ou différent de l'azote.

Selon une autre variante de l'invention, l'amine de formule **(Vf)** peut un β-aminoalcool provenant de la réduction de la fonction acide ou ester en alcool d'un des vingt acides aminés estérifiés ou non.

A titre d'exemples de composés **(Vf)** on peut citer les composés suivants, leurs énantiomères s'ils existent, ainsi que leurs sels, leurs hydrates : isopropanolamine, isopropylamine, méthyléthanolamine, méthylglucamine, stéaramine, , tromethamine, prométhazine, 1,3-Diméthylpentylamine, octodrine, spermidine, theanine, octamylamine, 2-amino-1-phényl-propane-1,3-diol, 1,3-dihydroxy-2-amino-2-méthylpropane, 2-amino-2-(hydroxyméthyl)propane-1,3-diol tris, 2-amino-1,3-dihydroxy-2-éthylpropane, 2-amino-3-méthylbutan-1-ol, 2-amino-2-méthylpropan-1-ol, phénylglycinol, alcool 2-aminopropyl, 2-hydroxyéthylamine, 2-aminohexan-1-ol, 1-amino-1-cyclopentaneméthanol, histidinol, 2-amino-3-(3-indolyl)propanol, 3-(4-hydroxyphényl)-2-amino-1-propanol, beta-aminoisobutanol, 2-amino-1-propanol, 2-amino-1,3-propanediol, 2-amino-4-méthyl-1-pentanol, 1-butanol-2-amino-3-méthyl, beta-amino benzènepropanol, 2-aminopropan-1-ol, 2-amino-1-butanol, 2-amino-4-méthylpetan-1-ol, 3-aminopropanethiol, éthyll 2-amino-4-mercaptobutanoate, 6-hydroxyhexylamine, beta-D-galactopyranosylamine, B-D-glucopyranosylamine, 1-amino-2,5-anhydro-D-mannitol, 1-amino-1-deosy-D-fructose, D-glucosamine, 2-pyrrolidinemethanol, 1-amino-2,3-dihydroxypropane, 3-propanolamine, 3-[(2-hydroxyéthyl)amino]propan-1-ol, di-beta-hydroéthylamine, bis(3-hydroxypropyl)amine, N-2'-aminoéthyl-N-propanolamine, ,alcool 4-amino-N-butyl, méthyl 3-amino-3-deoxy-A-D-mannopyranoside, N-butyl-4-hydroxybutylamine, 4-amino-4-(3-hydroxypropyl)-1,7-heptanediol, 1-hexylamine, 1-octylamine, 1-nonylamine, 1-décylamine, laurylamine, 1-tétradécylamine, 1-hexadécylamine, l'acide 3-amino-2-hydroxypropionique, l'acide 3-amino-2-hydroxy-4-phenylbutanoïque, l'acide 4-amino-3-hydroxybutyrique, l'ethyl 4-hydroxy-2-pyrrolidinecarboxylate et leurs mélanges.

◦ **Des composés aminés de formule générale (Vg) :**

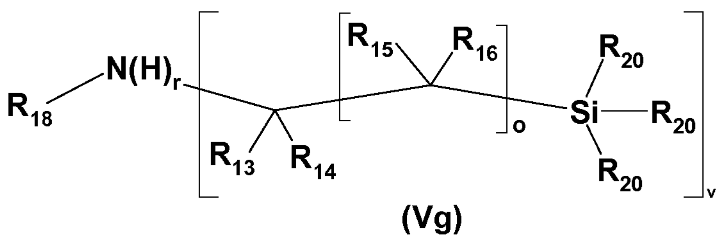

**(Vg)**

Formule (Vg) dans laquelle :

- **$R_{13}$, $R_{14}$, $R_{15}$, $R_{16}$** et **$R_{18}$** ont la même signification que précédemment ;
- **$R_{20}$** représente

  *un radical alkyle linéaire en $C_1$-$C_4$
  *un radical alcoxy linéaire en $C_1$-$C_4$

- **o** est un entier compris inclusivement entre 0 et 5 ;
- **v** est un entier valant 1 ou 2, étant entendu que lorsque v vaut 2 alors $R_{18}$ représente un hydrogène ;
- **r** vaut 0 ou 1, étant étendu que lorsque v vaut 1 alors r vaut 1 et lorsque v vaut 2, alors r vaut 0.

A titre d'exemples de composés **(Vg)** on peut citer les aminopropyl triéthoxysilane, (aminométhyl)triméthylsilane, 2-(triméthylsilyl)éthanamine, 3-(triméthylsilyl)propan-1-amine, 4-(triéthoxysilyl)butan-1-amine, N-[3-(triméthoxysilyl)-propyl]éthylène diamine, 3-(triméthoxysilyl)propylamine, 3-triéthoxysilyl-1-propanamine, (3-méthylaminopropyl) triméthoxysilane, et leurs mélanges.

◦ **Des composés aminés de formule générale (Vh)** :

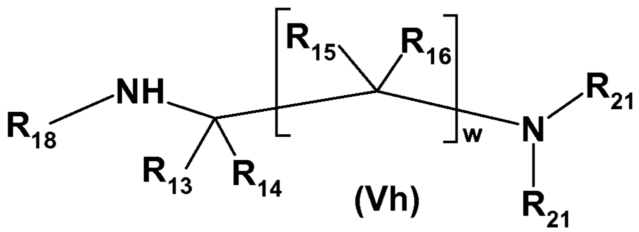

**(Vh)**

Formule **(Vh)** dans laquelle :

- **$R_{13}$, $R_{14}$, $R_{15}$, $R_{16}$** et **$R_{18}$** ont la même signification que précédemment. En outre, les radicaux **$R_{13}$, $R_{14}$, $R_{15}$** et **$R_{16}$** indépendamment les uns des autres peuvent aussi représenter un radical hydroxy, un radical ($C_1$-$C_4$)alcoxycarbonyl, un radical carboxaldéhyde, un radical ($C_1$-$C_3$)alkoxy ;
- **$R_{21}$** et **$R_{22}$** représentent indépendamment l'un de l'autre :

  *un atome d'hydrogène
  *un radical hydrocarboné en $C_1$-$C_{20}$, linéaire, ramifié et/ou cyclique, saturé et/ou insaturé, pouvant contenir de 1 à 5 doubles liaisons carbone-carbone, éventuellement substitué comme indiqué précédemment, éventuellement interrompu par un ou plusieurs hétéroatomes et/ou par un ou plusieurs groupements comprenant au moins un hétéroatome, de préférence choisis parmi l'oxygène, l'azote, le soufre, C=O, C=S, S(O), $S(O)_2$ ou leurs combinaisons, éventuellement porteurs d'au moins un groupement hydroxyle ou alcoxy en $C_1$-$C_2$, plus particulièrement un radical alkyle en $C_1$-$C_{10}$, éventuellement substitué ; et de préférence, un radical alkyle en $C_1$-$C_4$ linéaire ou ramifié éventuellement substitué par au moins un groupement hydroxyle, de préférence de 1 à 2 groupements hydroxyle ;

- **$R_{21}$** et **$R_{22}$** pouvant éventuellement former avec l'atome d'azote auxquels ils sont rattachés, un hétérocycle à 5 ou 7 chaînons, saturé ou insaturé, éventuellement substitué comme indiqué précédemment, éventuellement aromatique, comprenant éventuellement un autre hétéroatome identique ou différent de l'azote; le radical alkyle ne comportant pas de fonction nitro, nitroso, peroxo ou diazo,
- w est un entier compris entre 1 et 10.

Selon une autre variante de l'invention, les radicaux alkyle **$R_{16}$** et **$R_{21}$** peuvent éventuellement former avec l'atome de carbone pour $R_{16}$ et l'atome d'azote pour le radical $R_{21}$ auquel chacun est rattaché, un hétérocycle à 5 ou 6 chaînons, saturé ou insaturé, éventuellement substitué comme indiqué précédemment, éventuellement aromatique, comprenant éventuellement un autre hétéroatome identique ou différent de l'azote.

Selon une autre variante de l'invention, les radicaux alkyle **$R_{18}$** et **$R_{21}$** peuvent éventuellement former avec le

premier atome d'azote pour $R_{18}$ et le dernier atome d'azote pour le radical $R_{21}$ auquel chacun est rattaché, un hétérocycle comprenant de à 5 à 14 chaînons, saturé ou insaturé, éventuellement substitué comme indiqué précédemment, éventuellement aromatique, comprenant éventuellement un autre hétéroatome identique ou différent de l'azote.

Parmi les composés de formule **(Vh)** on peut citer particulier les amines ci-dessous, leurs énantiomères s'ils existent, ainsi que leurs sels, leurs hydrates : gérontine, N-[3-aminopropyl]-1,4-butane-diamine, 1,4-butanediamine, 4-(éthylamino)-N-butylamine, 2-[3-(2-hydroxy-1,1-bis-hydroxyméthyl-éthylamino)-propylamino]-2-hydroxyméthyl-propane-1,3-diol, 1,4,8, 11-tétraazacyclotétradécane, 1,4-diazacycloheptane, 1,3-diamino-2-hdroxypropane, N,N'-bis(2-aminoéthyl)propane-1,3-diamine, 3-méthylamino propylamine, 1,3-bis amino propane, N,N'-diméthyltriméthylènediamine, 2,2-diméthyltriméthylènediamine, 2,2-diméthyl-1,3-diaminopropane, N-(2-hydroxyéthyl)-1,3-diaminopropane, N-(2-hydroxyéthyl)-1,3-diaminopropane, cystamine, 1,5 diaminopentane, 1,6-diaminohexane, lauraminopropylamine, 2-Methylheptylamine (2-(N-methyl)heptylamine), éthylènediamine, N,N-bis (2-hydroxyéthyl)éthylènediamine, 3-amino- alanine, piperazine-2-carboxylic acid, beta-N-methylamino-alanine, l'ester methylique de la piperazine-2-carboxylic acid, ethyl 3-amino- prolinate, l'acide 2,4-diamino-N-butyrique, la N-[3-(triméthoxysilyl)propyl]éthylène diamine ou leurs mélanges.

◦ **Les composés aminés de formule générale (Vi) et/ou (Vi')** :

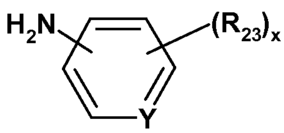

**(Vi)**

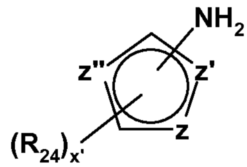

**(Vi')**

Formules **(Vi)** et/ou **(Vi')** dans laquelle :

- **$R_{23}$** et **$R_{24}$** représentent indépendamment l'un de l'autre :

  *un radical alkyle en $C_1$-$C_6$, éventuellement substitué, éventuellement interrompu par un ou plusieurs hétéroatomes et/ou par un ou plusieurs groupements comprenant au moins un hétéroatome, de préférence choisis parmi l'oxygène, l'azote, le soufre, C(O), S(O), $S(O)_2$ ou leurs combinaisons ; le radical alkyle ne comportant pas de fonction nitro, nitroso, peroxo ou diazo ;
  *un radical alkylcarbonyle (R-C(O)-) dans lequel R représente un radical alkyle en $C_1$-$C_4$ ;
  *un radical alkylsulfonyle (R-$S(O)_2$-) dans lequel R représente un radical alkyle en C1-$C_4$;
  *un radical (di-)(alkyl)aminosulfonyle ($R_2N$-$S(O)_2$-) dans lequel les radicaux R indépendamment représentent un hydrogène, un radical alkyle en $C_1$-$C_4$ ;
  *un radical (di-)(alkyl) aminocarbonyle ($R_2N$-C(O)-) dans lequel les radicaux R indépendamment représentent un hydrogène, un radical alkyle en $C_1$-$C_4$ ;
  *un atome d'halogène choisi de préférence parmi le brome, le chlore ou le fluor ;
  *un groupement alcoxy en $C_1$-$C_4$ ;
  *un groupement (poly)hydroxyalcoxy en $C_2$-$C_4$ ;
  *un groupement hydroxycarbonyle ou carboxy (HO-C(O)-)
  *un groupement alcoxycarbonyle (R-O-C(O)-) dans lequel R représente un radical alkyle en $C_1$-$C_4$,
  *un groupement alkylcarbonylamino (R-C(O)-NR'-) dans lequel le radical R représente un radical alkyle en $C_1$-$C_4$ et le radical R' représente un atome d'hydrogène ou un radical alkyle en $C_1$-$C_4$ ;
  *un radical alkylsulfonyle (R-$S(O)_2$-) dans lequel le radical R représente un radical alkyle en $C_1$-$C_4$ ;

- **Y** représente un atome de carbone ou d'azote ;
- **z, z', z"** représente indépendamment les uns des autres un atome de carbone, un atome d'azote ou un atome d'azote substitué par un hydrogène ;
- **x** est un entier compris entre 0 et 2 ; lorsque **x** est inférieur à 2, le ou les atomes de carbone non substitués porte(nt) un atome d'hydrogène ;
- **x'** est un entier égale à 0 ou 1 ; lorsque **x'** est inférieur à 1, le ou les atomes de carbone non substitués porte(nt) un atome d'hydrogène.

Parmi les composés de formule **(Vi)** et/ou **(Vi')**, on peut citer en particulier les composés listés ci-dessous, leurs énantiomères s'ils existent, ainsi que leurs sels, leurs hydrates alpha-pyridylamine, 2-amino-3-hydroxypyridine, acide 2 amino-nicotinique, 2-amino-3-méthylpyridine, 6-méthoxy-3-pyridylamine, 3-aminopyridine, 3-amino-4-pyridinylamine, 2,5-aminopyridine, gamma-pyridylamine, 2,3-diméthylpyridine-4-amine, acide amino(4-) salicylique, méthylpara-amino benzoate, benzocaïne, acide aminobenzoïque, acide 4-amino-M-anisique, acide 4-amino-3-hydroxy-benzoïque, ester méthylique de l'acide 3,4-diaminobenzoïque, acide méthyl 4-amino-3-méthoxybenzène carboxylique, acide 2-aminoanisole-4-carboxylique, l'acide 3-amino-4-hydroxyenzoïque, l'ester éthylique de l'acide 3-aminobenzoïque, 1-amino-3-carboxybenzène, l'ester méthylique de l'acide 2-aminobenzoïque, l'anthranylate d'éthyle, 1H-pyrazol-3-ylamine, 3-amino-4-carbéthoxy-1H-pyrazole, 5-amino-1-éthyl-pyrazole, 1H-benzoimidazol-2-amine, 2-imidazolamine, 1-méthylbenzoimidazol-2-amine,, et leurs mélanges.

On peut également utiliser les amines suivantes, sous forme de sels ou non, lauroyl Ethylenediamine, octopamine, oléamine, palmitamine, 2-(2-aminoéthoxy)éthanol, 2-amino-4,5-diméthylthiazole, hexetidine, mécamylamine, tranylcypromine, triamterene, méthyl[2-(3-triméthoxysilyllpropyl amino)-éthylamino], bis(triéthoxysilylpropyl)amine, N1-(3-(triméthoxysilyl)propyl)hexane-1,6-diamine, diéthylène triaminopropyl triméthoxy silane, N-(3-triéthoxysilylpropyl)éthylène diamine, N-(3-triméthoxysilyléthyl)éthylènediamine.

Selon une autre variante de l'invention, le composé nucléophile est choisi parmi les polymères aminés, ainsi que leurs sels d'addition.

On entend par polymère aminé des macromolécules de poids moléculaire plus ou moins élevé possédant une ou plusieurs fonctions amines primaires ou secondaires. Par « *polymère* », on entend un composé comportant au moins 5 motifs de répétition enchaînés par des liaisons covalentes.

Le polymère aminé peut être synthétisé :

- par des réactions radicalaires (polyacrylates, polyméthacrylates, polyvinyles...),
- par des réactions de condensation (polyesters, polyéthers, polyamides, polyuréthanes, polydiméthylsiloxanes, polypeptides...)
- par des réactions d'ouverture de cycle (polyesters ...).

Il peut être d'origine naturelle, modifié chimiquement ou non, comme par exemple les polysaccharides (cellulose, dextrane, chitosane, guar et leurs dérivés aminés ou thiolés,

Les polymères peuvent se présenter sous tout type de topologie : chaîne linéaire, ramifiée, en étoile ou hyperbranchée (comme les dendrimères par exemple), chaînes séquencées, statistiques ou alternées.

Les groupements chimiques peuvent être naturellement présents sur la chaîne polymérique, en bout de chaîne, greffés le long de la chaîne principale ou des chaînes secondaires, sur les branches des polymères en étoile ou hyperbranchés.

On préfère tout particulièrement :

1/ les polyacides aminés présentant des groupes hydroxy OH, amino $NH_2$, thiol SH ou carboxy C(O)-OH libres, par exemple la polylysine ;

2/les polysaccharides naturels ou modifiés présentant des fonctions $NH_2$ ou SH ;

3/ les silicones aminées ;

4/ les polymères synthétiques à fonctions $NH_2$ ou SH en particulier les polyvinyliques vinyliques substitués par une fonction amine et les polymères réalisés à partir des monomères commerciaux, ainsi que leur sels d'acide minéraux ou organiques, ci-après :

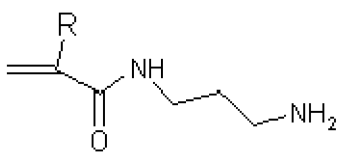
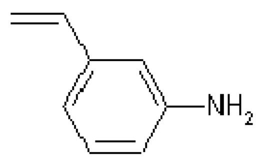

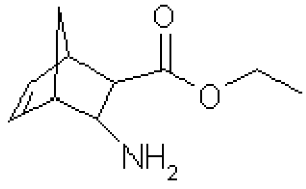
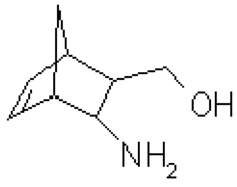
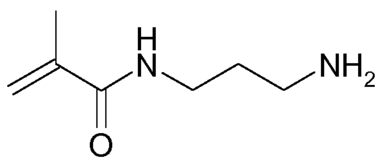

et en particulier parmi la polylysine ; le chitosan ; les amines polyéthoxylées, telles que les carboxyPEG8 amine,

carboxyPEG12 amine, carboxyPEG24 aminé ; ou leurs combinaisons.

Selon une autre variante de l'invention, la ou les amines sont choisies parmi les bases puriques, en particulier choisies parmi l'adénine, l'adénosine, la guanine, la guanosine G, la thymine, la thymidine T, l'uracile, l'uridine U, la cytosine, la cytidine C, leurs sels d'additions, et leurs combinaisons.
On ne sortirait pas du cadre de l'invention en combinant plusieurs de ces variantes.
De préférence, si la composition ou le procédé met en oeuvre une ou plusieurs amines ces dernières sont choisies parmi l'ammoniaque, les composés des formules **(Va), (Vb), (Vc), (Ve), (Vf), (Vg)** en particulier lorsque $\mathbf{R_{20}}$ représente un groupement alcoxy linéaire en $C_1$-$C_4$, **(Vi'),** ou leurs mélanges.
Plus particulièrement a composition comprend une ou plusieurs amines primaires ou secondaires, l'ammoniaque, ou les hydroxylamines, tels que définis précédemment et leur teneur représentent dans la composition entre 0,001 et 65 % en poids ; et de préférence entre 0,001 et 30 % en poids, par rapport au poids de la composition.
Selon un mode particulièrement préféré de l'invention le ou les dits précurseur(s) de formule **(I)** sont obtenus par réaction entre :

- au moins un iridoide non glycosylé de formule **(II)** ou **(IV)** tels que définis précédemment, plus particulièrement l'iridoïde non glycosylé est de formule **(II)** ou **(II')** tels que définis précédemment ; et
- au moins un composé nucléophile choisi parmi les amines de formule **(V)** telles que définies ci après, ou de formule **(Va)** à **(Vi')** telles que définies ci après, de préférence ladite amine est un amino alcool ou aminothiol, et en particulier un β- ou γ- aminoalcool ou aminothiol, plus particulièrement un amino alcool ou aminothiol, et en particulier un β- ou γ- aminoalcool ou aminothiol de formule **(Vf)** ou **(Vf')** tels que définis ci après, et plus particulièrement un amino alcool ou aminothiol, et en particulier un β- ou γ- aminoalcool ou aminothiol de formule **(Vf')** définis ci-dessous

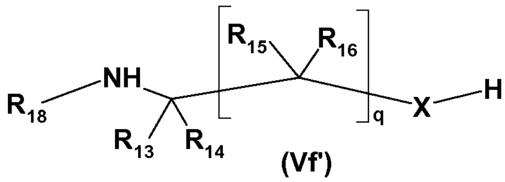

**(Vf')**

Formule **(Vf')** pour lesquels :

- $\mathbf{R_{13}}$ et $\mathbf{R_{14}}$ identiques ou différents représentent un atome d'hydrogène, ii) un groupe alkyle, linéaire ou ramifié, en $C_1$-$C_6$ éventuellement substitué par un groupe hydroxyle,, iii) un radical benzyle, iv) un radical hydroxycarbonyle -C(O)OH, v) un radical -C(O)-O-($C_1$-$C_{12}$)alkyl.
- $\mathbf{R_{15}}$ et $\mathbf{R_{16}}$ indépendamment l'un de l'autre représentent ii) un radical alkyle en $C_1$-$C_4$ linéaire ou ramifié, iii) un radical benzyle.
- $\mathbf{R_{18}}$ représente un atome d'hydrogène ;
- **X** représente un atome d'oxygène, ou de soufre, un radical -NH-, de préférence X représente un atome d'oxygène ;
- **q** est tel que défini précédemment, en particulier q est un entier compris incusivement entre 1 et 10, plus particulièrement entre 1 et 5, préférentiellement 1 ou 2.

Plus préférentiellement le ou les dits précurseur(s) de formule **(I)** sont obtenus par réaction entre au moins un iridoide non glycosylé de formule **(II)** ou **(IV)** tels que définis précédemment, plus particulièrement l'iridoïde non glycosylé est de formule **(II)** ou **(II')** tels que définis précédemment ; et au moins un β- ou γ-aminoalcool de formule **(Vf")** ainsi que ses sels d'acide minéral ou organique, ses solvates et ses isomères optiques :

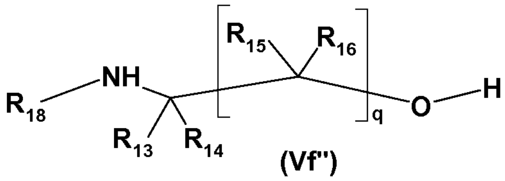

**(Vf")**

formule **(Vf")** pour lesquels :

- $\mathbf{R_{13}}$, $\mathbf{R_{14}}$, $\mathbf{R_{15}}$ et $\mathbf{R_{16}}$ représentent un atome d'hydrogène ou un groupe ($C_1$-$C_4$)alkyle, de préférence un atome

d'hydrogène ;

- **$R_{18}$** représente un atome d'hydrogène ou un groupe ($C_1$-$C_6$)alkyle, de préférence **$R_{18}$** représente un atome d'hydrogène ; et
- **q** est tel que défini précédemment, en particulier q est un entier compris incusivement entre 1 et 10, plus particulièrement entre 1 et 5, préférentiellement entre 1 et 3, tel que 2.

*Composé électrophile : les dérivés (thio)carbonylés*

Selon un mode de réalisation particulier le procédé de coloration met également en oeuvre dans la même composition **(A)** ou dans une autre composition :

- au moins un composé électrophile choisi parmi les composés (thio)carbonylés de préférence carbonylés en particulier choisi parmi les composés aldéhydiques, iminiques, naturels ou synthétiques, (hétéro)aromatique ou non, les composés nitriles, cétoniques, dicétoniques, les hydroxycétones.

Selon un mode préféré de l'invention le ou les composés électrophiles sont choisis parmi *les composés aldéhydiques ou iminiques* et de préférence choisi parmi les composés de formules suivantes **(i)** et **(ii)** ci-dessous, leurs isomères optiques ou géométriques, leurs sels d'acide organique ou minéral, leurs solvates, ainsi que les oligo- ou polysaccharides oxydés comprenant au moins une fonction aldéhyde ou imine :

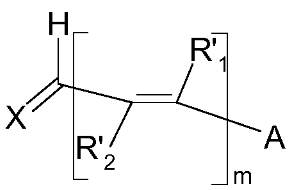

**(i)**

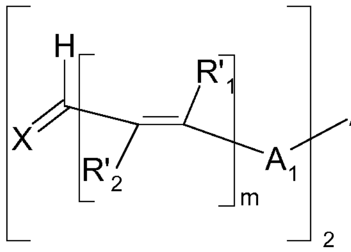

**(ii)**

formules **(i)** et **(ii)** dans lesquelles :

- **m** est un entier compris entre 0 et 2, de préférence valant 0 ou 1 ;
- **X** représente un atome d'oxygène, de soufre, NR"$_1$ avec R"$_1$ représentant un atome d'hydrogène, un radical alkyle en $C_1$-$C_{10}$ ;
- lorsque **X** représente un atome d'oxygène ou de soufre, de préférence X = O, les composés peuvent se trouver également :

  ◦ sous la forme de (thio)acétal de préférence acétal, cyclique à 5 ou 6 chaînons ou acyclique résultant de la condensation d'un monoalcool primaire additionnel (R'$_3$OH) dans lequel R'$_3$ représente un radical alkyle en $C_1$-$C_5$ ou un diol 1,2 ou 1,3 symétrique présentant une chaîne alkyle en $C_2$-$C_3$ ;
  ◦ sous la forme de (thio)hémiacétal de préférence acétal, résultant de la condensation d'un groupement hydroxyle présent sur A ou $A_1$ lorsque A ou $A_1$ représente un radical alkyle et lorsque n vaut 0 ;

- **R'$_1$** et **R'$_2$**, indépendamment l'un de l'autre, représentent un atome d'hydrogène, un radical alkyle en $C_1$-$C_6$ linéaire non substitué ;
- **A** représente un radical monovalent et **$A_1$** représente un radical divalent choisi parmi :

  * un groupement hydroxycarbonyle (-C(O)-OH) :
  * un groupement aryle de préférence en $C_6$ éventuellement substitué par au moins un groupe choisi parmi :

    - un groupement hydroxyle,
    - un radical alkyle en $C_1$-$C_4$,
    - un radical aryle-éthylényle, le groupement aryle étant en $C_6$ et éventuellement substitué par au moins un radical alkyle en $C_1$-$C_2$, alcoxy en $C_1$-$C_2$,
    - un groupement -C(O)-OR'$_4$ ou -O-C(O)-R'$_4$ où R'$_4$ représente un radical alkyle en $C_1$-$C_2$, un groupement

phényle ;

- un groupement -C(O)-OH, sous forme acide ou salifiée,
- un groupement -OR'$_5$ où R'$_5$ représente un radical alkyle en $C_1$-$C_8$ éventuellement substitué par au moins un groupement hydroxyle, un groupement ammonium -$N^+R''_6$ avec R"$_6$, identiques ou non, représentant un radical alkyle en $C_1$-$C_2$, un groupement -SiR'$_7$ avec R'$_7$, identiques ou non, représentant un radical alkyle en $C_1$-$C_2$ ; un radical benzyle (-$CH_2$-phényle),
- selon une variante particulière, deux radicaux -OR'$_5$ situés en ortho du groupement aryle en $C_6$ peuvent former un hétérocycle à 5 ou 6 chaînons par l'intermédiaire des deux radicaux R'$_5$ ;
- un groupement -N(R'$_8$)$_2$ où R'$_8$, identiques ou non, représentent un radical alkyle en $C_1$-$C_6$ éventuellement substitué par un groupement hydroxyle, carboxylique (-C(O)-OH) sous forme acide ou salifiée, sulfonique (-$SO_3H$) sous forme acide ou salifiée ; les radicaux R'$_8$ pouvant éventuellement former un hétérocycle à 5 ou 6 chaînons avec l'atome d'azote qui les porte, saturé ou non, comprenant éventuellement un autre hétéroatome choisis parmi O, N, NR'$_9$ où R'$_9$ représente un radical alkyle en $C_1$-$C_2$; l'hétérocycle étant éventuellement substitué par un groupement hydroxyle,
- un groupement -COR'$_{10}$ où R'$_{10}$ représente un groupement aryle en $C_6$ condensé à un cycle hydrocarboné à 6 chaînons, éventuellement substitué par au moins un radical alkyle en $C_1$-$C_2$,
- un -SR'$_{11}$ où R'$_{11}$ représente un radical alkyle en $C_1$-$C_2$,
- un atome d'halogène choisi de préférence le chlore, le brome,
- un groupement -O-S(O)$_2$-phényle,
- un groupement -$SO_3H$,
- un hétérocycle à 5 ou 6 chaînons insaturé, cationique ou non cationique, comprenant un ou deux hétéroatomes, choisis parmi O, N, NR'$_{12}$ où R'$_{12}$ représente un groupement alkyle en $C_1$-$C_2$, éventuellement condensé à un cycle à 5 ou 6 chaînons, saturé ou non, aromatique ou non, l'un des hétéroatomes pouvant éventuellement être inclus dans les deux cycles ; l'hétérocycle ou le cycle condensé pouvant être substitué par au moins un radical alcoxy en $C_1$-$C_2$,
- ledit groupement aryle étant éventuellement condensé à un groupement hétérocyclique à 5 ou 6 chaînons, comprenant un ou deux hétéroatomes choisi parmi O, N, NR'$_{13}$ où R'$_{13}$ représente un radical alkyle en $C_1$-$C_4$, l'hétérocycle étant éventuellement condensé à un groupement aryle en $C_6$,
- ledit groupement aryle étant éventuellement condensé à un groupement aryle en $C_6$ éventuellement substitué par au moins un groupement alcoxy en $C_1$-$C_2$ ;

* un groupement hétérocyclique à 5 ou 6 chaînons, cationique ou non cationique, insaturé ou non, aromatique ou non, comprenant un ou deux hétéroatomes, identiques ou non, choisis de préférence parmi O, N, NR'$_{14}$ avec R'$_{14}$ représentant un atome d'hydrogène, un radical alkyle en $C_1$-$C_6$ ou aryle en $C_6$ éventuellement substitué par un groupement (R'$_{15}$)$_2$N-C(O)- ou R'$_{15}$-C(O)-N(H)- où R'$_{15}$, identiques ou non représentent un radical alkyle en $C_1$-$C_2$;

  - ledit groupement hétérocyclique étant éventuellement condensé à un groupement aryle à 6 chaînons lui-même éventuellement substitué par au moins un groupement alkyle en $C_1$-$C_2$, alcoxy en $C_1$-$C_4$ ; phénoxy;
  - ledit groupement hétérocyclique étant éventuellement substitué par au moins :

    ◦ un groupement hydroxyle,
    ◦ un radical alkyle en $C_1$-$C_2$ éventuellement substitué par un radical hydroxyle
    ◦ un radical amino -N(R'$_{16}$)$_2$, où R'$_{16}$, identiques ou non, représentent un radical alkyle en $C_1$-$C_4$ éventuellement substitué par un groupement hydroxyle, les radicaux R'$_{16}$ pouvant éventuellement former un hétérocycle à 5 ou 6 chaînons avec l'atome d'azote qui les porte, saturé ou non, comprenant éventuellement un autre hétéroatome choisis parmi O, N, NR'$_{17}$ où R'$_{17}$ représente un radical alkyle en $C_1$-$C_2$,
    ◦ un radical aryle en $C_6$ éventuellement substitué par au moins un groupement carboxy -C(O)-OH, amido R'$_{18}$-C(O)-N(H)- avec R'$_{18}$ représentant un radical alkyle en $C_1$-$C_2$ ; ledit radical aryle en $C_6$ étant éventuellement relié à un atome d'azote du groupement hétérocyclique ;

  étant entendu que lorsque le radical aryle n'est pas substitué par un radical précédemment cité, les atomes de carbone le sont par un atome d'hydrogène ;

* un radical alkyle en $C_1$-$C_{10}$ linéaire ou ramifié, ou un radical alcényle en $C_2$-$C_{10}$, comprenant une ou plusieurs insaturations carbone-carbone, conjuguées entre elles ou non ; ledit radical alkyle ou alcényle étant éventuellement substitué par au moins un groupement hydroxyle ;

- $A_2$ représente un radical divalent alkylène en $C_1$-$C_{10}$, linéaire reliant deux radicaux $A_1$ par l'intermédiaire d'un atome de carbone, d'oxygène ou d'azote ;
- les composés de formules **(i)** et **(ii)** comprenant le cas échéant un contre ion anionique An ou un mélange d'anions cosmétiquement acceptables, garantissant l'électroneutralité des formules.

Selon une première variante de l'invention, le ou les composés de formules **(i)** et **(ii)** sont tels que **X** représente un atome d'oxygène.

Les composés (i) et (ii) peuvent se trouver également sous la forme d'acétal cyclique à 5 ou 6 chaînons ou acyclique résultant de la condensation d'un monoalcool primaire additionnel ($R'_3OH$) dans lequel $R'_3$ représente un radical alkyle en $C_1$-$C_5$ ou un diol 1,2 ou 1,3 symétrique présentant une chaîne alkyle en $C_2$-$C_3$.

Ces composés peuvent se trouver également sous la forme d'hémiacétal résultant de la condensation d'un groupement hydroxyle présent sur A ou $A_1$ lorsque A ou $A_1$ représente un radical alkyle et lorsque n vaut 0.

Il est à noter que l'électroneutralité des formules **(i)** et **(ii)** est vérifiée si nécessaire au moyen d'un anion ou un mélange d'anions, organiques ou minéraux permettant d'équilibrer la ou les charges desdits composés , par exemple choisi parmi un halogénure tel que chlorure, bromure, fluorure, iodure ; un hydroxyde ; un sulfate ; un hydrogénosulfate ; un alkylsulfate pour lequel la partie alkyle, linéaire ou ramifiée, est en $C_1$-$C_6$, comme l'ion méthylsulfate ou éthylsulfate ; les carbonates et hydrogénocarbonates ; des sels d'acides carboxyliques tels que le formiate, l'acétate, le citrate, le tartrate, l'oxalate ; les alkylsulfonates pour lesquels la partie alkyle, linéaire ou ramifiée, est en $C_1$-$C_6$ comme l'ion méthylsulfonate ; les arylsulfonates pour lesquels la partie aryle, de préférence phényle, est éventuellement substituée par un ou plusieurs radicaux alkyle en $C_1$-$C_4$ tel que par exemple le 4-toluylsulfonate ; les alkylsulfonyles tel que le mésylate.

Les polysaccharides oxydés non ioniques ou anioniques comprennent un ou plusieurs groupes aldéhydes et éventuellement un ou plusieurs groupes anioniques. Ces groupes anioniques sont de préférence des groupes carboxyliques ou carboxylates.

Les polysaccharides non ioniques ou anioniques oxydés mis en oeuvre peuvent être représentés par la formule (IZ) suivante :

$$P\text{-}(CHO)_m[C(O)OX]_n \qquad \textbf{(IZ)}$$

Formule **(IZ)** dans laquelle :

**P** représente une chaîne polysaccharidique constitués de monosaccharides comprenant 5 atomes de carbone ou plus de 5 atomes de carbone, de préférence 6 ou plus de 6 atomes de carbone et plus particulièrement 6 atomes de carbone.

**X** est choisi parmi un atome d'hydrogène, les ions issus d'un métal alcalin ou alcalino terreux tels que sodium, potassium, l'ammoniaque, les amines organiques comme la monoéthanolamine, la diéthanolamine, la triéthanolamine et l'amino-3 propanediol-1,2 et les aminoacides basiques tels que la lysine, l'arginine, la sarcosine, l'ornithine, la citrulline,

**m + n** est supérieur ou égal à 1,

**m** est tel que le degré de substitution du polysaccharide par un ou plusieurs groupements aldéhydes (DS(CHO)), est compris dans l'intervalle allant de 0,001 à 2, de préférence de 0,005 à 1,5.

**n** est tel que le degré de substitution du polysaccharide par un ou plusieurs groupements carboxyliques (DS(COOX)), est compris dans l'intervalle allant de 0 à 2, de préférence de 0,001 à 1,5.

Par degré de substitution DS(CHO) ou DS (COOX) des polysaccharides selon l'invention, on entend le rapport entre le nombre de carbones oxydés en un groupement aldéhyde ou carboxylique pour tous les motifs répétitifs et le nombre de monosaccharides élémentaires (même ouverts par préoxydation) constituant le polysaccharide.

Les groupes CHO et C(O)OX peuvent être obtenus lors de l'oxydation de certains atomes de carbone, par exemple en position C2, C3 ou C6, d'un motif saccharidique 6 atomes de carbone ; De préférence, l'oxydation peut se faire en C2 et en C3, plus particulièrement de 0,01% à 75% en nombre, et de préférence de 0,1% à 50% en nombre des cycles pouvant avoir été ouverts.

La chaîne polysaccharidique, représentée par **P,** est de préférence choisie parmi les inulines, celluloses, les amidons, les pectines, les gommes de guar, les gommes de xanthane, les gommes de pullulane, les gommes alginate les gommes d'agar-agar, les gommes de carragheenane, les gommes de gellane, les gommes arabique, les xyloses et les gommes adragante et leurs dérivés.

Par dérivé, on entend les composés obtenus par modification chimique des composés cités. Il peut s'agir d'esters, d'amides, d'éthers desdits composés.

L'oxydation peut se faire selon un procédé connu dans la technique, par exemple selon le procédé décrit dans

FR 2 842 200, dans le document FR 2 854 161 ou dans l'article "Hydrophobic films from maize bran hemicelluloses" de E. Fredon et al, Carbohydrate Polymers 49, 2002, pages 1 à 12. Un autre procédé d'oxydation est décrit dans l'article « water soluble oxidized starches by peroxide reaction extrusion » Industril Crops and Products 75 (1997) 45-52 - R. E. Wing, J. L. Willet. Ces procédés d'oxydation sont simples à mettre en oeuvre, sont efficaces, ne génèrent pas de sous-produits toxiques ou difficiles à éliminer.

Le peroxyde peut être un percarbonate ou un perborate de métal alcalin ou de métal alcalino-terreux, un peroxyde d'alkyle, l'acide peracétique ou le peroxyde d'hydrogène. La quantité de peroxyde dans le milieu réactionnel est généralement entre 0,05 et 1 équivalent molaire par unité glucose du polysaccharide.

On peut utiliser comme catalyseur une phtalocyanine unique ou un mélange de phtalocyanines, par exemple un mélange de phtalocyanine de Co et de phtalocyanine de Fe. La quantité de catalyseur dépend du degré de substitution souhaité. En général une faible quantité, par exemple une quantité correspondant à 0,003 à 0,016 équivalent molaire pour 100 unités glucose de polysaccharide est convenable.

Le procédé peut également être mis en oeuvre en mettant en contact le polysaccharide à l'état pulvérulent avec le catalyseur dissous dans un faible volume d'eau et avec le peroxyde. Ce procédé est désigné par procédé « demi-sec ».

Plus préférentiellement, le polysaccharide est obtenu par oxydation d'inuline, de cellulose, carboxyméthylcellulose, hydroxyéthylcellulose, hydroxypropylcellulose, hydroxypropylméthylcellulose, méthylcellulose, amidon, acétate d'amidon, hydroxyéthyl-amidon, hydroxypropylamidon, gomme de guar, gomme de carboxyméthylguar, gomme de carboxyméthylhydroxypropylguar, gomme d'hydroxyéthylguar, gomme d'hydroxy-propylguar, xylose ou gomme de xanthane, gomme de carraghenane, ou leurs mélanges.

Les polysaccharides les plus particulièrement préférés dans l'invention sont ceux répondant à la formule **(IZ)** dans laquelle P représente une chaîne polymérique issue d'inuline et d'amidon ; m est tel que le degré de substitution du polysaccharide par un ou plusieurs groupements aldéhydes (DS(CHO)), est compris dans l'intervalle allant de 0,005 à 2.5 ; n est tel que le degré de substitution du polysaccharide par un ou plusieurs groupements carboxyliques (DS(COOX)), est compris dans l'intervalle allant de 0,001 à 2.

A titre d'exemples de composés de formules **(i)** et **(ii),** oligo- et poly- saccharides oxydés comprenant au moins une fonction aldéhyde ou imine, convenant particulièrement à la réalisation de l'invention, on peut citer les composés suivants, leurs sels d'acide ou de base, organique ou minéral, leurs isomères, leurs solvates, ces composés étant seuls ou en mélange :

| Structure | Nom chimique | Structure | Nom chimique |
|---|---|---|---|
| 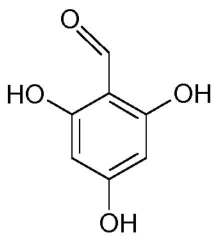 | 2,4,6-trihydroxybenzaldehyde | 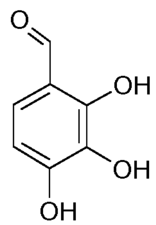 | 2,3,4-trihydroxybenzaldehyde |
| 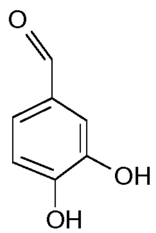 | 3,4-trihydroxybenzaldehyde | 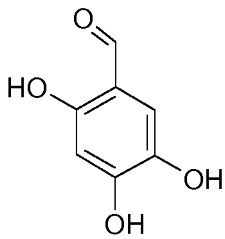 | 2,4,5-trihydroxybenzaldehyde |
| 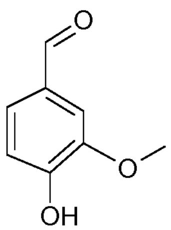 | Vanillin | 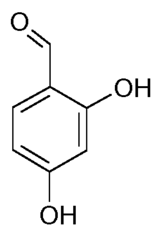 | 2,4-dihydroxybenzaldehyde |

(suite)

| Structure | Nom chimique | Structure | Nom chimique |
|---|---|---|---|
| 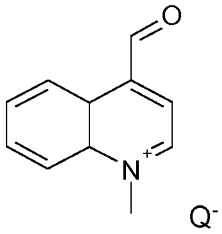 | Sel de 4-formyl-1-méthylquinolinium, de avec Q- représentant un contre ion anionique de préférence methyl benzène sulfonate | 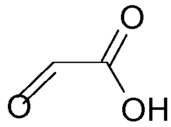 | oxoacetic acid |
| 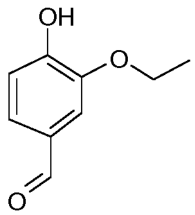 | Ethyl Vanillin | 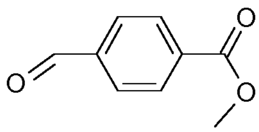 | methyl 4 formybenzoate |
| 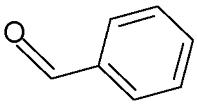 | Benzaldehyde | 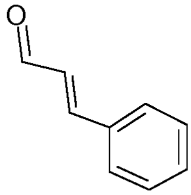 | 3-phenylprop-2-enal |
| 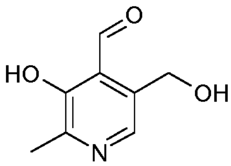 | 3-hydroxy-5-(hydroxymethyl)-2-methylpyridine-4-carbaldehyde | 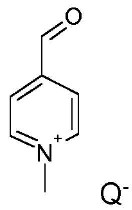 | Sel de 4-formyl-1-methylpyridinium avec $Q^-$ tel que défini de preference $Q^-$ benzenesulfonate |

| Structure | Nom chimique | Structure | Nom chimique |
|---|---|---|---|
| 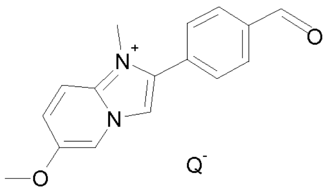 | Sel de 2-(4-formylphenyl)-6-methoxy-1-methylimidazo [1,2-a]pyridine-1-ium avec $Q^-$ tel que defini précédemment de préférence halogénure tel que $Cl^-$ | 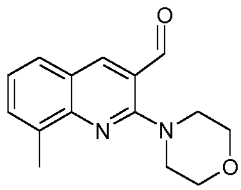 | 8-methyl-2-(morpholin-4-yl) quinoline-3-carbaldehyde |
| 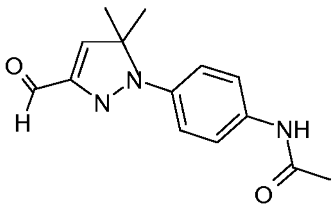 | N-[4-(3-formyl-5,5-dimethyl-4,5-dihydro-1H-pyrazol-1-yl)phenyl] acetamide | 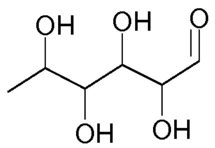 | 6-deoxyhexose |

(suite)

| Structure | Nom chimique | Structure | Nom chimique |
|---|---|---|---|
| 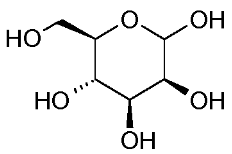 | D-mannopyranose | 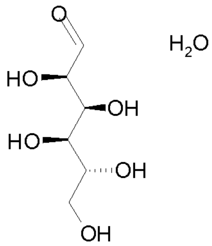 | D-glucose |
| 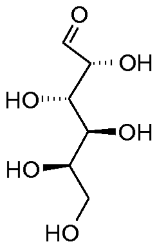 | D-galactose | 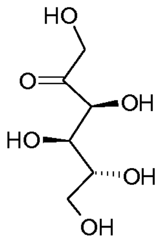 | D-Fructose |
| 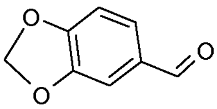 | 1,3-benzodioxole-5-carbaldehyde | 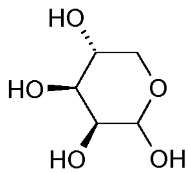 | D-lyxopyranose |
| 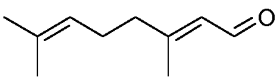 | 2,7-dimethylocta-2,6-dienal | 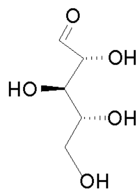 | D-ribose |
| 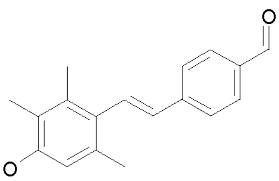 | 4-[2-(4-methoxy-2,3,6-trimethylphenyl)ethe nyl] benzaldehyde | 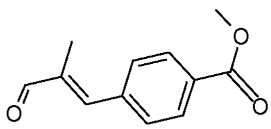 | methyl 4-[2-methyl-3-oxoprop-1-en-1-yl]benzoate |
| 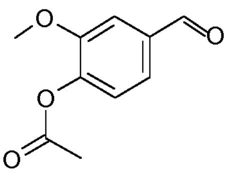 | 4-formyl-2-methoxyphenyl acetate | 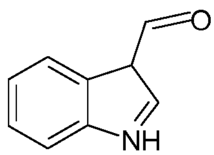 | 1H-indole-3-carbaldehyde |
| 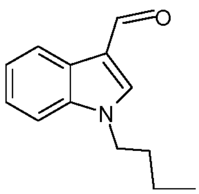 | 1-butyl-1H-indole-3-carbaldehyde | 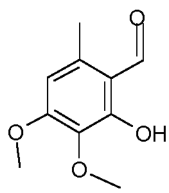 | 2-hydroxy-3,4-dimethoxy-6-methylbenzaldeh yde |

| Structure | Nom chimique | Structure | Nom chimique |
|---|---|---|---|
| 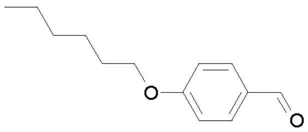 | 4-(hexyloxy) benzaldehyde | 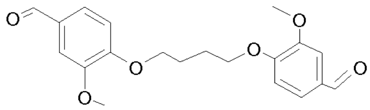 | 4,4'-[butane-1,4-diylbis(oxy)] bis (3-methoxybenzal dehyde) |
| 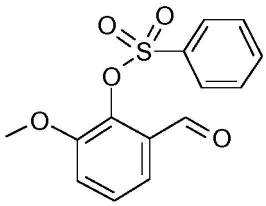 | 2-formyl-6-methoxyphenyl benzenesulfonate | 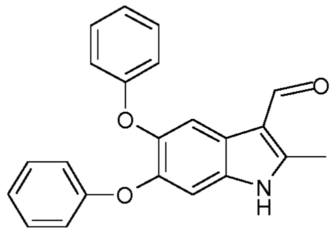 | 5,6-bis (benzyloxy)-2-methyl-1H-indole-3-carbaldehyde |
| 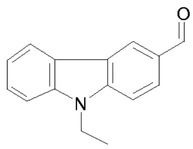 | 9-ethyl-9H-carbazole-3-carbaldehyde | 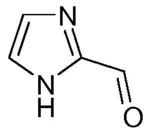 | 1H-imidazole-2-carbaldehyde |
| 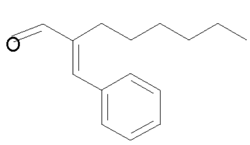 | 2-benzyllideneoctanal | 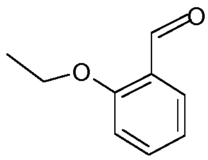 | 2-ethoxybenzald ehyde |
| 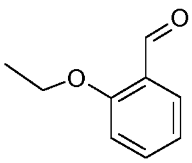 | 2-ehtoxybenzaldehyde | 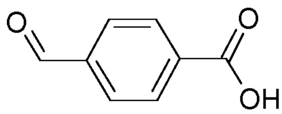 | 4-formylbenzoic acid |
| 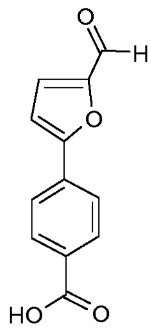 | 4-(5-formylfuran-2-yl) benzoic acid | 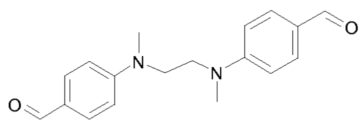 | 4,4'[ethane-1,2-diylbis(methyli mino)di benzaldehyde |
| 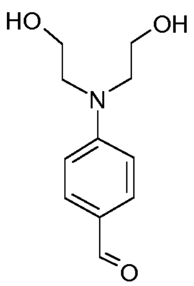 | 4-[bis(2-hydroxyethyl)amino] benzaldehyde | 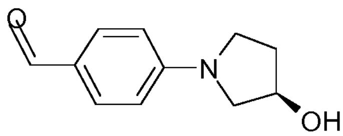 | 4-[(3R)-3-hydroxypyrroli din-1-yl] benzaldehyd e |

(suite)

| Structure | Nom chimique | Structure | Nom chimique |
|---|---|---|---|
| 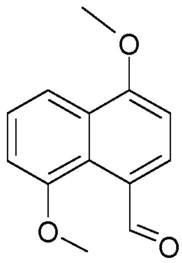 | 4,8-dimethoxynapht alene-1-carbaldehyde | 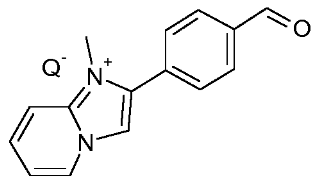 | Sel de 2-(4-formylphenyl)-1-methylimidazo [ 1,2-a]pyridine-1-ium avec $Q^-$ tel que define précédemment préférentiellement un halogénure tel que $Cl^-$ |
| 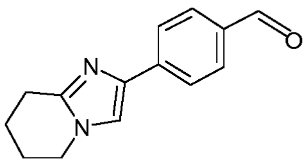 | 4-(5,6,7,8-tetrahydroimida zol [1,2-a]pyridin-2-yl) benzaldehyde | 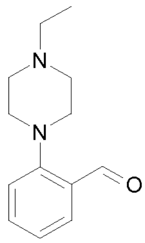 | 2-(4-ethylpiperazin-1-yl) benzaldehyd e |

| Structure | Nom chimique | Structure | Nom chimique |
|---|---|---|---|
| 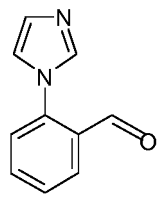 | 2-(1H-imidazol-1-yl) benzaldehyde | 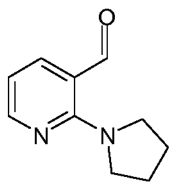 | 2-(pyrrolidin-1-yl) pyridine-3-carbaldehyde |
| 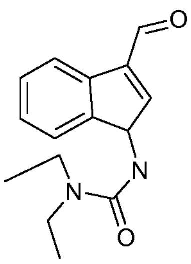 | N,N-diethyl-2-(3-formyl-1H-indol-1-yl) acetamide | 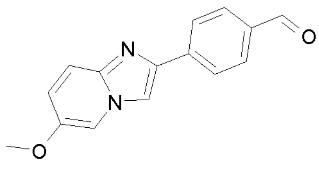 | 4-(6-methoxyimidazol [ 1,2-a]pyridin-2-yl) benzaldehyde |
| 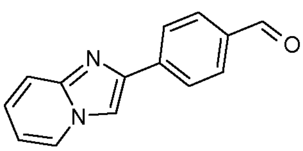 | 4-(imidazo[1,2-a] pyridin-2yl) benzaldehyde | 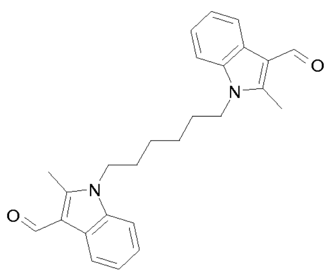 | 1,1'-hexane-1,6-diylbis(2-methyl-1-H-indole-3-carbaldehyde |
| | Amidon oxydé | | |

De préférence, la composition comprend au moins un troisième composé choisi parmi les composés de formule (i).

Avantageusement, la formule (i) est telle que :

* m vaut 0 ou 1
* les radicaux $R'_1$ et $R'_2$, indépendamment l'un de l'autre représentent un atome d'hydrogène, un radical alkyle en $C_1$-$C_6$ linéaire non substitué ;
* X représente un atome d'oxygène

A représente :

* un radical alkyle en $C_5$-$C_{10}$ linéaire ou ramifié,, éventuellement substitué par au moins un groupement hydroxyle ;
* un radical alcényle en $C_2$-$C_8$ comprenant une double liaison carbone-carbone
* un groupement hydroxycarbonyle (-C(O)OH)
* un groupement aryle en $C_6$ éventuellement substitué par au moins :

  ◦ un groupement hydroxyle,
  ◦ un radical alkyle en $C_1$-$C_4$,
  ◦ un groupement -C(O)-OR'$_4$ ou -O-C(O)R'$_4$ où $R'_4$ représente un radical alkyle en $C_1$-$C_2$,
  ◦ un groupement -OR'$_5$ où $R'_5$ représente un radical alkyle en $C_1$-$C_6$,
  ◦ un radical aryle-éthylényle, le groupement aryle étant en $C_6$ et éventuellement substitué par au moins un radical alkyle en $C_1$-$C_2$, alcoxy en $C_1$-$C_2$,
  ◦ un hétérocycle à 5 ou 6 chaînons insaturé, cationique ou non cationique, comprenant un ou deux hétéroatomes, choisis parmi O, N, NR'$_{12}$ où $R'_{12}$ représente un groupement alkyle en $C_1$-$C_2$, éventuellement condensé à un cycle à 5 ou 6 chaînons, saturé ou non, aromatique ou non, l'un des hétéroatomes pouvant éventuellement être inclus dans les deux cycles ; l'hétérocycle ou le cycle condensé pouvant être substitué par au moins un radical alcoxy en $C_1$-$C_2$

  - ledit groupement aryle étant éventuellement condensé à un groupement hétérocyclique à 5 ou 6 chaînons, comprenant un ou deux hétéroatomes choisi parmi O, N, NR'$_{13}$ où $R'_{13}$ représente un radical alkyle en $C_1$-$C_4$ ;

* un groupement hétérocyclique à 5 ou 6 chaînons, cationique ou non cationique, insaturé ou non, aromatique ou non, comprenant un ou deux hétéroatomes, identiques ou non, choisis de préférence parmi O, N, NR'$_{14}$ avec $R'_{14}$ représentant un atome d'hydrogène, un radical alkyle en $C_1$-$C_6$, ou aryle en $C_6$ éventuellement substitué par un groupement $(R'_{15})_2$NCO- ou $(R'_{15})$CO-NH- où $R'_{15}$, représentent un radical alkyle en $C_1$-$C_2$ ;

- ledit groupement hétérocyclique étant éventuellement condensé à un groupement aryle à 6 chaînons lui-même éventuellement substitué par au moins un radical alkyle en $C_1$-$C_2$, alcoxy en $C_1$-$C_4$ ;
- ledit groupement hétérocyclique étant éventuellement substitué par au moins :

  ◦ un groupement hydroxyle,
  ◦ un radical alkyle en $C_1$-$C_2$ éventuellement substitué par un radical hydroxyle ;
  ◦ un radical amino -N$(R'_{16})_2$, où $R'_{16}$, identiques ou non, représentent un radical alkyle en $C_1$-$C_4$ éventuellement substitué par un groupement hydroxyle, les radicaux $R'_{16}$ pouvant éventuellement former un hétérocycle à 5 ou 6 chaînons avec l'atome d'azote qui les porte, saturé ou non, comprenant éventuellement un autre hétéroatome choisis parmi O, N, NR'$_{17}$ où $R'_{17}$ représente un radical alkyle en $C_1$-$C_2$,

* les composés de formules (i) comprenant le cas échéant un contre ion anionique An ou un mélange d'anions cosmétiquement acceptables, garantissant l'électroneutralité des formules ;
* le ou les composés de formule (i) pouvant se trouver sous la forme d'acétal ou d'hémiacétal, tels que décrit précédemment.

A titre d'exemples de composés (i) préférés, on peut citer les composés suivants, leurs isomères, leurs sels, leurs solvates, ces composés étant seuls ou en mélanges :

| Structure | Nom chimique | Structure | Nom chimique |
|---|---|---|---|
| 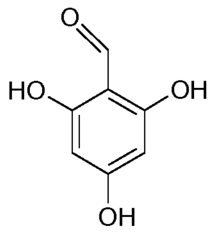 | 2,4,6-trihydroxy-benzaldéhyde | 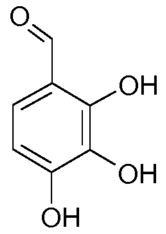 | 2,3,4-trihydroxy-benzaldéhyde |
| 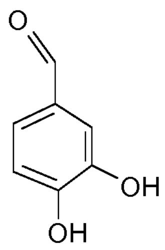 | 3,4-trihydroxy-benzaldéhyde | 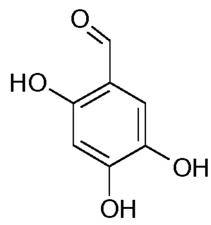 | 2,4,5-trihydroxy-benzaldéhyde |
| 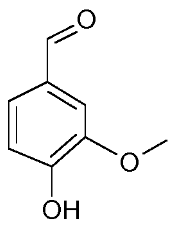 | Vanilline | 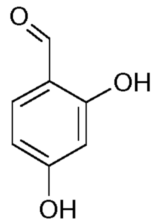 | 2,4-dihydroxy-benzaldéhyde |
| 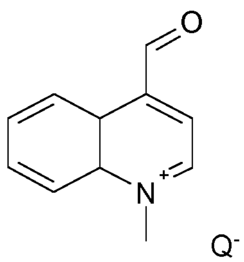 | Sel de 4-formyl-1-methylquinolinium Sel de 4-formyl-1-méthylquinolinium , avec Q- représentant un contre ion anionique tel que methyl benzène sulfonate | 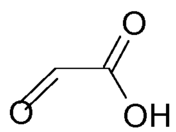 | oxoacetic acid |
| 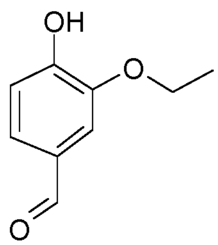 | Ethyl Vanillin | 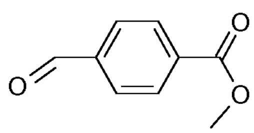 | methyl 4 formybenzoate |
| 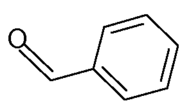 | Benzaldehyde | 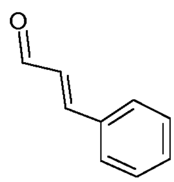 | 3-phenylprop-2-enal |

(suite)

| Structure | Nom chimique | Structure | Nom chimique |
|---|---|---|---|
| 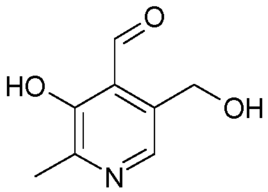 | 3-hydroxy-5-(hydroxymethyl)-2-methylpyridine-4-carbaldehyde de préférence hydrochloride | 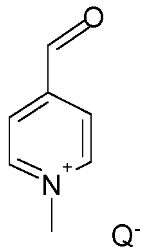 | Sel de 4-formyl-1-methylpyridinium avec Q- représentant un contre ion anionique tel que methyl benzène sulfonate |
| 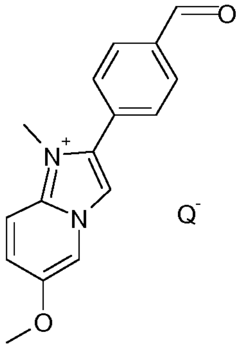 | 2-(4-formylphenyl)-6-methoxy-1-methylimidazo [1,2-a]pyridine-1-ium avec Q- tel que define précédemment, de préférence halogénure tel que Cl- | 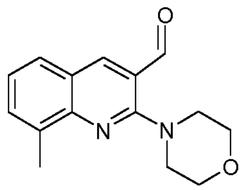 | 8-methyl-2-(morpholin-4-yl) quinoline-3-carbaldehyde |
| 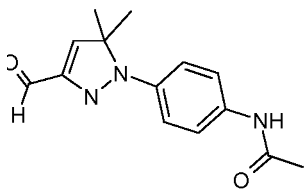 | N-[4-(3-formyl-5,5-dimethyl-4,5-dihydro-1H-pyrazol-1-yl) phenyl]-acetamide | 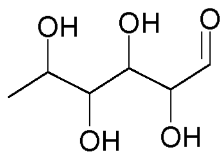 | 6-deoxyhexose |
| 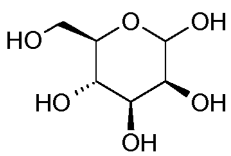 | D-mannopyranose | 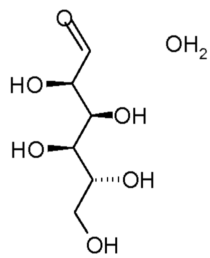 | D-glucose |
| 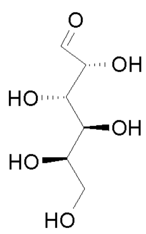 | D-galactose | 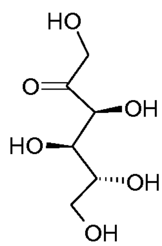 | D-Fructose |
| 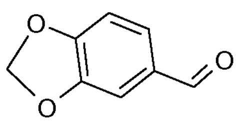 | 1,3-benzodioxole-5-carbaldehyde | 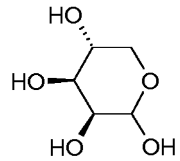 | D-lyxopyranose |

(suite)

| Structure | Nom chimique | Structure | Nom chimique |
|---|---|---|---|
| 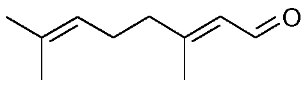 | (2E)-2,7-dimethylocta-2,6-dienal | 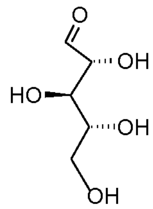 | D-ribose |
| 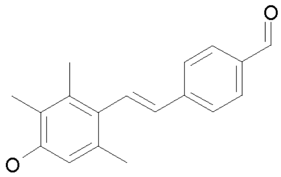 | 4-[(E)-2-(4-methoxy-2,3,6-trimethyl-phenyl) ethenyl]-benzaldehyde | 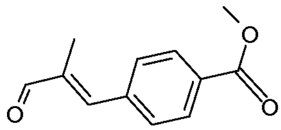 | methyl 4-[(1E)-2-methyl-3-oxoprop-1-en-1-yl]benzoate |
| 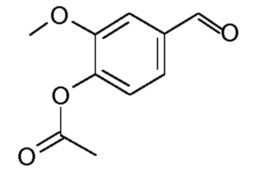 | 4-formyl-2-methoxyphenyl acetate | 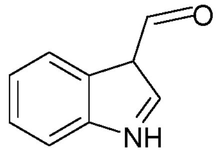 | 1H-indole-3-carbaldehyde |
| 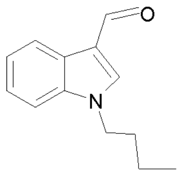 | 1-butyl-1H-indole-3-carbaldehyde | 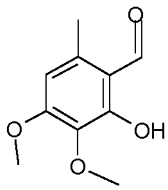 | 2-hydroxy-3,4-dimethoxy-6-methylbenzaldehyd e |

A titre d'exemples de d'oligo- ou poly-saccharides oxydés préférés, on peut citer les composés suivants :

- l'amidon oxydé (Rn 9047-50-1), amidon connue sous les noms commerciaux suivants : Caldas 10; Caldas 5; Caldas 5H; Caldas 5S; Caldas C 5; Caldas C 5GP; Caldas C 5GT; Caldas No. 5; DAS 100; Dialdehyde starch; Dialdehydostarch; Formamyl; Periodate starch; Polyaldehyde starch; Starch dialdehyde; Sumstar; Sumstar 150; Sumstar 190
- la cellulose oxydée (Rn 9032-52-4) connues sous les dénominations 2,3-dialdehyde cellulose; 2,3-Dialdehydocellulose; Aldehydocellulose; Cellulose 2,3-dialdehyde; Cellulose dialdehyde; Dialdehyde cellulose;
- l'inuline oxydée (Rn 82446-43-3)
- Le dextran dialdehyde (Rn 37317-99-0).

Selon un autre mode préféré de l'invention le ou les composés électrophiles sont choisis parmi *les composés carbonylés ou cétoniques* et de préférence choisi parmi les composés (dicétones) de formules **(a)** suivantes, leurs isomères optiques ou géométriques, leurs sels d'acide ou de base, organique ou minéral, leurs tautomères, et leurs solvates :

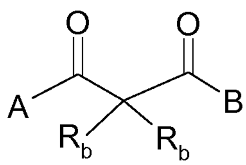

**(a)**

Formule **(a)** dans laquelle :

• **A** représente un groupe choisi parmi :

| | | |
|---|---|---|
| 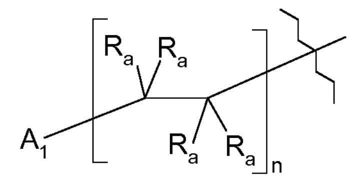 | 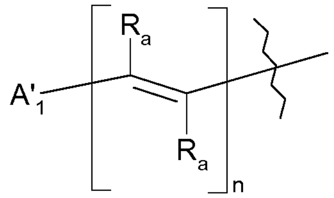 | 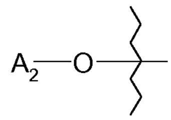 |
| (iiiA1) | (iiiA2) | (iiiA3) |

avec  représentant le point d'attache de la liaison avec le reste de la molécule (a) ;

• **B** représente un groupe choisi parmi:

| | | |
|---|---|---|
| 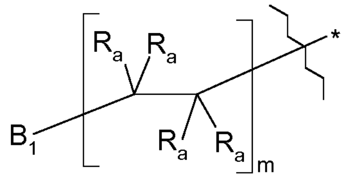 | 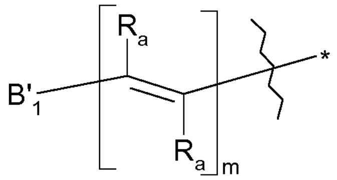 | 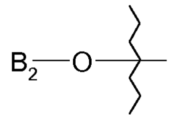 |
| (iiiB1) | (iiiB2) | (iiiB3) |

avec  représentant le point d'attache de la liaison avec le reste de la molécule (a) ;

• **n** et **m,** identiques ou différents, valent 0 ou 1 ;

• **$R_a$,** identiques ou différents, représentent un atome d'hydrogène, un radical alkyle en $C_1$-$C_4$ ;

• **$R_b$,** identiques ou différents, représentent un atome d'hydrogène, un groupement acétyle ($CH_3$-C(O)-) ; l'un des deux radicaux $R_b$ représentant un atome d'hydrogène, ou bien deux radicaux $R_b$ représentant ensemble, un groupement phényl-méthylényle phényl-C(H)=, ledit radical phényle étant éventuellement substitué par au moins un radical hydroxyle, alcoxy en $C_1$-$C_4$ ;

• **$A_1$, $B_1$, $A'_1$, $B'_1$,** identiques ou différents, représentent ;

◦ un groupement aryle en $C_6$ éventuellement substitué par au moins

- un radical alkyle en $C_1$-$C_{20}$ linéaire ou ramifié
- un radical hydroxyle
- un radical alcoxy -O-$R_1$ où $R_1$ représente un radical alkyle en $C_1$-$C_4$ éventuellement substitué par un groupement hydroxyle, un groupement -Si($R_2$)(OSi($R_3$)$_3$)$_3$ où $R_2$, $R_3$, identiques ou non, représentent un radical alkyle en $C_1$-$C_4$, de préférence méthyle
- un groupement ester -O-C(O)-$R_4$ où $R_4$ représente un radical phényle
- un groupement ammonium -$N^+$($R_5$)$_3$ où $R_5$ identiques ou non, représentent un radical alkyle en $C_1$-$C_4$ éventuellement porteur d'au moins un groupement hydroxyle
- un atome d'halogène, de préférence le chlore

    • éventuellement condensé à un hétérocycle à 5 ou 6 chaînons, insaturé ou aromatique, comprenant au moins un hétéroatome de préférence l'oxygène ;

◦ un groupement pyridinyle ou pyridinium ; l'atome d'azote quaternisé étant substitué par un radical alkyle en $C_1$-$C_4$ éventuellement porteur d'un groupement hydroxyle ;

◦ un groupement alkyle en $C_1$-$C_4$ ;

◦ les groupements A et B pouvant former ensemble un cycle ou hétérocycle à 5 ou 6 chaînons comprenant éventuellement un hétéroatome, de préférence l'oxygène ; ledit cycle ou hétérocycle étant éventuellement condensé à un radical phényle éventuellement substitué par au moins un groupement acétyle ($CH_3$-C(O)-), ester -C(O)-O-$R_6$ ou -O-C(O)-$R_6$ avec $R_6$ représentant un radical alkyle en $C_1$-$C_4$ ;

• **$A_2$** et **$B_2$,** identiques ou différents, représentent :

◦ un radical alkyle en $C_1$-$C_{10}$ linéaire ou ramifié, pouvant éventuellement présenter une ou plusieurs insaturations, éventuellement substitué par un groupement -$SiR_3$ les radicaux R, identiques ou non, représentant un radical alkyle en $C_1$-$C_4$ ;
◦ un radical alcényle en $C_3$-$C_6$ ;
◦ un atome d'hydrogène, un métal alcalin, alcalino-terreux, un groupement ammonium ;
◦ les radicaux $A_2$ et $B_2$ pouvant éventuellement former ensemble un hétérocycle à 6 chaînons ;
◦ les radicaux $R_b$ et $A'_1$, dans le cas où n vaut 1, peuvent former ensemble un cycle hydrocarboné à 6 chaînons, éventuellement substitué par un groupement alkyle en $C_1$-$C_2$, un groupement hydroxyle ;

Selon une variante, le composé électrophyle est de formule **(a)** précitée, ses isomères, ou ses sels, ses solvates ; ce composé étant seul ou en mélange.

A titre d'exemples de composés de ce type, on peut citer les composés suivants, leurs isomères optiques, géométriques, leurs sels, leurs solvates ; ces composés étant seuls ou en mélange :

| Structure | Nom chimique |
|---|---|
| 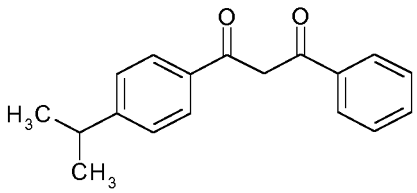 | 1-phenyl-3-[4-(propan-2-yl)phenyl]propane-1,3-dione |
| 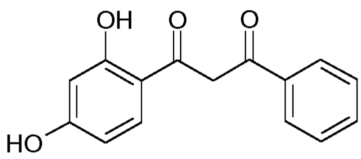 | 1-(2,4-dihydroxyphenyl)-3-phenylpropane-1,3-dione |
| 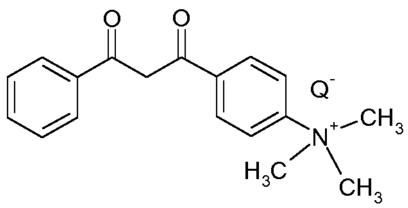 | Sel de N,N,N-triméthyl-4-(3-oxo-3-phenylpropanoyl) anilinium avec $Q^-$ tel que defini précédemment, préférentiellement un halogénure tel que $Cl^-$ |
| 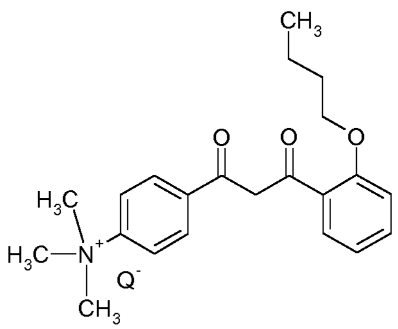 | Sel de 4-[3-(2-butoxyphényl)-3-oxopropanoyl]-N, N,N-trimethylanilinium avec $Q^-$ tel que defini précédemment, préférentiellement alkyl sulfate tel que méthylsulfate |
| 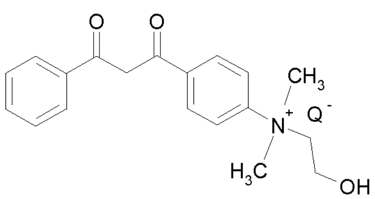 | Sel de N-(2-hydroxyethyl)-N,N-diméthyl-4-(3oxo-3-phenylpropanoyl)anilinium avec $Q^-$ tel que defini précédemment, préférentiellement un halogénure tel que Br |

(suite)

| Structure | Nom chimique |
|---|---|
| 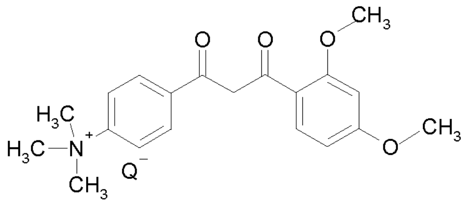 | Sel de 4-[3-(2,4-dimethoxyphenyl)-3-oxopropanoyl]-N, N,N,-trimethylanilinium avec $Q^-$ tel que defini précédemment, préférentiellement alkyl sulfate tel que méthylsulfate |
| 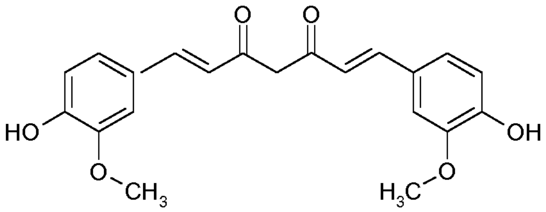 | 1,7-bis(4hydroxy-3 Methoxyphenyl)hepta-1,6-diène-3,5-dione |
| 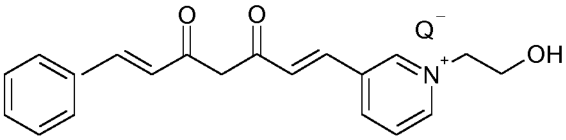 | Sel de 1-(2-hydroxyethyl) -3-(3-oxo-3-phenylpropanoyl) pyridinium avec $Q^-$ tel que defini précédemment, préférentiellement un halogénure tel que Br |
| 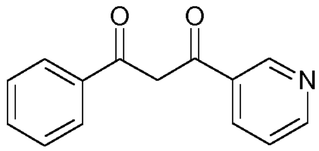 | 1-phenyl-3-(pyridin-3-yl)propane-1,3-dione |
| 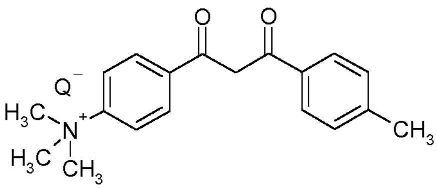 | Sel de N,N,N-trimethyl-4-[3-(4-methylphenyl)-3-oxopropanoyl]anilinium avec $Q^-$ tel que defini précédemment, préférentiellement alkyl sulfate tel que méthylsulfate |
| 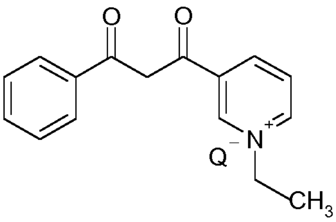 | Sel de 1-ethyl-3-(3-oxo-3-phenylpropanoyl)pyridinium avec $Q^-$ tel que defini précédemment, préférentiellement un halogénure tel que Br |
| 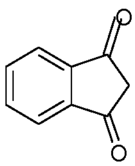 | 1H-indene-1,3(2H)-dione |
| 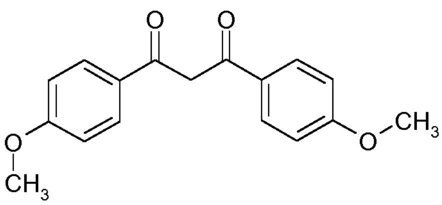 | 1,3-bis(4-methoxyphenyl)propane-1,3-dione |

(suite)

| Structure | Nom chimique |
|---|---|
| 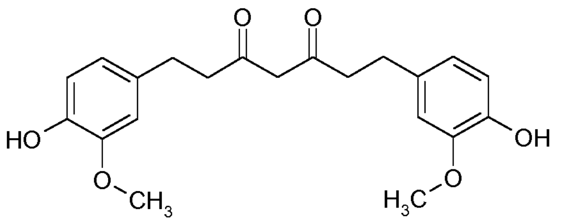 | 1,7-bis(4-hydroxy-3-methoxyphenyl)heptane-3,5-dione |
| 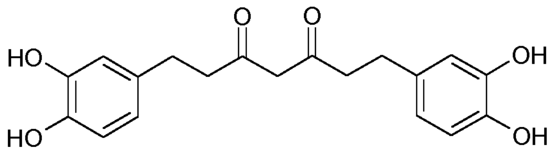 | 1,7-bis(3,4-dihydroxyphenyl)heptane-3,5-dione |
| 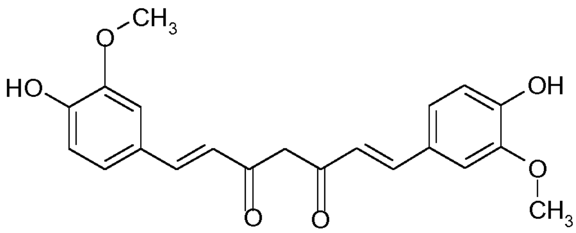 | 1,7-bis(4-hydroxy-3-methoxyphenyl)hepta-1,6-diene-3,5-dione |
| 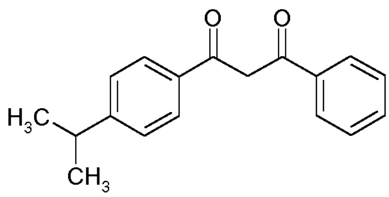 | 1-phenyl-3-[4-(propan-2-yl) phenyl]propane-1,3-dione |
| 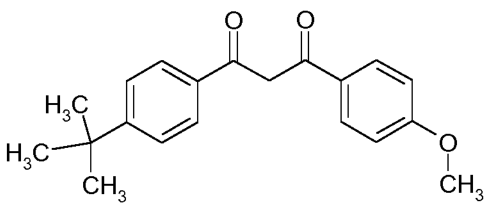 | 1-(4-tert-butylphenyl)-3-(4-methoxyphenyl)propane-1,3-dione |
| 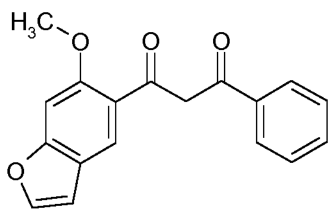 | 1-(6-methoxy-1-benzofuran-5-yl)-3-phenylpropane-1,3-dione |
| 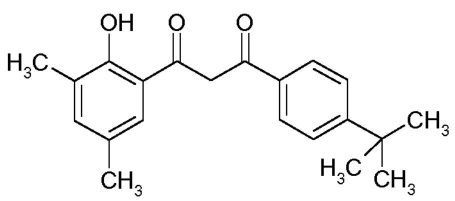 | 1-(4-tert-butylphenyl)-3-(2-hydroxy-3,5-dimethylphenyl) propane-1,3-dione |

(suite)

| Structure | Nom chimique |
|---|---|
| 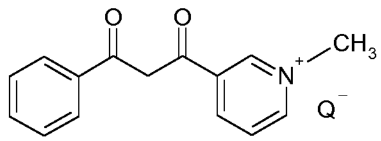 | Sel de 1 -methyl-3-(3-oxo-3-phenylpropanoyl)pyridinium avec $Q^-$ tel que defini précédemment, préférentiellement alkyl sulfate tel que méthylsulfate |
| 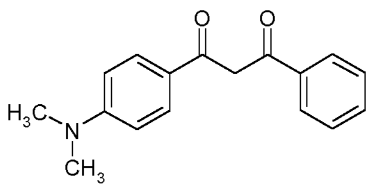 | 1-[4-dimethylamino]phenyl]-3-phenylpropane-1,3-dione |
| 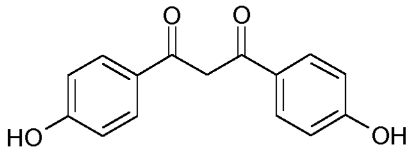 | 1,3-bis(4-hydroxyphenyl)propane-1,3-dione |
| 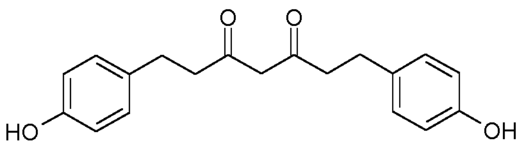 | 1,7-bis(4-hydroxyphenyl)heptane-3,5-dione |
| 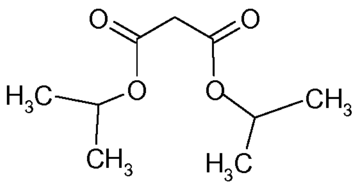 | bis(2-propyl) propanedioate |
| 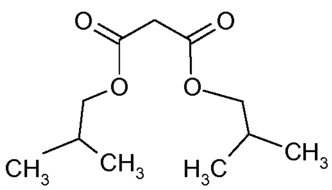 | bis(2-methylpropyl) propanedioate |
| 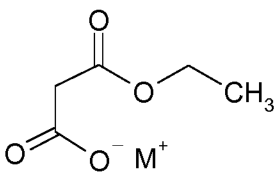 | Sel de 3-ethoxy-3-oxopropanoate avec $M^+$ représentant un contre ion cationique, de préférence métal alcalin ou alcalino terreux tel que $K^+$ |
| 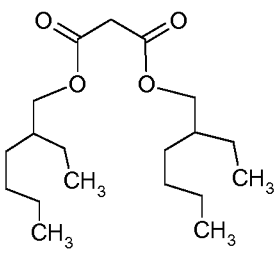 | bis(2-ethylhexyl) propanedioate |

(suite)

| Structure | Nom chimique |
|---|---|
| 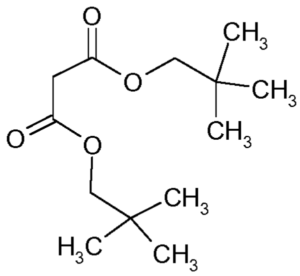 | Bis(2,2-dimethylpropyl)propanedioate |
| 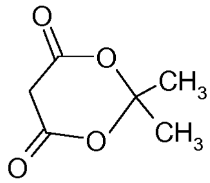 | 2,2-dimethyl-1,3-dioxane-4,6-dione |
| 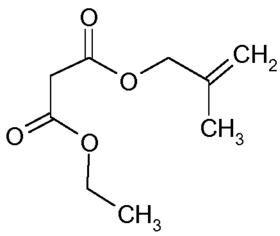 | ethyl 2 -methylprop-2-en-1-yl propanedioate |
| 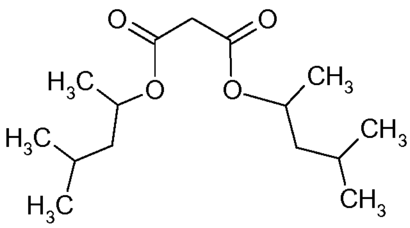 | bis (4-methylpentan-2-yl) propanedioate |
| 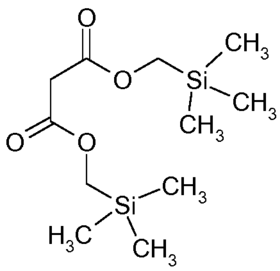 | bis[(trimethylsilyl)methyl] propanedioate |
| 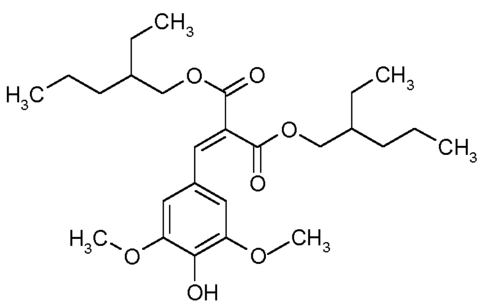 | bis (2-ethylpentyl)(4-hydroxy-3,5-dimethoxybenzylidene) propanedioate |

Selon un autre mode préféré de l'invention le ou les composés électrophiles sont choisis parmi *les composés*

*hydroxycétones et en particulier les 1,2- ou 1,3-hydroxycétones* qui sont de préférence choisis parmi les composés de formule **(b)** et **(c)** suivantes :

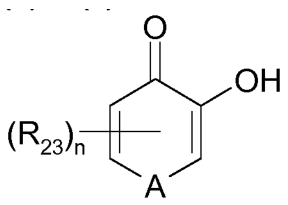 **(b)** 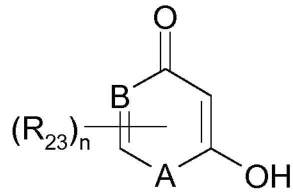 **(c)**

formule **(b)** ou **(c),** ainsi que leur sels d'acide ou de bases, organiques ou minérals, et leurs tautomères, dans lesquelles :

* **n** est un entier valant de 0 à 3 ; les atomes de carbone non substitués portent un atome d'hydrogène
* **A** représente $-C(R_{24})(R_{23})-$, $-C(R_{24})_2-$, O, -C(O)-, $-C(R_{24})=C(R_{24})-$ ;
* **B** représente $-C(R'_{23})-$, O ; $R'_{23}$ représente un atome d'hydrogène ou $R_{23}$
* $\mathbf{R_{23}}$ identiques ou non représentent un groupement hydroxyle, un radical alkyle en $C_1$-$C_4$ éventuellement substitué par au moins un groupement hydroxyle ;
* deux radicaux $R_{23}$ portés par deux atomes de carbone adjacents, peuvent former ensemble un cycle aromatique condensé éventuellement substitué par au moins un radical alcoxy en $C_1$-$C_4$, un groupement hydroxyle ;
* $\mathbf{R_{24}}$**,** identiques ou non, représentent un atome d'hydrogène, un radical alkyle linéaire ou ramifié en $C_1$-$C_4$ ;

étant entendu que les composés de formules **(a)** à **(c)** comprennent le cas échéant un contre ion anionique $An^-$ ou un mélange d'anions cosmétiquement acceptables, garantissant l'électroneutralité des formules ;

Plus particulièrement le cas où les composés électrophiles de l'invention sont sont de formules **(b)** ou **(c),** et de préférence tels que :

* n est un entier vaut de 0 à 2
* A représente -C(O)-, $-C(R_{24})=C(R_{24})-$ ,de préférence A représente -C(O)-,
* B représente $-CR'_{23}-$, O ; $R'_{23}$ représente un atome d'hydrogène ou $R_{23}$,
* $R_{23}$ identiques ou non représentent un groupement hydroxyle, un radical alkyle en $C_1$-$C_4$ éventuellement substitué par au moins un groupement hydroxyle ;
* deux radicaux $R_{23}$ portés par deux atomes de carbone adjacents, peuvent former ensemble un cycle aromatique condensé éventuellement substitué par au moins un radical alcoxy en $C_1$-$C_4$, un groupement hydroxyle ;
* $R_{24}$, identiques ou non, représentent un atome d'hydrogène, un radical alkyle linéaire ou ramifié en $C_1$-$C_4$.

A titre d'exemples de composés de formules **(b)** ou **(c)** particulièrement appropriés, on peut citer les composés suivants, leurs tautomères, leurs sels, leurs solvates, ces composés étant seuls ou en mélanges :

| Structure | Nom chimique |
|---|---|
| 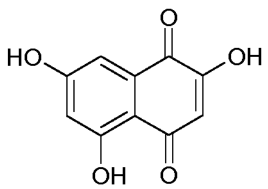 | 2,5,7-trihydroxynapthalene-1,4-dione |
| 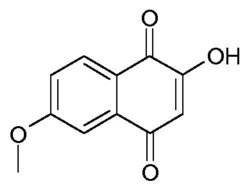 | 2-hydro-6-methoxynaphtalene-1,4-dione |

(suite)

| Structure | Nom chimique |
|---|---|
| 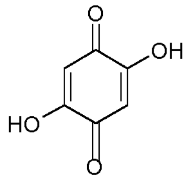 | 2,5-dihydroxycyclohexa-2,5-diene-1,4-dione |
| 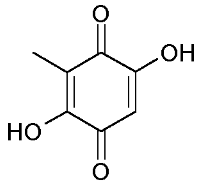 | 2,5-dihydroxy-3-methylcyclohexa-2,5-diene-1,4-dione |
| 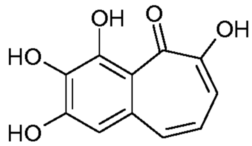 | 2,3,4,6,-tetrahydroxy-5H-benzo[7]annulen-5-one |
| 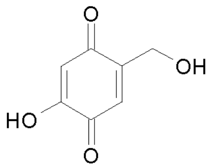 | 5-hydroxy-2-(hydroxymethyl)-4 H-pyran-4-on e |
| 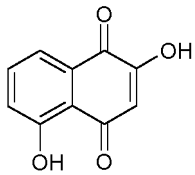 | 2,5,-dihydroxynapthalene-1,4-dione |
| 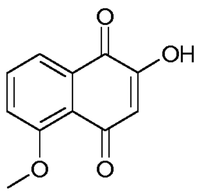 | 2-hydroxy-5-methoxynapthalene-1,4-dione |
| 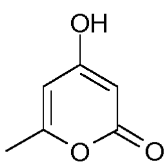 | 4-hydroxy-6-methyl-2 H-pyran-2-on e |
| 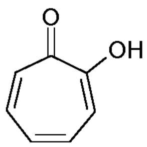 | 2-hydroxycyclohepta-2,4,6-trien-1-one |

(suite)

| Structure | Nom chimique |
|---|---|
| 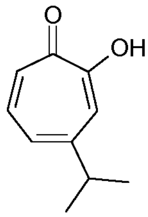 | 2-hydroxy-4-(propan-2-yl)cyclohepta-2,4,6-trien-1-one |

Selon un mode préféré de l'invention le ou les composés électrophiles sont choisis parmi *les composés nitriles* qui sont de préférence de formule **(d)** suivante :

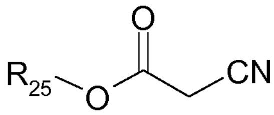

**(d)**

Composé de formule **(d)** dans laquelle $R_{25}$ représente un radical alkyle $C_1$-$C_6$ linéaire ou ramifié éventuellement substitué par un groupement $-SO_3^-M^+$ avec $M^+$ représentant un contre ion cationique tel que ammonium, alcalin ou alcalino terreux, de préférence $Na^+$, $-C(NHR_{26})=NR'_{26}$ où $R_{26}$, $R'_{26}$ identiques ou non, représentent un radical alkyle en $C_4$-$C_8$ cyclique ;

On peut citer à titre d'exemple de composés de formule **(d)** les composés suivants, leurs isomères optiques, géométriques, leurs sels, leurs solvates, ces composés étant seuls ou en mélanges :

| Structure | Nom chimique |
|---|---|
| 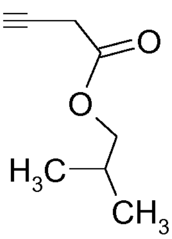 | 2-methylpropyl cyanoacetate |
| 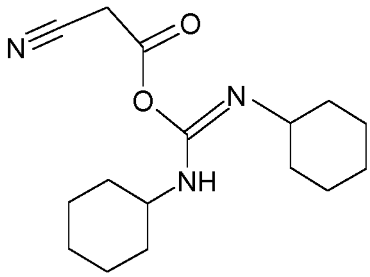 | cyanoacetyl N,N'-dicyclohexyl carbamimidate |
| 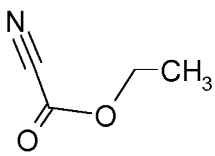 | ethyl cyanoacetate |

(suite)

| Structure | Nom chimique |
|---|---|
| 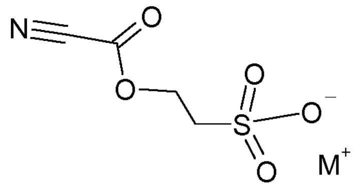 | Sel de 2-[(cyanoacetyl)oxy]ethanesulfonate avec $M^+$ tel que défini précédemment, en particulier $M^+$ représente un métal alcalin tel que sodium |

En ce qui concerne les oligomères ou polymères électrophiles carbonylés, ceux-ci sont choisis parmi les acides polymaloniques et leurs esters, les acides polysucciniques, les polymères d'acide sulfoacétique, ainsi que les sels de ces polymères.
Ces composés sont connus de l'homme du métier.
Il est à noter que les esters d'acide polymalonique sont notamment décrits dans US 4,444,928 , et tout particulièrement le polymère suivant :

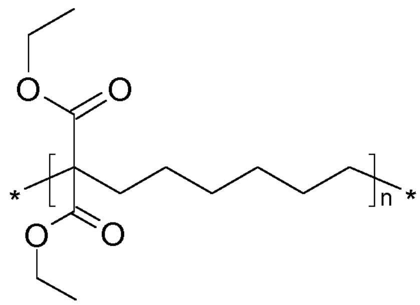

De préférence, le ou les composés carbonylés sont choisis parmi les composés de formules **(i), (ii), (a)** et **(d)** définies auparavant, ainsi que parmi les composés de formules (b) et (c), ou leurs mélanges.
La teneur en troisième composé peut être comprise entre 0,001% et 30 % en poids par rapport au poids de la composition **(A),** ou la composition le comprenant.
Parmi les composés de formule **(a)** à **(d)** décrits ci-dessus, on préfère plus particulièrement les composés de formule **(a),** les composés de formule **(b)** et **(c)** dans lesquelles **A** représente -C(O)-, et leurs mélanges.
Très préférentiellement le ou les composés électrophiles utilisés dans le procédé et la composition de l'invention sont choisis parmi *les composés aldéhydiques* et de préférence choisi parmi les composés de formules suivantes **(i')** ci-dessous, leurs isomères optiques ou géométriques, leurs sels d'acide organique ou minéral, leurs solvates :

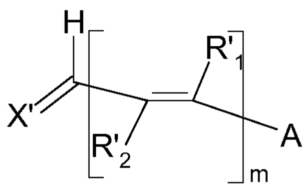

**(i')**

Composé de formule **(i')** dans lequel **X'** représente un atome d'oxygène ou de soufre, de préférence oxygène, $\mathbf{R'_1}$ et $\mathbf{R'_2}$ représentent un atome d'hydrogène ou une groupe ($C_1$-$C_4$)alkyle, **A** représente un groupe aryle éventuellement substitué par les mêmes groupements que définis pour **(i)** précédemment, tel que phényle, **m** est un entier compris inclusviement entre 0 et 2, de préférence entre 0 et 1 tel que 1.

*Enzyme*

La composition selon l'invention et le proécédé de l'invention peut éventuellement comprendre ou mettre en oeuvre au moins une enzyme, choisie par exemple parmi l'isolase, la β-glucosydase issue par exemple d'amande douce (EC 3.2.1.21), l'alcool oxydase (EC 1.1.3.13), les alcool déshydrogenases EC 1.1.1.1, les alcool déshydrogénases EC

1.1.1.2, les alcool déshydrogénases EC 1.1.1.71, les alcool aromatique déshydrogénases EC 1.1.1.90 encore appelées aryl alcool déshydrogénases, les alcool aromatique déshydrogénases EC 1.1.1.97, les alcool 3-hydroxybenzylique déshydrogénases EC 1.1.1.97, les alcool coniferylique déshydrogénases EC 1.1.1.194, les alcool cinnamylique déshydrogénases EC 1.1.1.195, les méthanol déshydrogénases EC 1.1.1.244, les alcool aromatique oxydases EC 1.1.3.7 encore appelées aryl alcool oxydases, les alcool oxydases EC 1.1.3.13, les 4-hydroxymandelate oxydases EC 1.1.3.19, les alcool à longue chaîne hydrocarbonée oxydases EC 1.1.3.20, les méthanol oxydases EC 1.1.3.31, les alcool déshydrogénases EC 1.1.99.20, les méthylglutamate déshydrogénases EC 1.5.99.5, les 2-oxo-acides décarboxylases EC 4.1.1.1, les benzoylformate décarboxylases EC 4.1.1.7, les phénylpyruvate décarboxylases EC 4.1.1.43, les threonine aldolase EC 4.1.2.5.

Si elle est présente, la concentration de l'enzyme utilisée dans la composition **(A)** est comprise entre 0,005 % et 40 % en poids par rapport au poids total de la dite composition et de préférence comprise entre 0,05 % et 10 % en poids par rapport au poids de cette composition.

*Sels*

Les compositions **(A),** comprenant le ou les composé(s) de formule **(I), (B), (C), (D),** ou **(E)** peuvent éventuellement comprendre un ou plusieurs sels.

Lorsqu'ils sont présents, ces derniers sont en général choisis parmi les sels organiques et/ou les sels inorganiques, ainsi que leurs combinaisons.

En particulier, les anions composant ces sels peuvent être aussi bien inorganiques (chlorure, carbonate, hydrogénocarbonate, sulfate, hydrogénosulfate, silicate, phosphate, hydrogénophosphate, hydroxyde...), qu'organiques (aspartate, formiate, acétate, lactate, citrate, gluconate, succinate, malate, fumarate, orotate...).

Les cations composant ces sels, associés aux anions ci-dessus, peuvent être issus aussi bien des métaux alcalins (de préférence lithium, sodium, potassium), que de métaux alcalino-terreux (de préférence magnésium, calcium), que de métaux de transition (scandium, titane, vanadium, manganèse, molybdène, fer, cobalt, nickel, cuivre, zinc, argent, or). D'autres cations peuvent aussi former des sels comme les ammoniums. De préférence, les cations seront choisis parmi les métaux alcalins (lithium, sodium, potassium), les métaux alcalino-terreux (magnésium, calcium), les ammoniums, ainsi que les métaux de transition suivants : manganèse, molybdène, fer, cuivre, zinc, argent et l'or.

Lorsqu'ils sont présents, leur teneur représente de 0,001 et 40% en poids par rapport au poids de la composition, et encore plus préférentiellement entre 0,001 et 20% en poids, par rapport au poids de la composition.

*Colorants additionnels*

Les compositions **(A),** comprenant le ou les composé(s) de formule **(I), (B), (C), (D),** ou **(E)** peuvent éventuellement en outre comprendre des colorants additionnels, différents des composés de formule **(I),** précités.

Parmi ces colorants additionnels, on peut citer les colorants directs naturels ou synthétiques, les colorants d'oxydation avec les bases éventuellement associées à des coupleurs, ainsi que leurs combinaisons.

Ces colorants directs peuvent être par exemple choisis parmi les colorants directs nitrés benzéniques neutres, acides ou cationiques, les colorants directs azoïques neutres, acides ou cationiques, les colorants tétraazapentaméthiniques, les colorants quinoniques et en particulier anthraquinoniques neutres, acides ou cationiques, les colorants directs aziniques, les colorants directs triarylméthaniques, les colorants directs indoaminiques et les colorants directs naturels.

Parmi les colorants directs naturels, on peut citer la lawsone, la juglone, l'alizarine, la purpurine, l'acide carminique, l'acide kermésique, la purpurogalline, l'anthragallol, l'indigo, la curcumine, la spinulosine, l'apigénidine, les chlorophylles, les chlorophyllines, les orcéines, l'hématéine, la braziline, la braziléine, les colorants du carthame (comme par exemple la carthamine), les flavonoïdes (avec par exemple la morine, l'apigénidine, le santal), les anthocyanes (du type de l'apigéninidine), les caroténoïdes, les tanins, le sorgho et le carmin de cochenille, ou leurs mélanges. On peut également utiliser les extraits ou décoctions contenant ces colorants naturels et notamment les cataplasmes ou extraits à base de henné. En particulier, la composition (A) peut comprendre au moins un composé additionnel choisi parmi la dihydroxyacétone, le protocatéchaldéhyde, l'isatine, l'hématoxyline, la vitamine C, la vitamine C oxydée et leurs mélanges

Parmi les bases d'oxydation, on peut citer les para-phénylènediamines, les bis-phénylalkylènediamines, les para-aminophénols, les bis-para-aminophénols, les ortho-aminophénols, les bases hétérocycliques et leurs sels d'addition.

Parmi ces coupleurs, on peut notamment citer les méta-phénylènediamines, les méta-aminophénols, les méta-diphénols, les coupleurs naphtaléniques, les coupleurs hétérocycliques et leur sels d'addition.

La ou les bases d'oxydation présentes dans la composition tinctoriale sont en général présentes chacune en quantité comprise entre 0,001 à 10% en poids du poids total de la composition tinctoriale, de préférence entre 0,005 et 6% en poids.

Le ou les coupleurs sont chacun généralement présents en quantité comprise entre 0,001 et 10% en poids du

poids total de la composition **(A),** de préférence entre 0,005 et 6%.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates.

Selon un mode de réalisation, la composition **(A)** comprend au moins un acide de Lewis, en particulier choisi parmi les halogénures de bore et les halogénures d'aluminium.

L'acide de Lewis peut être présente en une teneur allant de 0.001 à 10%, de préférence de 0.01 à 5% en poids par rapport au poids total de la composition **(A).**

Il est à noter que la composition **(A)** mise en oeuvre dans l'invention peut résulter du mélange extemporané de plusieurs compositions comprenant chacune un ou plusieurs premier, deuxième et troisième composés décrits ci-dessus.

*Agent oxydant chimique*

Le procédé de coloration des fibres kératiniques à partir de précurseurs de formule **(I)** tels que définis précédemment met en oeuvre une composition comprenant au moins un agent oxydant chimique. L'invention s'intesse également à une composition cosmétique comprenant au moins un précurseur de formule **(I)** tel que défini précédemment.

Selon un mode de réslisation avantageux de l'invention la composition de l'invention comprend au moins un agent oxydant chimique. En particulier, quand un agent oxydant chimique est présent, la composition prête à l'emploi est avantageusement obtenue par mélange extemporané avant l'application, d'une composition précédemment décrite, avec au moins une composition comprenant un ou plusieurs agents oxydants chimiques.

Par « *agent oxydant chimique* » on entend un agent oxydant différent de l'oxygène de l'air.

L'agent oxydant chimique est choisi de préférence parmi le peroxyde d'hydrogène, le peroxyde d'urée, les bromates ou ferricyanures de métaux alcalins, les sels peroxygénés comme par exemple les persulfates, les perborates et les percarbonates de métaux alcalins ou alcalino-terreux, comme le sodium, le potassium, le magnésium, les sels de métaux de transition choisis parmi les sels de zinc, de cuivre, de manganèse, de fer, sels pouvant être de nature organique ou minérale.

Lorsque les sels de métaux de transition sont des sels d'acide organique, ils peuvent contenir une ou plusieurs fonctions acide carboxylique et/ou acide sulfonique ($-SO_3H$), et/ou acide phosphonique ($-H_2PO_3$), et/ou acide phosphinique ($-H_2PO_2$ ou $=HPO_2$), et/ou acide phosphineux ($=POH$).

De préférence, l'acide organique contient une ou plusieurs fonctions acide carboxylique et/ou sulfonique. L'acide organique selon l'invention peut être saturé ou insaturé, linéaire, ramifié ou cyclique. L'acide organique selon l'invention peut. notamment être choisi parmi le gluconate, le lactate, le glycinate, l'aspartate, le pyrrolidone carboxylate, le phénolsulfonate, le salicylate, le citrate, l'acétate et leurs mélanges.

Au sens de la présente invention, on entend par sels minéraux, des sels inorganiques, c'est-à-dire ne comprenant pas dans leur structure d'atome de carbone lié à au moins un atome d'hydrogène. Les sels minéraux sont des sels issus de l'action d'un acide minéral ou d'une base minérale sur le métal. Parmi les sels, on peut citer les halogénures tels que les chlorure, fluorure et iodure ; les sulfate, phosphate, nitrate, les carbonate, perchlorate ainsi que leurs mélanges. De préférence, les sels minéraux utilisés sont le sulfate, le phosphate et le chlorure de zinc.

Conviennent également des agents oxydants de type enzymatique comme les laccases, les peroxydases et les oxydo-réductases à 2 électrons (telles que l'uricase), le cas échéant en présence de leur donneur ou co-facteurs respectifs.

L'utilisation du peroxyde d'hydrogène est particulièrement préférée.

L'agent oxydant est avantageusement constitué de peroxyde d'hydrogène en solution aqueuse (eau oxygénée) dont le titre peut varier, plus particulièrement, de 1 à 40 volumes, et encore plus préférentiellement de 5 à 40 volumes.

Il est ainsi à noter que la composition mise en oeuvre dans l'invention peut résulter du mélange extemporané de plusieurs compositions.

La composition qui vient d'être décrite est donc appliquée sur les fibres kératiniques humaines, en particulier, les cheveux.

*Autres ingrédients*

Le milieu cosmétiquement acceptable comprend en général au moins de l'eau ou bien encore un mélange d'eau et d'au moins un solvant organique. A titre de solvant organique, on peut par exemple citer les alcanols inférieurs en $C_1$-$C_4$, tels que l'éthanol et l'isopropanol ; les polyols tels que le 1,3 propanediol ou encore le 1,6-hexanediol et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants lorsqu'ils sont présents sont, de préférence présents dans des proportions de préférence comprises entre 1 et 99% en poids par rapport au poids de la composition, et encore plus préférentiellement entre 5 et 95% en poids, par rapport au poids de la composition.

Les compositions mises en oeuvre dans le procédé selon l'invention peut également renfermer divers adjuvants utilisés classiquement dans les compositions pour la coloration des cheveux, tels que des agents tensioactifs anioniques, cationiques, non ioniques, amphotères, zwittérioniques ou leurs mélanges, des polymères anioniques, cationiques, non ioniques, amphotères, zwittérioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non tels que les silicones aminés, des agents filmogènes, des céramides, des agents conservateurs, des agents opacifiants, des polymères conducteurs.

Les adjuvants ci dessus sont en général présents en quantité comprise pour chacun d'eux entre 0,01 et 20% en poids par rapport au poids de la composition.

Bien entendu, l'homme de l'art veillera à choisir ce ou ces éventuels composés complémentaires de manière telle que les propriétés avantageuses attachées intrinsèquement à la composition de teinture conforme à l'invention ne soient pas, ou substantiellement pas, altérées par la ou les adjonctions envisagées.

*pH de la composition :*

Selon un mode de réalisation de l'invention les compositions **(A), (C), (D)** et/ou **(E)** telles que définies ci après présentent un pH inférieur ou égal à 9.5. Le pH de la composition **(A), (C), (D)** et/ou **(E)** est en particulier compris inclusivement entre 3 et 14, de préférence entre 3 et 9.5 et de façon encore plus préférée entre 7 et 9.5.

Parmi les agents acidifiants, on peut citer, à titre d'exemple, les acides minéraux ou organiques comme l'acide chlorhydrique, l'acide orthophosphorique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les amines citées auparavant.

La composition peut se présenter sous des formes diverses, telles que sous forme de liquide, de crème, de gel, de poudres à mélanger avant l'emploi pour obtenir des cataplasmes, des infusions, ou sous toute autre forme appropriée pour réaliser une teinture des fibres kératiniques, et notamment des cheveux.

Selon un mode de réalisation, elle se présente sous forme de poudre. Dans ce cas, elle est additionnée d'eau avant l'application sur les fibres kératiniques.

Les ingrédients de la composition **(A)** précitée peuvent être stockés ensemble ou séparément.

*Dispositifs ou kits :*

L'invention concerne également un dispositif ou kit, à plusieurs compartiments comprenant :

- dans un premier compartiment au moins un composé de formule **(I)** tels que définis précédemment ;
- et dans un deuxième compartiment au moins un agent oxydant chimique tel que défini précédemment ;

étant entendu que dans ce mode de réalisation, les composés du premier compartiment, se présente préférentiellment sous forme de poudre, de préférence anhydre, c'est-à-dire comprenant moins de 5%, de préférence moins de 2% et mieux moins de 1% d'eau.

Selon un mode de réalisation particulier de l'invention, le dispositif à plusieurs compartiments comprend :

- dans un premier compartiment au moins un composé de formule **(I)** tels que définis précédemment ;
- dans un deuxième compartiment au moins un composé nucléophile choisi parmi les amines de formule **(V)** telles que définies précédemment, ou de formule (Va) à **(Vi')** telles que définies précédemment, de préférence ladite amine est choisi parmi l'ammoniaque, les composés des formules **(Va), (Vb), (Vc), (Ve), (Vf), (Vf'), (Vf"), (Vg)** en particulier lorsque $\mathbf{R_{20}}$ représente un groupement alcoxy linéaire en $C_1$-$C_4$, **(Vi'),** ou leurs mélanges , et
- dans un troisième compartiment au moins un composé électrophile choisi parmi les composés (thio)carbonylés de formule **(i), (i'), (ii), (a), (b), (c),** et **(d)** tels que définis précédemment, et de préférence de formule **(i), (i'), (ii), (a)** et **(d)** tels que définis précédemment ; et
- dans un quatrième compartiment au moins un agent oxydant chimique tel que défini précédemment ;

étant entendu que dans ce mode de réalisation, les composés du premier compartiment, et éventuellement du troisième se présente(nt) préférentiellement sous forme de poudre, de préférence anhydre, c'est-à-dire comprenant moins de 5 %, de préférence moins de 2 % et mieux moins de 1 % d'eau.

Selon un autre mode de réalisation particulier de l'invention, le dispositif à plusieurs compartiments comprend :

- dans un premier compartiment au moins un composé de formule **(I)** tels que définis précédemment ;
- dans un deuxième compartiment au moins un composé nucléophile choisi parmi les amines de formule **(V)** telles que définies précédemment, ou de formule **(Va)** à **(Vi')** telles que définies précédemment, de préférence ladite amine est un amino alcool ou aminothiol, et en particulier un β- ou γ- aminoalcool ou aminothiol, de préférence amino alcool, plus particulièrement un amino alcool de formule **(Vf)** ou **(Vf')** plus préférentiellement **(Vf")** tels que définis précédemment, et
- dans un troisième compartiment au moins un composé électrophile choisi parmi les composés (thio)carbonylés de formule **(i), (i'), (ii), (a), (b), (c),** et **(d)** tels que définis précédemment, et de préférence de formule **(i)** ou **(i')** tels que définis précédemment ; et
- dans un quatrième compartiment au moins un agent oxydant chimique tel que défini précédemment ;

étant entendu que dans ce mode de réalisation, les composés du premier compartiment, et éventuellement du troisième se présente(nt) sous forme de poudre, de préférence anhydre, c'est-à-dire comprenant moins de 5%, de préférence moins de 2% et mieux moins de 1% d'eau.

Les composés présents dans les différents compartiments sont mélangés de façon extemporanée avant application sur les fibres kératiniques.

Il peut également être avantageux de stocker au moins le(s) composé(s) de formules du premier compartiment **(I),** en comprenant dans un récipient à l'abri de l'air, par exemple en milieu sous vide ou sous atmosphère inerte telle que l'azote ou l'argon.

*Procédé de coloration*

La présente invention a donc pour premier objet un procédé de coloration des fibres kératiniques de préférence humaines, tels que les cheveux, comprenant l'application sur lesdites fibres :

i) d'au moins une composition cosmétique **(A)** comprenant au moins un précurseur de coloration biomimétique, composé de formule **(I)** tels que définis précédemment ; et
ii) d'au moins une composition cosmétique **(B)** comprenant au moins un agent oxydant chimique tels que définis précédemment ;

étant entendu que les compositions **(A)** et **(B)** peuvent être appliquées simultanément, ou séquentiellement par l'application d'abord de **(A),** puis par l'application de **(B).**

De préférence la composition **(A)** est d'abord appliquée puis est appliqué sur les fibres kératiniques la composition **(B),** plus particulièrement la composition **(B)** étant appliquée après un temps de pause d'au moins 5 minutes après application de la composition **(A).**

Selon un mode de réalisation particulier de l'invention, le procédé de coloration de fibres kératiniques met en oeuvre :

i) une composition **(A)** comprenant un ou plusieurs précurseur(s) de formule **(I)** tels que définis précédemment ; et
ii) une composition **(B)** comprenant au moins un agent oxydant chimique tels que définis précédemment ;
étant entendu que peuvent également être mis en oeuvre dans le procédé de coloration :
iii) une ou plusieurs amine(s) choisie(s) parmi les amines de formule **(V)** telles que définies précédemment, ou de formule **(Va)** à **(Vi')** telles que définies précédemment, de préférence ladite amine est choisi parmi l'ammoniaque, les composés des formules **(Va), (Vb), (Vc), (Ve), (Vf), (Vf'), (Vf"), (Vg)** en particulier lorsque $\mathbf{R_{20}}$ représente un groupement alcoxy linéaire en $C_1$-$C_4$, **(Vi'),** ou leurs mélanges ; et
iv) éventuellement un ou plusieurs composés (thio)carbonylé(s) de formule de formule **(i), (i'), (ii), (a), (b), (c),** et **(d)** tels que définis précédemment, et de préférence de formule **(i), (i'), (ii), (a)** et **(d)** tels que définis précédemment, la ou lesdites amines et le ou lesdits composés (thio)carbonylés pouvant se trouver dans la composition **(A),** ensemble dans une composition cosmétique **(E)** ou dans deux compositions cosmétiques séparées **(C)** et **(D)** ;

- les compositions **(A), (B), (C), (D)** et **(E)** peuvent être appliquées simultanément, ou séquentiellement par l'application d'abord de **(A),** puis éventuellement de **(C), (D)** et enfin par l'application de **(B),** de préférence séquentiellement ; et

- le pH de la composition **(A)** est inférieur à 9,5 et à un pH supérieur ou égal à 7.

Selon un mode de réalisation avantageux du procédé de coloration, le temps de pause après application de la composition **(A)** est compris inclusivement entre 10 minutes et 1 heure.

Selon une mode de réalisation particulier de l'invention, la composition **(A)** du procédé de coloration est appliquée à une température comprise entre 20 et 50°C.

A l'issue du temps de pause, la composition comprenant l'agent oxydant chimique **(B),** de préférence de l'eau oxygénée ou peroxyde d'hydrogène en solution aqueuse dont le titre peut varier, plus particulièrement, de 1 à 40 volumes et encore plus préférentiellemnt de 5 à 40 volumes est appliquée, de préférence à une température ambiante (25 °C), pour révéler la couleur avec un temps de pause compris inclusivement de préférence entre 1 minute et 20 minutes.

Plus specifiquement, l'invention concerne un procédé de coloration mettant en oeuvre les étapes suivantes :

1) application sur les fibres kératiniques de la composition **(A)** telle que définie précédemment possédant un pH compris entre 7 et 9,5, avec un temps de pause compris inclusivement entre 10 minutes et 1 heure à une température comprise inclusivement entre 20 et 50 °C ; puis

2) application sur lesdites fibres d'une composition **(C)** comprenant au moins une amine de formule **(V)** telles que définies précédemment, ou de formule **(Va)** à **(Vi')** telles que définies précédemment, de préférence ladite amine est choisi parmi l'ammoniaque, les composés des formules **(Va), (Vb), (Vc), (Ve), (Vf), (Vf'), (Vf"), (Vg)** en particulier lorsque $\mathbf{R_{20}}$ représente un groupement alcoxy linéaire en $C_1$-$C_4$, **(Vi'),** ou leurs mélanges ; et éventuellement un ou plusieurs composés (thio)carbonylé(s) de formule de formule **(i), (i'), (ii), (a), (b), (c),** et **(d)** tels que définis précédemment, et de préférence de formule **(i), (i'), (ii), (a)** et **(d)** tels que définis précédemment, ladite composition **(C)** possédant un pH compris entre 7 et 9,5, avec un temps de pause compris inclusivement entre 30 minutes et 1 heure à une température comprise inclusivement entre 20 et 50 °C, puis

3) révélation de la couleur par application sur lesdites fibres de la composition **(B)** telle que définie précédemment, en particulier d'une composition oxydante comprenant de l'eau oxygénée en solution aqueuse dont le titre peut varier, plus particulièrement, de 1 à 40 volumes et encore plus préférentiellement de 5 à 40 volumes.

Selon une variante avantageuse le procédé de coloration de l'invention met en oeuvre les étapes suivantes :

1) application sur les fibres kératiniques de la composition **(A)** comprenant :

- au moins un précurseur de formule **(I)** tels que définis précédemment et
- au moins une ou plusieurs amines de formule **(V)** telles que définies précédemment, ou de formule **(Va)** à **(Vi')** telles que définies précédemment, de préférence ladite amine est choisi parmi l'ammoniaque, les composés des formules **(Va), (Vb), (Vc), (Ve), (Vf), (Vf'), (Vf"), (Vg)** en particulier lorsque $\mathbf{R_{20}}$ représente un groupement alcoxy linéaire en $C_1$-$C_4$, **(Vi'),** ou leurs mélanges ; ladite composition **(A)** possédant un pH compris entre 7 et 9,5, avec un temps de pause compris inclusivement entre 10 minutes et 1 heure à une température comprise inclusivement entre 20 et 50 °C ; puis

2) application sur lesdites fibres d'une composition **(D)** comprenant au moins un composé (thio)carbonylé de formule **(i), (i'), (ii), (a), (b), (c),** et **(d)** tels que définis précédemment, et de préférence de formule **(i), (i'), (ii), (a)** et **(d)** tels que définis précédemment,, ladite composition **(D)** possédant un pH compris entre 7 et 9,5, avec un temps de pause compris inclusivement entre 30 minutes et 1 heure à une température comprise inclusivement entre 20 et 50 °C, puis

3) révélation de la couleur par application sur lesdites fibres de la composition **(B)** telle que définie précédemment, en particulier d'une composition oxydante comprenant en solution aqueuse dont le titre peut varier, plus particulièrement, de 1 à 40 volumes et encore plus préférentiellement de 5 à 40 volumes.

Selon une autre variante avantageuse le procédé de coloration de l'invention met en oeuvre les étapes suivantes :

1) application sur les fibres kératiniques de la composition **(A)** telle que définie précédemment possédant un pH compris entre 7 et 9,5, avec un temps de pause compris inclusivement entre 10 minutes et 1 heure à une température comprise inclusivement entre 20 et 50 °C ; puis

2) application sur lesdites fibres d'une composition **(C)** comprenant au moins une amine de formule **(V)** telle que définie précédemment, ou de formule **(Va)** à **(Vi')** telles que définies précédemment, de préférence ladite amine est choisi parmi l'ammoniaque, les composés des formules **(Va), (Vb), (Vc), (Ve), (Vf), (Vf'), (Vf"), (Vg)** en particulier lorsque $\mathbf{R_{20}}$ représente un groupement alcoxy linéaire en $C_1$-$C_4$, **(Vi'),** ou leurs mélanges ; ladite composition **(C)** possédant un pH compris entre 7 et 9,5, avec un temps de pause compris inclusivement entre 30 minutes et 1

heure à une température comprise inclusivement entre 20 et 50 °C, puis
3) application sur lesdites fibres d'une composition **(D)** comprenant au moins un composé (thio)carbonylé de formule **(i), (i'), (ii), (a), (b), (c),** et **(d)** tels que définis précédemment, et de préférence de formule **(i), (i'), (ii), (a)** et **(d)** tels que définis précédemment, ladite composition **(D)** possédant un pH compris entre 7 et 9,5, avec un temps de pause compris inclusivement entre 30 minutes et 1 heure à une température comprise inclusivement entre 20 et 50 °C, puis
4) révélation de la couleur par application sur lesdites fibres de la composition **(B)** telle que définie précédemment, en particulier d'une composition oxydante comprenant de l'eau oxygénée en solution aqueuse dont le titre peut varier, plus particulièrement, de 1 à 40 volumes et encore plus préférentiellement de 5 à 40 volumes.

Selon une autre variante avantageuse le procédé de coloration de l'invention met en oeuvre les étapes suivantes :

1) application sur les fibres kératiniques de la composition **(A)** telle que définie précédemment possédant un pH compris entre 7 et 9,5, avec un temps de pause compris inclusivement entre 10 minutes et 1 heure à une température comprise inclusivement entre 20 et 50 °C ; puis
2) application sur lesdites fibres d'une composition **(E)** comprenant :

- au moins une amine de formule **(V)** telles que définies précédemment, ou de formule **(Va)** à **(Vi')** telles que définies précédemment, de préférence ladite amine est choisi parmi l'ammoniaque, les composés des formules **(Va), (Vb), (Vc), (Ve), (Vf), (Vf'), (Vf"), (Vg)** en particulier lorsque $\mathbf{R_{20}}$ représente un groupement alcoxy linéaire en $C_1$-$C_4$, **(Vi'),** ou leurs mélanges;
- au moins un composé (thio)carbonylé de formule **(i), (i'), (ii), (a), (b), (c),** et **(d)** tels que définis précédemment, et de préférence de formule **(i), (i'), (ii), (a)** et **(d)** tels que définis précédemment, ladite composition **(E)** possédant un pH compris entre 7 et 9,5, avec un temps de pause compris inclusivement entre 30 minutes et 1 heure à une température comprise inclusivement entre 20 et 50 °C, puis

3) révélation de la couleur par application sur lesdites fibres de la composition (B) telle que définie précédemment, en particulier d'une composition oxydante comprenant de l'eau oxygénée en solution aqueuse dont le titre peut varier, plus particulièrement, de 1 à 40 volumes et encore plus préférentiellement de 5 à 40 volumes.

Selon une autre variante avantageuse le procédé de coloration de l'invention met en oeuvre les étapes suivantes :

1) application sur les fibres kératiniques de la composition **(A)** comprenant :

- au moins un précurseur de formule **(I)** tels que définis précédemment et
- au moins une amine de formule **(V)** telles que définies précédemment, ou de formule **(Va)** à **(Vi')** telles que définies précédemment, de préférence ladite amine est choisi parmi l'ammoniaque, les composés des formules **(Va), (Vb), (Vc), (Ve), (Vf), (Vf'), (Vf"), (Vg)** en particulier lorsque $\mathbf{R_{20}}$ représente un groupement alcoxy linéaire en $C_1$-$C_4$, **(Vi'),** ou leurs mélanges ; ou de la vaniline,
- au moins un composé (thio)carbonylé de formule **(i), (i'), (ii), (a), (b), (c),** et **(d)** tels que définis précédemment, et de préférence de formule **(i), (i'), (ii), (a)** et **(d)** tels que définis précédemment ;

ladite composition **(A)** possédant un pH compris entre 7 et 9,5, avec un temps de pause compris inclusivement entre 10 minutes et 1 heure à une température comprise inclusivement entre 20 et 50 °C ; puis
2) révélation de la couleur par application sur lesdites fibres de la composition **(B)** telle que définie précédemment, en particulier d'une composition oxydante comprenant de l'eau oxygénée en solution aqueuse dont le titre peut varier, plus particulièrement, de 1 à 40 volumes et encore plus préférentiellement de 5 à 40 volumes.

Selon un mode de réalisation particulièrement avantageux les compositions **(A), (C), (D)** et/ou **(E)** se trouvent sous forme de poudre de préférence anhydres prêtes à l'emploi ou alors disposées dans plusieurs compartiments si l'amine utilisée et/ou le composé (thio)carbonylé et plus généralement le dérivé carbonylé est (sont) liquide(s).
Plus particulièrement la ou les compositions mises en oeuvre dans le procédé de coloration et dans le dispositif à plusieurs compartiments peuvent se présenter comme suit :

a- Tous les ingrédients sonts solides i.e. les précurseurs de formule **(I)** tels que définis précédemment, et les amines solides de formule **(V)** telles que définies précédemment, ou de formule **(Va)** à **(Vi')** telles que définies précédemment ou les amines rendue solides, les composés (thio)carbonylés de formule **(i), (i'), (ii), (a), (b), (c),** et **(d)** tels que

définis précédemment, rendus éventuellement solide par encapsulation dans une matrice, sel inorganique pour ajuster le pH auquel cas tous les ingrédients solides peuvent se trouver dans le même compartiment, et la composition (B) dans un autre compartiment ;

b- Les précurseurs de formule **(I)** tels que définis précédemment, et les amines de formule **(V)** telles que définies précédemment, ou de formule **(Va)** à **(Vi')** telles que définies précédemment sont solides et les composés (thio)carbonylés de formule **(i), (i'), (ii), (a), (b), (c),** et **(d)** tels que définis précédemment sont liquides, auquel cas tous les ingrédients solides se trouvent dans le même compartiment, et la composition **(B)** dans un autre compartiment le dispositif comprend alors au moins trois compartiments ;

c- Les précurseurs de formule **(I)** tels que définis précédemment, et les composés (thio)carbonylés de formule **(i), (i'), (ii), (a), (b), (c),** et **(d)** tels que définis précédemment sont solides et les amines de formule **(V)** telles que définies précédemment, ou de formule **(Va)** à **(Vi')** telles que définies précédemment sont liquides, auquel cas tous les ingrédients solides se trouvent dans le même compartiment, et la composition **(B)** dans un autre compartiment le dispositif comprend alors au moins trois compartiments ;

Dans chacun des cas, l'agent oxydant, en particulier l'eau oxygénée sera stockée à part.

La température d'application de la composition **(B)** précédemment décrite est comprise entre 15°C et 80°C, de façon encore plus préférée entre 15°C et 55°C. La seconde composition formulant le ou les oxydant(s) est appliquée à une température comprise entre 15°C et 30°C.

Selon un mode de réalisation particulier de l'invention, le temps d'application des deux, trois ou quatre compositions est compris entre 10 minutes et 5 heures, de préférence entre 10 minutes et 3 heures et de façon encore plus préférée entre 30 minutes et 2 heures.

Les exemples ci-dessous illustrent l'invention sans en limiter la portée. Tous les composés ont été identifiés par methodes spectroscopique ou spectrométriques classiques.

L'évaluation de la coloration peut être réalisée visuellement ou mesurée par spectrocolorimétrie (tel qu'avec un Minolta CM3600d, illuminat D65, angle 10°, SCI values) pour la mesure des L*, a* et b*. Dans le sytème L*, a* et b*, l* représente l'intensité de la couleur, a* représente l'axe du vert et rouge, b* reprsente l'axe du bleu et jaune. Plus la valeur du L* est faible, plus l'intensité est forte de la couleur, et plus la couleur apparait sombre. Plus la valeur de a* est élevée plus on observera des reflet rouge, et plus la valeur de b* est élevée plus on verra des reflets jaunes. La variation entre la couleur de la mèche blanc naturel non traitée et celle de la mèche colorée est définie par DE* selon la formule suivante :

$$\Delta E^* = \sqrt{(L^* - L_o^*)^2 + (a^* - a_o^*)^2 + (b^* - b_o^*)^2}$$

Dans cette equation, L*, a* et b* sont les valeurs mesurée de la mèche une fois colorée et $L^*_0$, $a^*_0$ et $b^*_0$ sont celles de la mèche Blanc Naturel à 90 % (BN90 %) blanc, non traitée. Plus la valeur est élevée plus la montée de la couleur est grande.

**Exemples 1 - composé (1)**

Schéma de synthèse :

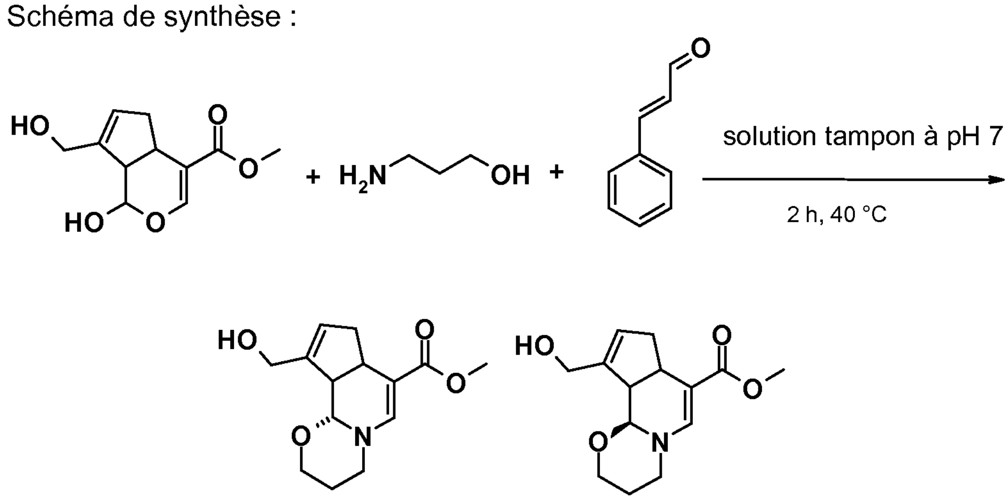


83/17

Dans un ballon de 50ml on introduit 339 mg de génipine (1,5 mmol, 1 éq), 30ml de solution aqueuse tamponnée à un pH de 7. La solution est mise sous agitation. 337 µl de cinnamaldéhyde (3 mmol, 2 éq) et 225 µl de 3-amino-1-propanol (3 mmol, 2 éq) sont ajoutés au milieu réactionnel précédent. Après 2 heures d'agitation à 40 °C, le milieu réactionnel est extrait avec 2x50 ml de dichlorométhane. La phase organique est ensuite lavée avec 50 ml d'une solution saturée de NaCl. La phase organique est ensuite séchée sur sulfate de sodium. Après évaporation à sec sous vide à 40 °C, un résidu bleu est obtenu.

Celui-ci est ensuite purifié sur colonne de silice gradient d'éluant dicloromethane/ méthanol 100/0 puis 80/20 pour conduire après purification à un résidu bleu vert.

Les analyses spectroscopiques RMN et de masse confirment la structure du produit obtenu.

**Exemple 2 - composé (2) : synthèse du méthyl (9bR)-9-(hydroxymethyl)-2-methyl-2,3,6a,7,9a,9b-hexahydrocyclopenta[c][1,3]oxazolo[3,2-a]pyridine-6-carboxylate**

Schéma de synthèse :

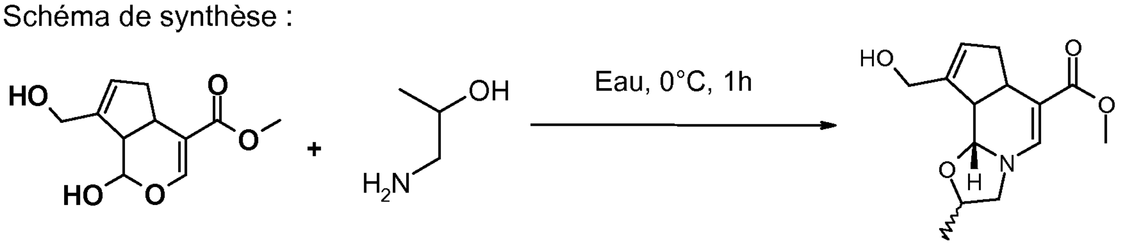


65/35

Dans un ballon, de 100 ml, on introduit à 0°C, 2 g (8,841mmol, 1eq) de génipine dans 4 ml d'eau puis 0.681 ml d'amino-2-propanol (8,841 mmol, 1eq) est ajouté. On ajoute ensuite petit à petit 6 ml d'eau.

Après 1h de réaction à 0°C, le milieu réactionnel de couleur orange est ramené à température ambiante. Ce dernier est ensuite filtré.

300 mg de solide blanc légèrement orangé sont obtenus.

Les analyses RMN et Spectrométtrie de masse sont compatibles avec la structure attendue. On observe un mélange de diastéréisomères.

**Exemples de coloration**

Chaque composition de coloration formulant les composés (1) et (2) est obtenue en dissolvant dans une solution aqueuse, au sonicateur pendant 5 minutes, 10 mg de composé (1) et (2) de l'invention, éventuellement un composé électrophile tel qu'un aldéhyde.

Chaque composition est appliquée sur des cheveux gris naturels à 90% de cheveux blancs à 40°C, avec un temps de pose d'1 heure. Par la suite, la mèche est ensuite rincée, lavée avec un shampoing et séchée au sèche-cheveux.
Les 10 compositions du tableau ci-dessous ont été réalisées et testées sur cheveux :

| **Compositions** | **1** | **2** |
|---|---|---|
| Composé 1 | 10 mg | - |
| Composé 2 | - | 10 mg |
| vaniline | 10 mg | 10 mg |
| Solution aqueuse* | 2 ml | 2 ml |
| *La solution* aqueuse est constituée de 15 % d'éthanol et 5 % d'alcool benzylique et 80 % d'eau.* | | |

Les colorations obtenues sont jaunes, très esthétiques, avec une bonne montée. La cosmétique des fibres est respectée au touché.

## Revendications

1. Procédé de coloration des fibres kératiniques de préférence humaines, tels que les cheveux, comprenant l'application sur lesdites fibres :

    i) d'au moins une composition **(A)** comprenant au moins un composé de formule **(I)** suivante :

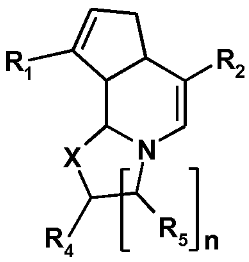

formule **(I)**

ainsi que ses isomères optiques ou géométriques, ses tautomères, ses sels d'acide ou de base, minéral ou organique, leurs solvates tels que les hydrates ;
formule **(I)** dans laquelle :

- **$R_1$** représente i) un groupe ($C_1$-$C_6$)alkyle, linéaire ou ramifié, tel que méthyle

    - $CH_3$, ii) -($C_1$-$C_6$)alk-O-H de préférence -$CH_2$-O-H avec ($C_1$-$C_6$)alk représente un groupe alkylène, linéaire ou ramifié en $C_1$-$C_6$ ;

- **$R_2$** représente un atome ii) un groupe -C(O)-R avec R représente un atome d'hydrogène ou un groupe ($C_1$-$C_4$)alkyle tel que -C(O)-$CH_3$, iii) carboxy

    - C(O)-OH, iv) -C(O)-O-($C_1$-$C_6$)alkyl, v) (di)($C_1$-$C_6$)(alkyl)aminocarbonyle tel que
    - C(O)-$NH_2$ ou -C(O)-N(H)-($C_1$-$C_6$)alkyl : de préférence -C(O)-O-($C_1$-$C_6$)alkyl ;

- **$R_4$** représente un atome d'hydrogène, ii) un radical alkyle en $C_1$-$C_4$ linéaire ou ramifié, ou iii) un radical benzyle ; et
- **$R_5$** représente i) un atome d'hydrogène, ii) un groupe alkyle, linéaire ou ramifié, en $C_1$-$C_6$ éventuellement substitué par un groupe hydroxy, iii) un radical benzyle, iv) un radical hydroxycarbonyle -C(O)OH, v) un radical -C(O)-O-($C_1$-$C_{12}$)alkyl ; en particulier $R_4$ et $R_5$ représentent un atome d'hydrogène;
- **X** représente un atome d'oxygène ;
- **n** vaut 1 ou 2 ;

ii) d'au moins une composition **(B)** comprenant au moins un agent oxydant chimique ; les compositions **(A)** et **(B)** pouvant être appliquées simultanément, ou séquentiellement par l'application d'abord de **(A),** puis par l'application de **(B),** de préférence la composition **(A)** est d'abord appliquée puis est appliqué sur lesdites fibres la composition **(B),** après un temps de pause d'au moins 5 minutes après application de la composition **(A).**

**2.** Procédé de coloration selon la revendication précédente dans lequel le composé de formule : (I) est choisi parmi:

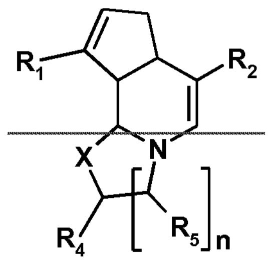

~~formule **(I')**~~

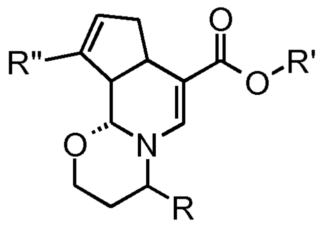

(A)

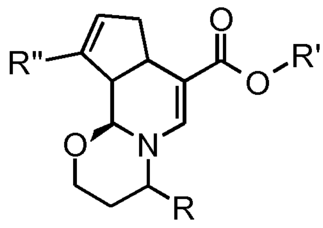

(B)

et

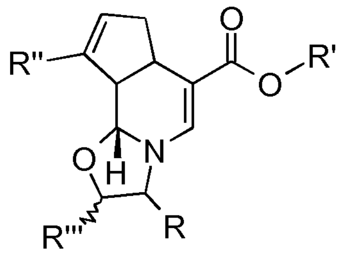

(C)

ainsi que leurs isomères optiques ou géométriques, leurs tautomères, leurs sels d'acide ou de base, minéral ou organique, leurs solvates tels que les hydrates ; Formules **(A), (B)** ou **(C)** dans lesquelles :

- **R** représente un atome d'hydrogène ou un groupe carboxy ;
- **R'** représente un atome d'hydrogène ou un groupe $(C_1-C_6)$alkyle ;
- **R"** représente un groupe $(C_1-C_6)$alkyle éventuellement substitué par au moins un groupe hydroxy ; et
- **R'"** représente un atome d'hydrogène ou un groupe $(C_1-C_6)$alkyle ; de préférence les composés de formule **(I)** ou **(I')** sont choisis parmi :

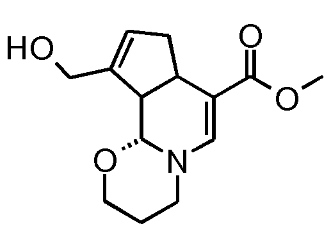

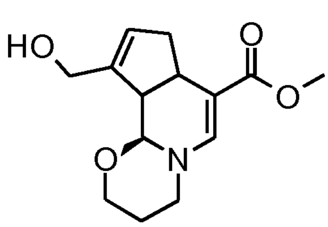

et

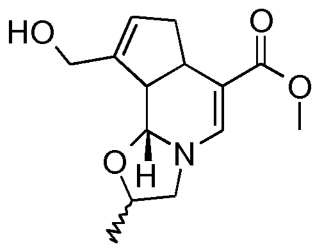

.

**3.** Procédé de coloration selon une quelconque des revendications précédentes qui met en oeuvre en outre iii) une ou plusieurs amine(s) choisie(s) parmi les amines de formule **(V)** :

$$R'_7R'_8NH \qquad (V)$$

formule **(V)** dans laquelle
**$R'_7$, $R'_8$,** représentent indépendamment l'un de l'autre

- un atome d'hydrogène

- un radical hydrocarboné en $C_1$-$C_{20}$, linéaire, ramifié et/ou cyclique, saturé et/ou insaturé, aromatique ou non, pouvant contenir de 1 à 5 doubles liaisons carbone-carbone et/ou éventuellement substitué, éventuellement interrompu par un ou plusieurs hétéroatomes et/ou par un ou plusieurs groupements comprenant au moins un hétéroatome ou groupement comprenant au moins un hétéroatome (de préférence choisi parmi l'oxygène, l'azote, le soufre, C=O, C=S, S(O), $S(O)_2$ ou leurs combinaisons) ; lesdits radicaux R'$_7$ et R'$_8$ hydrocarbonés pouvant éventuellement former avec l'atome d'azote auquel chacun est rattaché, un hétérocycle à 5 ou 7 chaînons, saturé ou insaturé, éventuellement substitué, éventuellement aromatique, éventuellement condensé à un noyau aromatique ou hétéroaromatique à 6 chaînons, comprenant éventuellement un autre hétéroatome identique ou différent de l'azote; le radical hydrocarboné ne comportant pas de fonction nitro, nitroso, peroxo ou diazo.

**4.** Procédé de coloration selon une quelconque des revendications précédentes qui met en oeuvre en outre iii) une ou plusieurs amine(s) choisie(s) parmi les amines de formule **(Va)** à **(Vi')** suivantes, ainsi que leurs sels d'addition à un acide ou à une base, minérale ou organique :

◦ **les acides aminés de formule générale (Va)** :

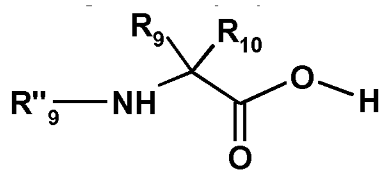

**(Va)**

Formule **(Va)** dans laquelle :

- **R$_9$** représente un atome d'hydrogène un radical alkyle en $C_1$-$C_6$ linéaire ou ramifié, de préférence substitué par un ou plusieurs groupements hydroxyle, hydroxycarbonyle, thiol, ($C_1$-$C_4$)alkylthio, amido, amino, guanidine, un radical phényle, éventuellement substitué par un ou plusieurs hydroxyle, un radical indolyle éventuellement substitué par un ou plusieurs hydroxyle, un radical imidazolyle, un radical pyrrolinyle éventuellement substitué par un groupement alkyle en $C_1$-$C_2$ ; ou un radical phényle non substitué ;
- **R"$_9$** représente un hydrogène, un radical alkyle en $C_1$-$C_4$, ou un radical phényle non substitué ;
- **R$_{10}$** représente un hydrogène ou un radical alkyle en $C_1$-$C_4$ ;
- **R"$_9$** et **R$_9$** pouvant former ensemble avec l'atome d'azote auquel ils sont rattachés, un hétérocycle à 5 ou 6 chaînons saturé ;

◦ **les esters issus d'acides aminés et/ou dérivés de formule générale (Vb)** :

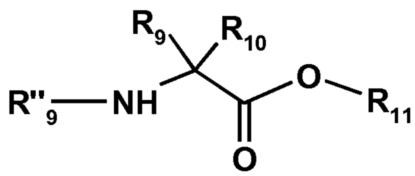

**(Vb)**

formule **(Vb)** dans laquelle :

- **R$_9$** représente un atome d'hydrogène un radical alkyle en $C_1$-$C_6$ linéaire ou ramifié, de préférence substitué par un ou plusieurs groupements hydroxyle, hydroxycarbonyle, ($C_1$-$C_4$)alcoxycarbonyle, thiol, ($C_1$-$C_4$)alkylthio, amido, amino, guanidine, un radical phényle, éventuellement substitué par un ou plusieurs hydroxyle, un radical indolyle éventuellement substitué par un ou plusieurs hydroxyle, un radical imidazolyle, un radical pyrrolinyle éventuellement substitué par un groupement alkyle en $C_1$-$C_2$ ; ou un radical phényle non substitué ;
- **R"$_9$** représente un hydrogène, un radical alkyle en $C_1$-$C_4$, ou un radical phényle non substitué ;

- $\mathbf{R_{10}}$ représente un hydrogène ou un radical alkyle en $C_1$-$C_4$ ;
- $\mathbf{R''_9}$ et $\mathbf{R_9}$ pouvant former ensemble avec l'atome d'azote auquel ils sont rattachés, un hétérocycle à 5 ou 6 chaînons saturé ;
- $\mathbf{R_{11}}$ représente :

  ▪ un radical hydrocarboné en $C_1$-$C_{18}$ linéaire ou ramifié, saturé ou insaturé et comprenant éventuellement de une à 5 doubles liaisons carbone-carbone conjuguées ou non, éventuellement substitué comme indiqué précédemment, éventuellement interrompu par un ou plusieurs hétéroatomes et/ou par un ou plusieurs groupements comprenant au moins un hétéroatome, de préférence choisis parmi l'oxygène, l'azote, le soufre, C=O, C=S, S(O), $S(O)_2$ ou leurs combinaisons ; le radical alkyle ne comportant pas de fonction nitro, nitroso, peroxo ou diazo ;
  ▪ un radical benzyle non substitué ;

◦ **les amides et les thioesters issus d'acides aminés et/ou dérivés de formule générale (Vc)** :

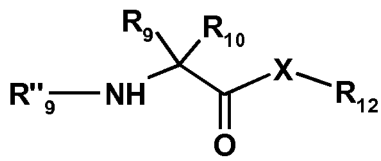

**(Vc)**

Formule **(Vc)** dans laquelle :

- $\mathbf{R_9}$ représente un atome d'hydrogène, un radical alkyle en $C_1$-$C_6$, linéaire ou ramifié, de préférence substitué par un ou plusieurs groupements hydroxyle, alcoxy($C_1$-$C_4$)carbonyle, hydroxycarbonyle, thiol, ($C_1$-$C_4$)alkylthio, amido, amino, guanidine, un radical phényle, éventuellement substitué par un ou plusieurs hydroxyle, un radical indolyle éventuellement substitué par un ou plusieurs hydroxyle, un radical imidazolyle, un radical pyrrolinyle éventuellement substitué par un groupement alkyle en $C_1$-$C_2$ ;
- $\mathbf{R''_9}$ représente un hydrogène ou un radical alkyle en $C_1$-$C_4$ éventuellement substitué par un radical hydroxysulfonyle ;
- $\mathbf{R_{10}}$ représente un hydrogène ou un radical alkyle en $C_1$-$C_4$ ;
- $\mathbf{R''_9}$ et $\mathbf{R_9}$ pouvant former ensemble avec l'atome d'azote auquel ils sont rattachés, un hétérocycle à 5 chaînons saturé ;
- $\mathbf{R_{12}}$ représente :

  *un atome hydrogène
  * un radical alkyle en $C_1$-$C_6$, de préférence substitué par un ou plusieurs groupements hydroxyle, thiol, ($C_1$-$C_4$)alkylthio, amido, amino, un radical phényle, éventuellement substitué par un ou plusieurs hydroxyle, un radical indolyle éventuellement substitué par un ou plusieurs hydroxyle, un radical imidazolyle, un radical pyrrolinyle éventuellement substitué par un groupement alkyle en $C_1$-$C_2$ ;

- **X** représente un atome de soufre ou d'azote ;

◦ **les composés aminés de formule générale (Vd)** :

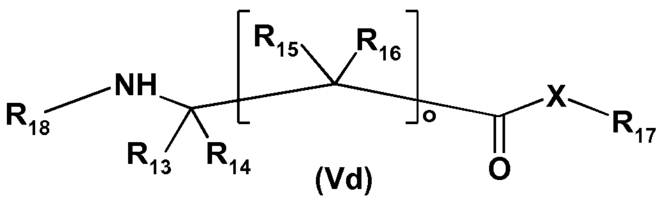

**(Vd)**

Formule **(Vd)** dans laquelle :

- **$R_{13}$, $R_{14}$, $R_{15}$, $R_{16}$** représentent indépendamment les uns des autres :

*un atome hydrogène
*un radical hydrocarboné en $C_1$-$C_{20}$, linéaire, ramifié et/ou cyclique, saturé et/ou insaturé, pouvant contenir de 1 à 5 doubles liaisons carbone-carbone, éventuellement aromatique, éventuellement substitué comme indiqué précédemment, éventuellement interrompu par un ou plusieurs hétéroatomes et/ou par un ou plusieurs groupements comprenant au moins un hétéroatome, de préférence choisis parmi l'oxygène, l'azote, le soufre, C=O, C=S, S(O), $S(O)_2$ ou leurs combinaisons, éventuellement porteurs d'au moins un groupement hydroxyle ou alcoxy en $C_1$-$C_2$, lesdits radicaux alkyle $R_{13}$ et $R_{14}$ ou $R_{14}$ et $R_{15}$ ou $R_{15}$ et $R_{16}$ pouvant éventuellement former avec l'atome de carbone auquel chacun est rattaché, un hétérocycle à 5 ou 7 chaînons, saturé ou insaturé, éventuellement substitué comme indiqué précédemment, éventuellement aromatique, comprenant éventuellement un autre hétéroatome identique ou différent de l'azote; le radical alkyle ne comportant pas de fonction nitro, nitroso, peroxo ou diazo ; plus particulièrement un radical alkyle en $C_1$-$C_{10}$, éventuellement substitué ; et de préférence, un radical alkyle en $C_1$-$C_8$ linéaire ou ramifié éventuellement substitué par au moins un groupement hydroxyle, de préférence de 1 à 2 groupements hydroxyle, un radical hydroxycarbonyle, un radical ureido, un radical alkoxy($C_1$-$C_4$)carbonyle ; un radical phenyle non substitué ;

- **X** représente un atome d'azote, d'oxygène ou de soufre.
- **$R_{17}$** représente :

*un atome hydrogène
*un radical hydrocarboné en $C_1$-$C_{18}$ linéaire ou ramifié, saturé ou insaturé et comprenant éventuellement de une à 5 doubles liaisons carbone-carbone conjuguées ou non, éventuellement substitué comme indiqué précédemment, éventuellement interrompu par un ou plusieurs hétéroatomes et/ou par un ou plusieurs groupements comprenant au moins un hétéroatome, de préférence choisis parmi l'oxygène, l'azote, le soufre, C=O, C=S, S(O), $S(O)_2$ ou leurs combinaisons ; le radical alkyle ne comportant pas de fonction nitro, nitroso, peroxo ou diazo ; plus particulièrement $R_{17}$ représente un hydrogène, un radical alkyle en $C_1$-$C_{10}$, linéaire ou ramifié, éventuellement substitué ; et de préférence, un hydrogène, un radical alkyle en $C_1$-$C_4$ linéaire ou ramifié éventuellement substitué par au moins un groupement hydroxyle, de préférence de 1 à 2 groupements hydroxyle ;

- **$R_{18}$** représente :

*un atome d'hydrogène
*un radical alkyle en $C_1$-$C_8$ linéaire ou ramifié éventuellement substitué comme indiqué précédemment, éventuellement interrompu par un ou plusieurs hétéroatomes et/ou par un ou plusieurs groupements comprenant au moins un hétéroatome, de préférence choisis parmi l'oxygène, l'azote, le soufre, CO, C=S, SO, $SO_2$ ou leurs combinaisons, éventuellement porteurs d'au moins un groupement hydroxyle ou alcoxy en $C_1$-$C_2$ ; le radical alkyle ne comportant pas de fonction nitro, nitroso, peroxo ou diazo,

- **o** est un entier compris inclusivement entre 0 et 5 ;

◦ **les composés aminés de formule générale (Ve)** :

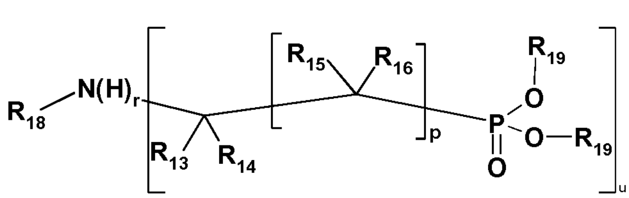

**(Ve)**

Formule **(Ve)** dans laquelle :

- **$R_{13}$, $R_{14}$, $R_{15}$, $R_{16}$** et **$R_{18}$** ont la même signification que précédemment pour la formule (Vd) ;
- **$R_{19}$** représente :

*un atome d'hydrogène
*un radical alkyle en $C_1$-$C_8$ linéaire ou ramifié éventuellement substitué comme indiqué précédemment, éventuellement interrompu par un ou plusieurs hétéroatomes et/ou par un ou plusieurs groupements comprenant au moins un hétéroatome, de préférence choisis parmi l'oxygène, l'azote, le soufre, C=O, C=S, S(O), $S(O)_2$ ou leurs combinaisons, éventuellement porteurs d'au moins un groupement hydroxyle ou alcoxy en $C_1$-$C_2$ ; le radical alkyle ne comportant pas de fonction nitro, nitroso, peroxo ou diazo,

- **p** est un entier compris inclusivement entre 0 et 7 ;
- **u** est un entier égal à 1 ou 2, étant entendu que lorsque u vaut 2 alors le radical $R_{18}$ représente un atome d'hydrogène,
- **r** vaut 0 ou 1, étant étendu que lorsque u vaut 1 alors r vaut 1 et lorsque u vaut 2, alors r vaut 0 ;

◦ **les composés aminés de formule générale (Vf)** :

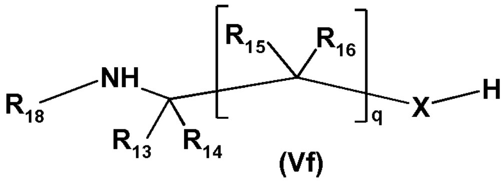

**(Vf)**

Formule **(Vf)** dans laquelle :

- **$R_{13}$, $R_{14}$, $R_{15}$, $R_{16}$** et **$R_{18}$** ont la même signification que précédemment ; en outre, les radicaux **$R_{13}$, $R_{14}$, $R_{15}$** et **$R_{16}$** indépendamment les uns des autres peuvent aussi représenter un radical hydroxy, un radical ($C_1$-$C_4$)alkoxycarbonyle, un radical carboxaldéhyde, un ($C_1$-$C_3$)alkoxy ;
- **q** est un entier compris incusivement entre 1 et 18 ;
- **X** représente, un atome d'oxygène, ou de soufre, un groupement méthylène éventuellement substitué par un radical hydroxy ; de préférence X représente un atome d'oxygène ;
- étant entendu que lorsque **X** représente un atome d'oxygène, alors **$R_{18}$** forme un cycle à 5 ou 6 chaînons éventuellement substitué par un ou plusieurs hydroxy(méthyl), de préférence de 1 à 4 groupements hydroxy(méthyle) ;

◦ **les composés aminés de formule générale (Vg)** :

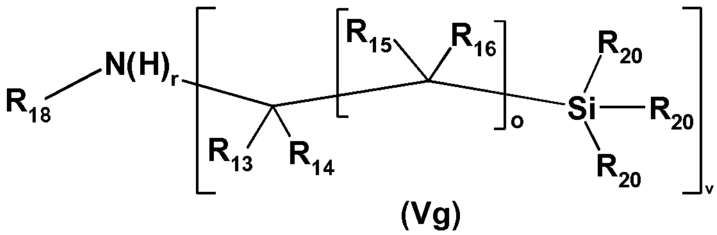

**(Vg)**

Formule **(Vg)** dans laquelle :

- **$R_{13}$, $R_{14}$, $R_{15}$, $R_{16}$** et **$R_{18}$** ont la même signification que précédemment ;
- **$R_{20}$** représente

*un radical alkyle linéaire en $C_1$-$C_4$
*un radical alcoxy linéaire en $C_1$-$C_4$

- ◦ est un entier compris inclusivement entre 0 et 5 ;
- **v** est un entier valant 1 ou 2, étant entendu que lorsque v vaut 2 alors $R_{18}$ représente un hydrogène ;
- **r** vaut 0 ou 1, étant étendu que lorsque v vaut 1 alors r vaut 1 et lorsque v vaut 2, alors r vaut 0 ;

◦ **les composés aminés de formule générale (Vh)** :

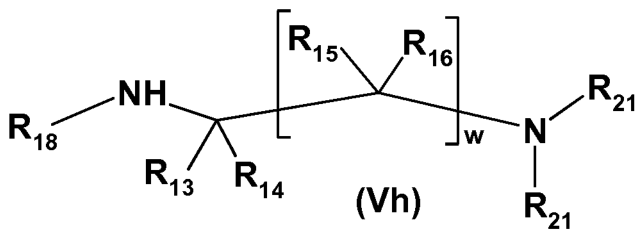

(Vh)

Formule **(Vh)** dans laquelle :

- $\mathbf{R_{13}}$, $\mathbf{R_{14}}$, $\mathbf{R_{15}}$, $\mathbf{R_{16}}$ et $\mathbf{R_{18}}$ ont la même signification que précédemment. En outre, les radicaux $\mathbf{R_{13}}$, $\mathbf{R_{14}}$, $\mathbf{R_{15}}$ et $\mathbf{R_{16}}$ indépendamment les uns des autres peuvent aussi représenter un radical hydroxy, un radical ($C_1$-$C_4$)alcoxycarbonyl, un radical carboxaldéhyde, un radical ($C_1$-$C_3$)alkoxy ;
- $\mathbf{R_{21}}$ et $\mathbf{R_{22}}$ représentent indépendamment l'un de l'autre :

  *un atome d'hydrogène
  *un radical hydrocarboné en $C_1$-$C_{20}$, linéaire, ramifié et/ou cyclique, saturé et/ou insaturé, pouvant contenir de 1 à 5 doubles liaisons carbone-carbone, éventuellement substitué comme indiqué précédemment, éventuellement interrompu par un ou plusieurs hétéroatomes et/ou par un ou plusieurs groupements comprenant au moins un hétéroatome, de préférence choisis parmi l'oxygène, l'azote, le soufre, C=O, C=S, S(O), $S(O)_2$ ou leurs combinaisons, éventuellement porteurs d'au moins un groupement hydroxyle ou alcoxy en $C_1$-$C_2$, plus particulièrement un radical alkyle en $C_1$-$C_{10}$, éventuellement substitué ; et de préférence, un radical alkyle en $C_1$-$C_4$ linéaire ou ramifié éventuellement substitué par au moins un groupement hydroxyle, de préférence de 1 à 2 groupements hydroxyle ;

- $\mathbf{R_{21}}$ et $\mathbf{R_{22}}$ pouvant éventuellement former avec l'atome d'azote auxquels ils sont rattachés, un hétérocycle à 5 ou 7 chaînons, saturé ou insaturé, éventuellement substitué comme indiqué précédemment, éventuellement aromatique, comprenant éventuellement un autre hétéroatome identique ou différent de l'azote; le radical alkyle ne comportant pas de fonction nitro, nitroso, peroxo ou diazo,
- **w** est un entier compris entre 1 et 10 ;

◦ **les composés aminés de formule générale (Vi) et/ou (Vi')** :

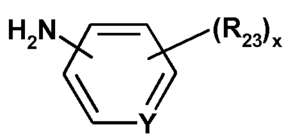

(Vi)

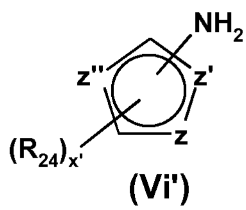

(Vi')

Formules **(Vi)** et/ou **(Vi')** dans laquelle :

- $\mathbf{R_{23}}$ et $\mathbf{R_{24}}$ représentent indépendamment l'un de l'autre :

  *un radical alkyle en $C_1$-$C_6$, éventuellement substitué, éventuellement interrompu par un ou plusieurs hétéroatomes et/ou par un ou plusieurs groupements comprenant au moins un hétéroatome, de préférence choisis parmi l'oxygène, l'azote, le soufre, C(O), S(O), $S(O)_2$ ou leurs combinaisons ; le radical alkyle ne comportant pas de fonction nitro, nitroso, peroxo ou diazo ;
  *un radical alkylcarbonyle (R-C(O)-) dans lequel R représente un radical alkyle en $C_1$-$C_4$ ;
  *un radical alkylsulfonyle (R-$S(O)_2$-) dans lequel R représente un radical alkyle en C1-$C_4$;
  *un radical (di-)(alkyl)aminosulfonyle ($R_2N$-$S(O)_2$-) dans lequel les radicaux R indépendamment représentent un hydrogène, un radical alkyle en $C_1$-$C_4$ ;
  *un radical (di-)(alkyl) aminocarbonyle ($R_2N$-C(O)-) dans lequel les radicaux R indépendamment représentent un hydrogène, un radical alkyle en $C_1$-$C_4$ ;
  *un atome d'halogène choisi de préférence parmi le brome, le chlore ou le fluor ;
  *un groupement alcoxy en $C_1$-$C_4$ ;

*un groupement (poly)hydroxyalcoxy en $C_2$-$C_4$ ;
*un groupement hydroxycarbonyle ou carboxy (HO-C(O)-)
*un groupement alcoxycarbonyle (R-O-C(O)-) dans lequel R représente un radical alkyle en $C_1$-$C_4$,
*un groupement alkylcarbonylamino (R-C(O)-NR'-) dans lequel le radical R représente un radical alkyle en $C_1$-$C_4$ et le radical R' représente un atome d'hydrogène ou un radical alkyle en $C_1$-$C_4$ ;
*un radical alkylsulfonyle (R-S(O)$_2$-) dans lequel le radical R représente un radical alkyle en $C_1$-$C_4$ ;

- **Y** représente un atome de carbone ou d'azote ;
- **z, z', z"** représente indépendamment les uns des autres un atome de carbone, un **atome** d'azote ou un atome d'azote substitué par un hydrogène ;
- **x** est un entier compris entre 0 et 2 ; lorsque **x** est inférieur à 2, le ou les atomes de carbone non substitués porte(nt) un atome d'hydrogène ;
- **x'** est un entier égale à 0 ou 1 ; lorsque **x'** est inférieur à 1, le ou les atomes de carbone non substitués porte(nt) un atome d'hydrogène ;

de préférence la ou les amines sont des aminoalcools ou aminothiols, et en particulier un β- ou γ- aminoalcool ou aminothiol, de préférence amino alcool, plus particulièrement de formule **(Vf), (Vf')** ou **(Vf")** ainsi que leurs sels d'acide minéral ou organique, leurs solvates et leurs isomères optiques :

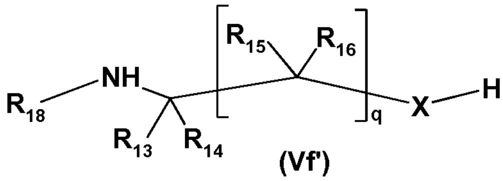
**(Vf')**

Formule **(Vf')** pour lesquels :

- $\mathbf{R_{13}}$ et $\mathbf{R_{14}}$ identiques ou différents représentent un atome d'hydrogène, ii) un groupe alkyle, linéaire ou ramifié, en $C_1$-$C_6$ éventuellement substitué par un groupe hydroxyle,, iii) un radical benzyle, iv) un radical hydroxycarbonyle -C(O)OH, v) un radical -C(O)-O-($C_1$-$C_{12}$)alkyl.
- $\mathbf{R_{15}}$ et $\mathbf{R_{16}}$ indépendamment l'un de l'autre représentent ii) un radical alkyle en $C_1$-$C_4$ linéaire ou ramifié, iii) un radical benzyle.
- $\mathbf{R_{18}}$ représente un atome d'hydrogène ;
- **X** représente un atome d'oxygène, ou de soufre, un radical -NH-, de préférence X représente un atome d'oxygène ;
- **q** est tel que défini précédemment, en particulier q est un entier compris incusivement entre 1 et 10, plus particulièrement entre 1 et 5, préférentiellement 1 ou 2 ;

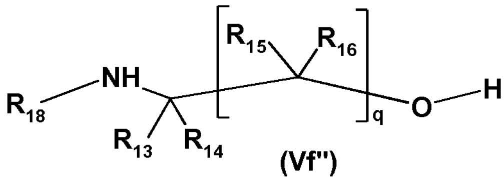
**(Vf")**

Formule **(Vf")** pour lesquels :

- $\mathbf{R_{13}}$, $\mathbf{R_{14}}$, $\mathbf{R_{15}}$ et $\mathbf{R_{16}}$ représentent un atome d'hydrogène ou un groupe ($C_1$-$C_4$)alkyle, de préférence un atome d'hydrogène ;
- $\mathbf{R_{18}}$ représente un atome d'hydrogène ou un groupe ($C_1$-$C_6$)alkyle, de préférence $\mathbf{R_{18}}$ représente un atome d'hydrogène ; et
- **q** est tel que défini précédemment, en particulier q est un entier compris incusivement entre 1 et 10, plus particulièrement entre 1 et 5, préférentiellement entre 1 et 3, tel que 2.

**5.** Procédé de coloration selon une quelconque des revendications précédentes qui met en oeuvre en outre iv) un ou plusieurs composés (thio)carbonylé(s) choisis parmi ceux de formule **(i), (i'), (ii), (a), (b), (c),** et **(d)** leurs isomères optiques ou géométriques, leurs tautomères, et leurs solvates , leurs sels d'acide organique ou minéral, leurs solvates, ainsi que les oligo- ou polysaccharides oxydés :

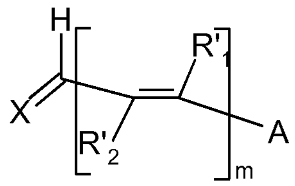

**(i)**

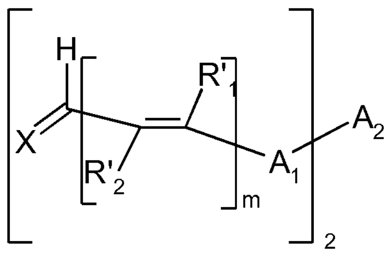

**(ii)**

formules **(i)** et **(ii)** dans lesquelles :

- **m** est un entier compris entre 0 et 2, de préférence valant 0 ou 1 ;
- **X** représente un atome d'oxygène, de soufre, NR"$_1$ avec R"$_1$ représentant un atome d'hydrogène, un radical alkyle en $C_1$-$C_{10}$ ;
- lorsque **X** représente un atome d'oxygène ou de soufre, de préférence X = O, les composés peuvent se trouver également :

    ◦ sous la forme de (thio)acétal de préférence acétal, cyclique à 5 ou 6 chaînons ou acyclique résultant de la condensation d'un monoalcool primaire additionnel (R'$_3$OH) dans lequel R'$_3$ représente un radical alkyle en $C_1$-$C_5$ ou un diol 1,2 ou 1,3 symétrique présentant une chaîne alkyle en $C_2$-$C_3$ ;
    ◦ sous la forme de (thio)hémiacétal de préférence acétal, résultant de la condensation d'un groupement hydroxyle présent sur A ou A$_1$ lorsque A ou A$_1$ représente un radical alkyle et lorsque n vaut 0 ;

- **R'$_1$** et **R'$_2$,** indépendamment l'un de l'autre, représentent un atome d'hydrogène, un radical alkyle en $C_1$-$C_6$ linéaire non substitué ;
- **A** représente un radical monovalent et **A$_1$** représente un radical divalent choisi parmi :
* un groupement hydroxycarbonyle (-C(O)-OH) :

    * un groupement aryle de préférence en $C_6$ éventuellement substitué par au moins un groupe choisi parmi :

        - un groupement hydroxyle,
        - un radical alkyle en $C_1$-$C_4$,
        - un radical aryle-éthylényle, le groupement aryle étant en $C_6$ et éventuellement substitué par au moins un radical alkyle en $C_1$-$C_2$, alcoxy en $C_1$-$C_2$,
        - un groupement -C(O)-OR'$_4$ ou -O-C(O)-R'$_4$ où R'$_4$ représente un radical alkyle en $C_1$-$C_2$, un groupement phényle ;
        - un groupement -C(O)-OH, sous forme acide ou salifiée,
        - un groupement -OR'$_5$ où R'$_5$ représente un radical alkyle en $C_1$-$C_8$ éventuellement substitué par au moins un groupement hydroxyle, un groupement ammonium -N$^+$R"$_6$ avec R"$_6$, identiques ou non, représentant un radical alkyle en $C_1$-$C_2$, un groupement -SiR'$_7$ avec R'$_7$, identiques ou non, représentant un radical alkyle en $C_1$-$C_2$ ; un radical benzyle (-$CH_2$-phényle),
        - selon une variante particulière, deux radicaux -OR'$_5$ situés en ortho du groupement aryle en $C_6$ peuvent former un hétérocycle à 5 ou 6 chaînons par l'intermédiaire des deux radicaux R'$_5$ ;
        - un groupement -N(R'$_8$)$_2$ où R'$_8$, identiques ou non, représentent un radical alkyle en $C_1$-$C_6$ éventuellement substitué par un groupement hydroxyle, carboxylique (-C(O)-OH) sous forme acide ou salifiée, sulfonique (-$SO_3$H) sous forme acide ou salifiée ; les radicaux R'$_8$ pouvant éventuellement former un hétérocycle à 5 ou 6 chaînons avec l'atome d'azote qui les porte, saturé ou non, comprenant éventuellement un autre hétéroatome choisis parmi O, N, NR'$_9$ où R'$_9$ représente un radical alkyle en $C_1$-$C_2$; l'hétérocycle étant éventuellement substitué par un groupement hydroxyle,
        - un groupement -COR'$_{10}$ où R'$_{10}$ représente un groupement aryle en $C_6$ condensé à un cycle hydrocarboné à 6 chaînons, éventuellement substitué par au moins un radical alkyle en $C_1$-$C_2$,
        - un -SR'$_{11}$ où R'$_{11}$ représente un radical alkyle en $C_1$-$C_2$,
        - un atome d'halogène choisi de préférence le chlore, le brome,

- un groupement $-O-S(O)_2$-phényle,
- un groupement $-SO_3H$,
- un hétérocycle à 5 ou 6 chaînons insaturé, cationique ou non cationique, comprenant un ou deux hétéroatomes, choisis parmi O, N, $NR'_{12}$ où $R'_{12}$ représente un groupement alkyle en $C_1$-$C_2$, éventuellement condensé à un cycle à 5 ou 6 chaînons, saturé ou non, aromatique ou non, l'un des hétéroatomes pouvant éventuellement être inclus dans les deux cycles ; l'hétérocycle ou le cycle condensé pouvant être substitué par au moins un radical alcoxy en $C_1$-$C_2$,
- ledit groupement aryle étant éventuellement condensé à un groupement hétérocyclique à 5 ou 6 chaînons, comprenant un ou deux hétéroatomes choisi parmi O, N, $NR'_{13}$ où $R'_{13}$ représente un radical alkyle en $C_1$-$C_4$, l'hétérocycle étant éventuellement condensé à un groupement aryle en $C_6$,
- ledit groupement aryle étant éventuellement condensé à un groupement aryle en $C_6$ éventuellement substitué par au moins un groupement alcoxy en $C_1$-$C_2$ ;

* un groupement hétérocyclique à 5 ou 6 chaînons, cationique ou non cationique, insaturé ou non, aromatique ou non, comprenant un ou deux hétéroatomes, identiques ou non, choisis de préférence parmi O, N, $NR'_{14}$ avec $R'_{14}$ représentant un atome d'hydrogène, un radical alkyle en $C_1$-$C_6$ ou aryle en $C_6$ éventuellement substitué par un groupement $(R'_{15})_2N$-C(O)- ou $R'_{15}$C(O)-N(H)- où $R'_{15}$, identiques ou non représentent un radical alkyle en $C_1$-$C_2$;

- ledit groupement hétérocyclique étant éventuellement condensé à un groupement aryle à 6 chaînons lui-même éventuellement substitué par au moins un groupement alkyle en $C_1$-$C_2$, alcoxy en $C_1$-$C_4$ ; phénoxy;
- ledit groupement hétérocyclique étant éventuellement substitué par au moins :

◦ un groupement hydroxyle,
◦ un radical alkyle en $C_1$-$C_2$ éventuellement substitué par un radical hydroxyle
◦ un radical amino $-N(R'_{16})_2$, où $R'_{16}$, identiques ou non, représentent un radical alkyle en $C_1$-$C_4$ éventuellement substitué par un groupement hydroxyle, les radicaux $R'_{16}$ pouvant éventuellement former un hétérocycle à 5 ou 6 chaînons avec l'atome d'azote qui les porte, saturé ou non, comprenant éventuellement un autre hétéroatome choisis parmi O, N, $NR'_{17}$ où $R'_{17}$ représente un radical alkyle en $C_1$-$C_2$,
◦ un radical aryle en $C_6$ éventuellement substitué par au moins un groupement carboxy -C(O)-OH, amido $R'_{18}$-C(O)-N(H)- avec $R'_{18}$ représentant un radical alkyle en $C_1$-$C_2$ ; ledit radical aryle en $C_6$ étant éventuellement relié à un atome d'azote du groupement hétérocyclique ;

étant entendu que lorsque le radical aryle n'est pas substitué par un radical précédemment cité, les atomes de carbone le sont par un atome d'hydrogène ;
* un radical alkyle en $C_1$-$C_{10}$ linéaire ou ramifié, ou un radical alcényle en $C_2$-$C_{10}$, comprenant une ou plusieurs insaturations carbone-carbone, conjuguées entre elles ou non ; ledit radical alkyle ou alcényle étant éventuellement substitué par au moins un groupement hydroxyle ;

• $\mathbf{A_2}$ représente un radical divalent alkylène en $C_1$-$C_{10}$, linéaire reliant deux radicaux $\mathbf{A_1}$ par l'intermédiaire d'un atome de carbone, d'oxygène ou d'azote ;
• les composés de formules **(i)** et **(ii)** comprenant le cas échéant un contre ion anionique An ou un mélange d'anions cosmétiquement acceptables, garantissant l'électroneutralité des formules ;

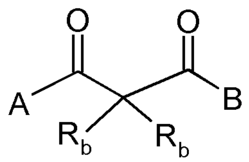
**(a)**

Formule **(a)** dans laquelle :

• **A** représente un groupe choisi parmi :

| | | |
|---|---|---|
| 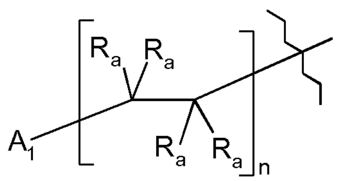 | 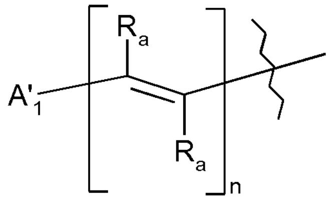 | 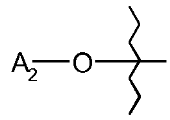 |
| (iiiA1) | (iiiA2) | (iiiA3) |

avec  représentant le point d'attache de la liaison avec le reste de la molécule (a) ;

• **B** représente un groupe choisi parmi:

| | | |
|---|---|---|
| 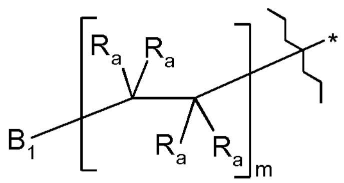 | 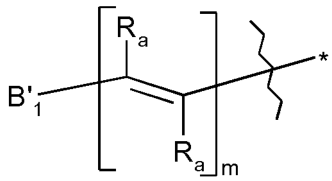 | 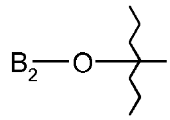 |
| (iiiB1) | (iiiB2) | (iiiB3) |

avec  représentant le point d'attache de la liaison avec le reste de la molécule (a) ;

• **n** et **m,** identiques ou différents, valent 0 ou 1 ;

• **$R_a$,** identiques ou différents, représentent un atome d'hydrogène, un radical alkyle en $C_1$-$C_4$ ;

• **$R_b$,** identiques ou différents, représentent un atome d'hydrogène, un groupement acétyle ($CH_3$-C(O)-) ; l'un des deux radicaux $R_b$ représentant un atome d'hydrogène, ou bien deux radicaux $R_b$ représentant ensemble, un groupement phényl-méthylényle phényl-C(H)=, ledit radical phényle étant éventuellement substitué par au moins un radical hydroxyle, alcoxy en $C_1$-$C_4$ ;

• **$A_1$, $B_1$, $A'_1$, $B'_1$,** identiques ou différents, représentent ;

◦ un groupement aryle en $C_6$ éventuellement substitué par au moins

- un radical alkyle en $C_1$-$C_{20}$ linéaire ou ramifié
- un radical hydroxyle
- un radical alcoxy -O-$R_1$ où $R_1$ représente un radical alkyle en $C_1$-$C_4$ éventuellement substitué par un groupement hydroxyle, un groupement -Si($R_2$)(OSi($R_3$)$_3$)$_3$ où $R_2$, $R_3$, identiques ou non, représentent un radical alkyle en $C_1$-$C_4$, de préférence méthyle
- un groupement ester-O-C(O)-$R_4$ où $R_4$ représente un radical phényle
- un groupement ammonium -$N^+$($R_5$)$_3$ où $R_5$ identiques ou non, représentent un radical alkyle en $C_1$-$C_4$ éventuellement porteur d'au moins un groupement hydroxyle
- un atome d'halogène, de préférence le chlore

• éventuellement condensé à un hétérocycle à 5 ou 6 chaînons, insaturé ou aromatique, comprenant au moins un hétéroatome de préférence l'oxygène ;

◦ un groupement pyridinyle ou pyridinium ; l'atome d'azote quaternisé étant substitué par un radical alkyle en $C_1$-$C_4$ éventuellement porteur d'un groupement hydroxyle ;

◦ un groupement alkyle en $C_1$-$C_4$ ;

◦ les groupements A et B pouvant former ensemble un cycle ou hétérocycle à 5 ou 6 chaînons comprenant éventuellement un hétéroatome, de préférence l'oxygène ; ledit cycle ou hétérocycle étant éventuellement condensé à un radical phényle éventuellement substitué par au moins un groupement acétyle ($CH_3$-C(O)-), ester -C(O)-O-$R_6$ ou -O-C(O)-$R_6$ avec $R_6$ représentant un radical alkyle en $C_1$-$C_4$ ;

• **$A_2$ et $B_2$,** identiques ou différents, représentent :

◦ un radical alkyle en $C_1$-$C_{10}$ linéaire ou ramifié, pouvant éventuellement présenter une ou plusieurs insaturations, éventuellement substitué par un groupement -$SiR_3$ les radicaux R, identiques ou non, représentant un radical alkyle en $C_1$-$C_4$ ;
◦ un radical alcényle en $C_3$-$C_6$ ;
◦ un atome d'hydrogène, un métal alcalin, alcalino-terreux, un groupement ammonium ;
◦ les radicaux $A_2$ et $B_2$ pouvant éventuellement former ensemble un hétérocycle à 6 chaînons ;
◦ les radicaux $R_b$ et $A'_1$, dans le cas où n vaut 1, peuvent former ensemble un cycle hydrocarboné à 6 chaînons, éventuellement substitué par un groupement alkyle en $C_1$-$C_2$, un groupement hydroxyle ;

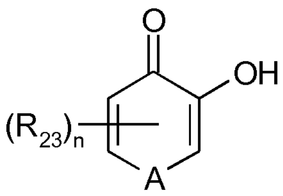
**(b)**

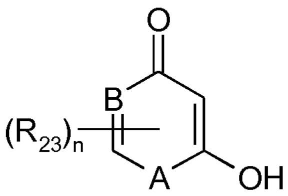
**(c)**

formule **(b)** ou **(c),** ainsi que leur sels d'acide ou de bases, organiques ou minérals, et leurs tautomères, dans lesquelles :

* **n** est un entier valant de 0 à 3 ; les atomes de carbone non substitués portent un atome d'hydrogène
* **A** représente -$C(R_{24})(R_{23})$-, -$C(R_{24})_2$-, O, -C(O)-, -$C(R_{24})=C(R_{24})$- ;
* **B** représente -$C(R'_{23})$-, O ; $R'_{23}$ représente un atome d'hydrogène ou $R_{23}$
* $\mathbf{R_{23}}$ identiques ou non représentent un groupement hydroxyle, un radical alkyle en $C_1$-$C_4$ éventuellement substitué par au moins un groupement hydroxyle ;
* deux radicaux $R_{23}$ portés par deux atomes de carbone adjacents, peuvent former ensemble un cycle aromatique condensé éventuellement substitué par au moins un radical alcoxy en $C_1$-$C_4$, un groupement hydroxyle ;
* $\mathbf{R_{24}}$**,** identiques ou non, représentent un atome d'hydrogène, un radical alkyle linéaire ou ramifié en $C_1$-$C_4$ ;

étant entendu que les composés de formules **(a)** à **(c)** comprennent le cas échéant un contre ion anionique $An^-$ ou un mélange d'anions cosmétiquement acceptables, garantissant l'électroneutralité des formules ;

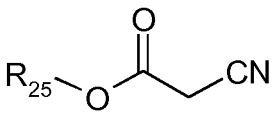
**(d)**

Composé de formule **(d)** dans laquelle $R_{25}$ représente un radical alkyle $C_1$-$C_6$ linéaire ou ramifié éventuellement substitué par un groupement -$SO_3^-M^+$ avec $M^+$ représentant un contre ion cationique tel que ammonium, alcalin ou alcalino terreux, de préférence $Na^+$, -$C(NHR_{26})=NR'_{26}$ où $R_{26}$, $R'_{26}$ identiques ou non, représentent un radical alkyle en $C_4$-$C_8$ cyclique ;

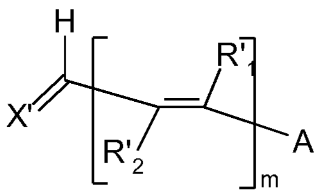

**(i')**

ses isomères optiques ou géométriques, ses sels d'acide organique ou minéral, ses solvates ;
composé de formule **(i')** dans lequel **X'** représente un atome d'oxygène ou de soufre, de préférence oxygène, $\mathbf{R'_1}$ et $\mathbf{R'_2}$ représentent un atome d'hydrogène ou une groupe ($C_1$-$C_4$)alkyle, **A** représente un groupe aryle

éventuellement substitué par les mêmes groupements que définis pour **(i)** précédemment, tel que phényle, **m** est un entier compris inclusviement entre 0 et 2, de préférence entre 0 et 1 tel que 1 ; préférentiellement le ou les composés (thio)carbonylé(s) sont choisis parmi **(i),** et plus particulièrement **(i').**

**6.** Procédé de coloration selon une quelconque des revendications 3 à 5 dans lequel la ou les amines iii) et/ou le ou les composés (thio)carbonylé(s) iv) se trouvent dans la composition **(A),** ou la ou les amines iii) telles que définies dans la revendication 3 ou 4, se trouvent dans une autre composition **(C),** et le ou les composés (thio)carbonylé(s) iv) tels que définis dans la revendication précédente, se trouvent dans une composition **(D),** ou alors la ou les amines iii) et le ou les composés (thio)carbonylé(s) iv) se trouvent ensemble dans la même composition **(E)** ;

- les compositions **(A), (B), (C), (D)** et **(E)** pouvant être appliquées simultanément, ou
- séquentiellement par d'abord l'application de la composition **(A),** puis éventuellement de **(C), (D)** et enfin par l'application de **(B),** de préférence les compositions sont appliquées séquentiellement.

**7.** Procédé de coloration selon la revendication précédente dans lequel le pH de la composition **(A), (C), (D)** et/ou **(E)** est compris inclusivement entre 7 et 9,5, en particulier la composition **(A)** possède un pH compris inclusivement entre 7 et 9,5 et préferentiellement le pH de **(A)** est neutre.

**8.** Procédé de coloration selon une quelconque des revendications précédentes dans lequel le ou les agents oxydants chimique de la composition **(B)** sont choisis parmi le peroxyde d'hydrogène, le peroxyde d'urée, les bromates ou ferricyanures de métaux alcalins, les sels peroxygénés comme par exemple les persulfates, les perborates et les percarbonates de métaux alcalins ou alcalino-terreux, comme le sodium, le potassium, le magnésium, les sels de métaux de transition choisis parmi les sels de zinc, de cuivre, de manganèse, de fer, sels pouvant être de nature organique ou minérale, les agents oxydants de type enzymatique comme les laccases, les peroxydases et les oxydo-réductases à 2 électrons telles que l'uricase, le cas échéant en présence de leur donneur ou co-facteurs respectifs ; de préférence l'agent oxydant chimique est du peroxyde d'hydrogène est particulièrement préférée ; avantageusement la composition **(B)** comprend du peroxyde d'hydrogène en solution aqueuse dont le titre peut de 1 à 40 volumes, et plus préférentiellement de 5 à 40 volumes.

**9.** Procédé de coloration selon une quelconque des revendications précédentes qui met en oeuvre les étapes suivantes :
Soit :

1) application sur les fibres kératiniques de la composition **(A)** comprenant au moins un précurseur de formule **(I)** tels que définis dans la revendication 1 ou 2, avec un temps de pause compris inclusivement entre 10 minutes et 1 heure à une température comprise inclusivement entre 20 et 50 °C ; puis
2) application sur lesdites fibres d'une composition **(C)** comprenant au moins une amine de formule **(V)** ou de formule **(Va)** à **(Vi')** telles que définies dans les revendications 3 et 4, ladite composition **(C)** possédant un pH compris entre 7 et 9,5, avec un temps de pause compris inclusivement entre 30 minutes et 1 heure à une température comprise inclusivement entre 20 et 50 °C, puis
3) révélation de la couleur par application sur lesdites fibres de la composition **(B)** telle que définie dans la revendication 1 ou 8 ;

Soit :

1) application sur les fibres kératiniques de la composition **(A)** comprenant :

- au moins un précurseur de formule **(I)** tels que définis dans la revendication 1 ou 2, et
- au moins une amine de formule **(V)** ou de formule **(Va)** à **(Vi')** telle que définie dans la revendication 3 ou 4, ladite composition **(A)** possédant un pH compris entre 7 et 9,5, avec un temps de pause compris inclusivement entre 10 minutes et 1 heure à une température comprise inclusivement entre 20 et 50 °C ; puis

2) application sur lesdites fibres d'une composition **(D)** comprenant au moins un composé (thio)carbonylé de formule **(i), (i'), (ii), (a), (b), (c),** et **(d)** tels que définis dans la revendication 5, ladite composition **(D)** possédant un pH compris entre 7 et 9,5, avec un temps de pause compris inclusivement entre 30 minutes et 1 heure à une température comprise inclusivement entre 20 et 50 °C, puis
3) révélation de la couleur par application sur lesdites fibres de la composition **(B)** telle que définie dans la revendication 1 ou 8 ;

Soit :

1) application sur les fibres kératiniques de la composition **(A)** comprenant au moins un précurseur de formule **(I)** ou **(I')** tels que définis dans la revendication 1 ou 2, possédant un pH compris entre 7 et 9,5, avec un temps de pause compris inclusivement entre 10 minutes et 1 heure à une température comprise inclusivement entre 20 et 50 °C ; puis
2) application sur lesdites fibres d'une composition **(C)** comprenant au moins une amine de formule **(V)** ou de formule **(Va)** à **(Vi')** telles que définies dans les revendications 3 et 4 ; ladite composition **(C)** possédant un pH compris entre 7 et 9,5, avec un temps de pause compris inclusivement entre 30 minutes et 1 heure à une température comprise inclusivement entre 20 et 50 °C, puis
3) application sur lesdites fibres d'une composition **(D)** comprenant au moins un composé (thio)carbonylé de formule **(i), (i'), (ii), (a), (b), (c),** et **(d)** tels tels que définis dans la revendication 5, ladite composition **(D)** possédant un pH compris entre 7 et 9,5, avec un temps de pause compris inclusivement entre 30 minutes et 1 heure à une température comprise inclusivement entre 20 et 50 °C, puis
4) révélation de la couleur par application sur lesdites fibres de la composition **(B)** telle que définie telle que définie dans la revendication 1 ou 8 ;

Soit :

1) application sur les fibres kératiniques de la composition **(A)** comprenant au moins un précurseur de formule **(I)** tels que définis dans la revendication 1 ou 2, possédant un pH compris entre 7 et 9,5, avec un temps de pause compris inclusivement entre 10 minutes et 1 heure à une température comprise inclusivement entre 20 et 50 °C ; puis
2) application sur lesdites fibres d'une composition **(E)** comprenant :

- au moins une amine de formule **(V)** ou de formule **(Va)** à **(Vi')** telles que définies telles que définies dans les revendications 3 et 4 ;
- au moins un composé (thio)carbonylé de formule **(i), (i'), (ii), (a), (b), (c),** et **(d)** tels que définis dans la revendication 5, ladite composition **(E)** possédant un pH compris entre 7 et 9,5, avec un temps de pause compris inclusivement entre 30 minutes et 1 heure à une température comprise inclusivement entre 20 et 50 °C, puis

3) révélation de la couleur par application sur lesdites fibres de la composition **(B)** telle que définie telle que définie telle que définie dans la revendication 1 ou 8 ;

Soit :

1) application sur les fibres kératiniques de la composition **(A)** comprenant :

- au moins un précurseur de formule **(I)** tels que définis dans la revendication 5 ;
- au moins une amine de formule **(V)** ou de formule **(Va)** à **(Vi')** telles que définies dans les revendications 3 et 4 ; et
- au moins un composé (thio)carbonylé de formule **(i), (i'), (ii), (a), (b), (c),** et **(d)** tels que définis dans la revendication 5 ;

ladite composition **(A)** possédant un pH compris entre 7 et 9,5, avec un temps de pause compris inclusivement entre 10 minutes et 1 heure à une température comprise inclusivement entre 20 et 50 °C ; puis

2) révélation de la couleur par application sur lesdites fibres de la composition **(B)** telle que définie dans la revendication 1 ou 8.

**10.** Composition comprenant :

- un ou plusieurs composés de formule **(I)** tel que défini dans une quelconque des revendications 1 ou 2 ;
- éventuellement une ou plusieurs amines de formule **(V)** ou de formule **(Va)** à **(Vi')** telles que définies dans les revendications 3 et 4 ;
- éventuellement un ou plusieurs polymères aminés ;
- éventuellement un ou plusieurs composés (thio)carbonylés de formule **(i), (i'), (ii), (a), (b), (c),** et **(d)** tels que définis dans la revendication 5 ; et

- éventuellement un ou plusieurs agents oxydants chimiques tels que définis dans la revendication 1 ou 8.

**11.** Composé de formule **(I)** selon une quelconque des revendications 1 ou 2.

**12.** Procédé de préparation de composés de formule **(I)** obtenus par réaction entre :

- au moins un iridoide non glycosylé de formule **(II),** ou **(IV)** tels que définis ci après, plus particulièrement l'iridoïde non glycosylé est de formule **(II)** tels que définis ci après ;
- au moins un composé nucléophile choisi parmi les amines de formule **(V)** ou de formule **(Va)** à **(Vi')** telles que définies dans les revendications 3 et 4 ; et
- au moins un composé électrophile choisi parmi les composés (thio)carbonylés de formule **(i), (i'), (ii), (a), (b), (c),** et **(d)** tels que définis dans la revendication 5 :

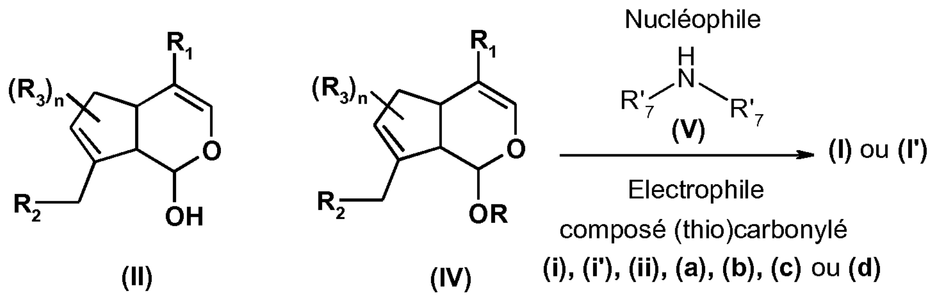

**(II)** **(IV)**

avec formule **(II)** dans laquelle :

- **$R_1$** représente un atome d'hydrogène, un radical méthyle, un radical hydroxyméthyle, un groupement aldéhyde ; un groupement -C(O)-O-$R_4$ dans lequel $R_4$ représente un atome d'hydrogène ou un radical ($C_1$-$C_6$)alkyle, linéaire ou ramifié de préférence en $C_1$-$C_2$ ; ou un groupement -$CH_2$-glucose ; de préférence **$R_1$** représente un atome d'hydrogène, un groupement aldéhyde ; un groupement -C(O)-O-$R_4$ dans lequel $R_4$ représente un atome d'hydrogène ou un radical ($C_1$-$C_6$)alkyle, linéaire ou ramifié de préférence en $C_1$-$C_2$ ; de préférence un groupement -C(O)-O-$R_4$ ;
- **$R_2$** représente un atome d'hydrogène, un radical hydroxyle, un radical glucose ; de préférence **$R_2$** représente un atome d'hydrogène, un radical hydroxyle, un radical glucose ; de préférence un radical hydroxyle ;
- **$R_3$**, identiques ou non, représentent un atome d'hydrogène, un radical hydroxyle, un radical ($C_1$-$C_4$)alkyloxy ; le nombre de groupement hydroxyle n'étant pas supérieur à 2 ;
- **n** est un entier compris entre 1 et 5 ;

ou un extrait végétal comprenant ledit composé de formule **(II)** ; et
formule **(IV)** dans laquelle :

- **$R_1$** représente un radical hydroxyméthyle, un groupement -C(O)-O-$R_4$ dans lequel $R_4$ représente un atome d'hydrogène ou un radical alkyle en $C_1$-$C_2$ ; un radical sucre ;
- **$R_2$** représente un atome d'hydrogène, un radical hydroxyle, un radical sucre ;
- **$R_3$**, identiques ou non, représentent un atome d'hydrogène, un radical hydroxyle, un radical alkyl($C_1$-$C_4$)oxy ; le nombre de groupement hydroxyle n'étant pas supérieur à 2 ;
- **R** représente un radical sucre ;
- **n** est un entier compris entre 1 et 5 ;
- le radical sucre est un dérivé issu d'un aldose ou d'un dérivé d'aldose : ou un extrait végétal comprenant ledit composé de formule **(IV).**

**13.** Procédé de préparation de composé de formule **(I)** selon la revendication précédente dans lequel les composés de départ **(II)** ou **(IV)** tels que définis dans la revendication précédente, sont mis en solution de préférence tamponnée à pH neutre, et sont ajoutés un composé nucléophile choisi parmi les amines de formule **(V)** telles ou de formule **(Va)** à **(Vi')** telles que définies dans les revendications 3 et 4, et un composé électrophile choisi parmi les composés (thio)carbonylés de formule **(i), (i'), (ii), (a), (b), (c),** et **(d)** tels que définis dans la revendication 5, ces ingrédients étant laissé à une température comprise entre 15 °C et 60 °C de préférence entre la température ambiante (25 °C) et 50 °C tel que 40 °C, pendant une durée comprise entre 1 minute et 24 heures, préférentiellement entre 20 minutes

et 6 heures, plus particulièrement entre 1 heures et 4 heures telle que 2 heures ; le produit de réaction peut ensuite être purifié par méthodes classiques connues par l'homme du métier tel que par extraction, dans un solvant organique non miscible à l'eau, de préférence halogéné tel que le dichlorométhane, ou de l'acétate d'éthyle, suivi ou non de chromatographie préparative comme sur colonne de silice avec un éluant de type solvant organique halogéné tel que le dichlorométhane, ou l'acétate d'éthyle en mélange ou non avec un solvant protique polaire tel que l'éthanol ou le méthanol.

**14.** Dispositif à plusieurs compartiments comprenant :

- dans un premier compartiment au moins un composé de formule **(I)** tels que définis dans une quelconque des revendications 1 ou 2 ; et
- dans un deuxième compartiment au moins un agent oxydant chimique tel que défini dans les revendications 1 ou 8 ;

étant entendu que les composés du premier compartiment se présentent préférentiellement sous forme de poudre, particulièrement anhydre.

**15.** Dispositif à plusieurs compartiments tel que défini précédemment comprenant en outre :

- dans un autre compartiment au moins un composé nucléophile choisi parmi les amines de formule **(V)** ou de formule **(Va)** à **(Vi')** telles que définies dans les revendications 3 et 4, et
- dans un autre compartiment au moins un composé électrophile choisi parmi les composés (thio)carbonylés de formule **(i), (i'), (ii), (a), (b), (c),** et **(d)** tels que définis dans la revendication 5 ; et

étant entendu que les amines et les composés (thio)carbonylés se présentent préférentiellement sous forme de poudre, de préférence anhydre.

## Patentansprüche

**1.** Verfahren zum Färben von Keratinfasern, vorzugsweise menschlichen Keratinfasern wie dem Haar, bei dem man auf die Fasern

i) mindestens eine Zusammensetzung **(A),** die mindestens eine Verbindung der folgenden Formel **(I)** :

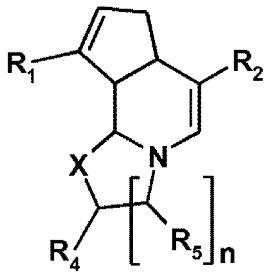
Formel **(I)**

sowie optische oder geometrische Isomere davon, Tautomere davon, Salze davon mit einer mineralischen oder organischen Säure oder Base und Solvate davon wie Hydrate umfasst;
wobei in der Formel **(I):**

• $\mathbf{R_1}$ für i) eine lineare oder verzweigte ($C_1$-$C_6$)-Alkylgruppe, wie Methyl $-CH_3$, ii) -($C_1$-$C_6$)Alk-O-H, vorzugsweise $-CH_2$-O-H, wobei ($C_1$-$C_6$)Alk für eine lineare oder verzweigte $C_1$-$C_6$-Alkylengruppe steht;
• $\mathbf{R_2}$ für ein Atom, ii) eine Gruppe -C(O)-R, wobei R für ein Wasserstoffatom oder eine ($C_1$-$C_4$)-Alkylgruppe steht, wie -C(O)-$CH_3$, iii) Carboxyl -C(O)-OH, iv) -C(O)-O-($C_1$-$C_6$)Alkyl, v) (Di)($C_1$-$C_6$)(alkyl)aminocarbonyl wie -C(O)-$NH_2$ oder-C(O)-N(H)-($C_1$-$C_6$)Alkyl; vorzugsweise -C(O)-O-($C_1$-$C_6$)Alkyl; steht;
• $\mathbf{R_4}$ für ein Wasserstoffatom, ii) einen linearen oder verzweigten $C_1$-$C_4$-Alkylrest oder iii) einen Benzylrest steht; und
• $\mathbf{R_5}$ für i) ein Wasserstoffatom, ii) eine lineare oder verzweigte $C_1$-$C_6$-Alkylgruppe, die gegebenenfalls durch eine Hydroxylgruppe substituiert ist, iii) einen Benzylrest, iv) einen Hydroxycarbonylrest -C(O)OH,

v) einen -C(O)-O-($C_1$-$C_{12}$)Alkylrest steht; insbesondere $R_4$ und $R_5$ für ein Wasserstoffatom stehen;
- **X** für ein Sauerstoffatom steht;
- **n** für 1 oder 2 steht;

ii) mindestens eine Zusammensetzung **(B),** die mindestens ein chemisches Oxidationsmittel umfasst;

aufbringt, wobei man die Zusammensetzungen **(A)** und **(B)** gleichzeitig oder nacheinander aufbringen kann, indem man zunächst **(A)** und dann **(B)** aufbringt, wobei man vorzugsweise die Zusammensetzung **(A)** zuerst aufbringt und dann nach einer Wartezeit von mindestens 5 Minuten nach dem Aufbringen der Zusammensetzung **(A)** Zusammensetzung **(B)** auf die Fasern aufbringt.

**2.** Färbeverfahren nach dem vorhergehenden Anspruch, bei dem die Verbindung der Formel (I) aus:

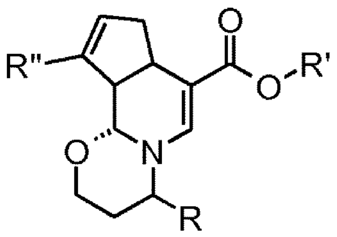

(A)

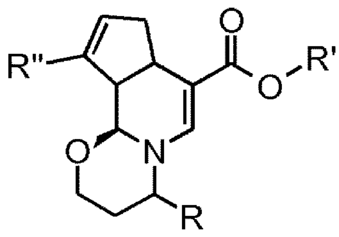

(B)

und

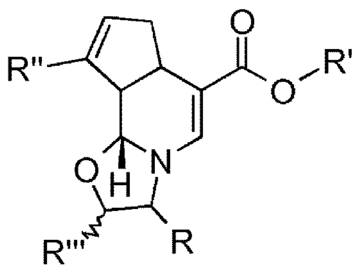

(C)

sowie optischen oder geometrischen Isomere davon, Tautomeren davon, Salzen davon mit einer mineralischen oder organischen Säure oder Base und Solvaten davon wie Hydraten ausgewählt ist;
wobei in den Formeln **(A), (B)** oder **(C):**

- **R** für ein Wasserstoffatom oder eine Carboxygruppe steht;
- **R'** für ein Wasserstoffatom oder eine ($C_1$-$C_6$)-Alkylgruppe steht;
- **R''** für eine ($C_1$-$C_6$) Alkylgruppe, die gegebenenfalls durch mindestens eine Hydroxylgruppe substituiert ist, steht; und
- **R'''** für ein Wasserstoffatom oder eine ($C_1$-$C_6$)-Alkylgruppe steht;

vorzugsweise die Verbindungen der Formel (I) oder (I') aus:

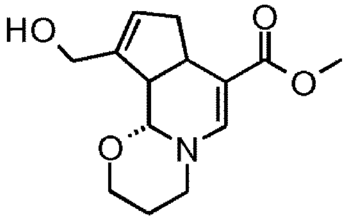
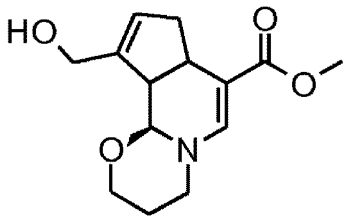

und

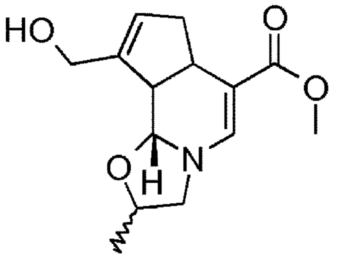

ausgewählt sind.

3. Färbeverfahren nach einem der vorhergehenden Ansprüche, bei dem außerdem iii) ein oder mehrere Amine verwendet werden, die aus den Aminen der Formel **(V):**

$$R'_7R'_8NH \qquad (V)$$

ausgewählt sind, wobei in der Formel **(V)**
**R'**$_7$ and **R'**$_8$ unabhängig voneinander für

- ein Wasserstoffatom;
- einen linearen, verzweigten und/oder cyclischen, gesättigten und/oder ungesättigten, aromatischen oder nichtaromatischen $C_1$-$C_{20}$-Kohlenwasserstoffrest, der 1 bis 5 Kohlenstoff-Kohlenstoff-Doppelbindungen enthalten kann und/oder gegebenenfalls substituiert und gegebenenfalls durch ein oder mehrere Heteroatome und/oder durch eine oder mehrere Gruppen, die mindestens ein Heteroatom oder eine Gruppe mit mindestens einem Heteroatom umfassen, (vorzugsweise ausgewählt aus Sauerstoff, Stickstoff, Schwefel, C=O, C=S, S(O), $S(O)_2$ oder Kombinationen davon) unterbrochen ist; wobei die Kohlenwasserstoffreste $R'_7$ und $R'_8$ gegebenenfalls mit dem Stickstoffatom, an das sie gebunden sind, einen gesättigten oder ungesättigten 5- oder 7-gliedrigen Heterocyclus bilden, der gegebenenfalls substituiert ist, gegebenenfalls aromatisch ist und gegebenenfalls mit einem 6-gliedrigen aromatischen oder heteroaromatischen Kern, der gegebenenfalls ein anderes Heteroatom, das mit Stickstoff identisch oder davon verschieden ist, umfasst, kondensiert ist; wobei der Kohlenwasserstoffrest keine Nitro-, Nitroso-, Peroxo- oder Diazofunktion umfasst; stehen.

4. Färbeverfahren nach einem der vorhergehenden Ansprüche, bei dem außerdem iii) ein oder mehrere Amine verwendet werden, die aus den Aminen der folgenden Formel **(Va)** bis **(Vi')** sowie den Additionssalzen davon mit einer minderalischen oder organischen Säure oder Base ausgewählt sind:

◦ **Aminosäuren der allgemeinen Formel (Va):**

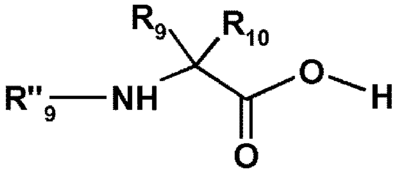

**(Va)**

wobei in der Formel **(Va):**

- $\mathbf{R_9}$ für ein Wasserstoffatom, einen linearen oder verzweigten $C_1$-$C_6$-Alkylrest, der vorzugsweise durch eine oder mehrere Hydroxyl-, Hydroxycarbonyl-, Thiol-, ($C_1$-$C_4$) Alkylthio-, Amido-, Amino- oder Guanidingruppen substituiert ist, einen Phenylrest, der gegebenenfalls durch eine oder mehrere Hydroxylgruppen substituiert ist, einen Indolylrest, der gegebenenfalls durch eine oder mehrere Hydroxylgruppen substituiert ist, einen Imidazolylrest, einen Pyrrolinylrest, der gegebenenfalls durch eine $C_1$-$C_2$-Alkylgruppe substituiert ist; oder einen unsubstituierten Phenylrest steht;
- $\mathbf{R''_9}$ für Wasserstoff, einen $C_1$-$C_4$-Alkylrest oder einen unsubstituierten Phenylrest steht;
- $\mathbf{R_{10}}$ für Wasserstoff oder einen $C_1$-$C_4$-Alkylrest steht;
- $\mathbf{R''_9}$ und $\mathbf{R_9}$ zusammen mit dem Stickstoffatom, an das sie gebunden sind, einen gesättigten 5- oder 6-gliedrigen Heterocyclus bilden können;

◦ **Estern von Aminosäuren und/oder Derivaten der allgemeinen Formel (Vb):**

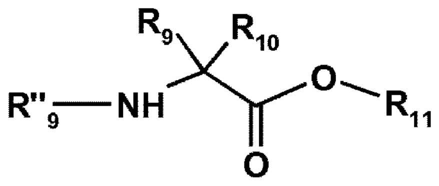

**(Vb)**

wobei in der Formel (Vb):

- $\mathbf{R_9}$ für ein Wasserstoffatom, einen linearen oder verzweigten $C_1$-$C_6$-Alkylrest, der vorzugsweise durch eine oder mehrere Hydroxyl-, Hydroxycarbonyl-, ($C_1$-$C_4$)Alkoxycarbonyl-, Thiol-, ($C_1$-$C_4$) Alkylthio-, Amido-, Amino- oder Guanidingruppen substituiert ist, einen Phenylrest, der gegebenenfalls durch eine oder mehrere Hydroxylgruppen substituiert ist, einen Indolylrest, der gegebenenfalls durch eine oder mehrere Hydroxylgruppen substituiert ist, einen Imidazolylrest, einen Pyrrolinylrest, der gegebenenfalls durch eine $C_1$-$C_2$-Alkylgruppe substituiert ist; oder einen unsubstituierten Phenylrest steht;
- $\mathbf{R''_9}$ für Wasserstoff, einen $C_1$-$C_4$-Alkylrest oder einen unsubstituierten Phenylrest steht;
- $\mathbf{R_{10}}$ für Wasserstoff oder einen $C_1$-$C_4$-Alkylrest steht;
- $\mathbf{R''_9}$ und $\mathbf{R_9}$ zusammen mit dem Stickstoffatom, an das sie gebunden sind, einen gesättigten 5- oder 6-gliedrigen Heterocyclus bilden können;
- $\mathbf{R_{11}}$ für:

  - einen linearen oder verzweigten, gesättigten oder ungesättigten $C_1$-$C_{18}$-Kohlenwasserstoffrest, der gegebenenfalls eine bis 5 konjugierte oder nichtkonjugierte Kohlenstoff-Kohlenstoff-Doppelbindungen umfasst, gegebenenfalls wie oben angegeben substituiert ist und gegebenenfalls durch ein oder mehrere Heteroatome und/oder durch eine oder mehrere Gruppen, die mindestens ein Heteroatom umfassen, vorzugsweise ausgewählt aus Sauerstoff, Stickstoff, Schwefel, C=O, C=S, S(O), $S(O)_2$ oder Kombinationen davon, unterbrochen ist; wobei der Alkylrest keine Nitro-, Nitroso-, Peroxo- oder Diazofunktion umfasst;
  - einen unsubstituierten Benzylrest

  steht;

◦ **Amiden und Thioestern von Aminosäuren und/oder Derivaten der allgemeinen Formel (Vc):**

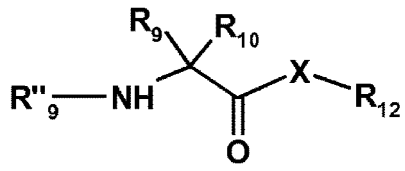

**(Vc)**

wobei in der Formel **(Vc):**

- $\mathbf{R_9}$ für ein Wasserstoffatom, einen linearen oder verzweigten $C_1$-$C_6$-Alkylrest, der vorzugsweise durch eine oder mehrere Hydroxyl-, ($C_1$-$C_4$)Alkoxycarbonyl-, Hydroxycarbonyl-, Thiol-, ($C_1$-$C_4$)Alkylthio-, Amido-, Amino- oder Guanidingruppen substituiert ist, einen Phenylrest, der gegebenenfalls durch eine oder mehrere Hydroxylgruppen substituiert ist, einen Indolylrest, der gegebenenfalls durch eine oder mehrere Hydroxylgruppen substituiert ist, einen Imidazolylrest, einen Pyrrolinylrest, der gegebenenfalls durch eine $C_1$-$C_2$-Alkylgruppe substituiert ist; steht;
- $\mathbf{R''_9}$ für Wasserstoff oder einen $C_1$-$C_4$-Alkylrest, der gegebenenfalls durch einen Hydroxsulfonylrest substituiert ist, steht;
- $\mathbf{R_{10}}$ für Wasserstoff oder einen $C_1$-$C_4$-Alkylrest steht;
- $\mathbf{R''_9}$ und $\mathbf{R_9}$ zusammen mit dem Stickstoffatom, an das sie gebunden sind, einen gesättigten 5-gliedrigen Heterocyclus bilden können;
- $\mathbf{R_{12}}$ für:

  * ein Wasserstoffatom;
  * einen $C_1$-$C_6$-Alkylrest, der vorzugsweise durch eine oder mehrere Hydroxyl-, Thiol-, ($C_1$-$C_4$) - Alkylthio-, Amido- oder Aminogruppen substituiert ist, einen Phenylrest, der gegebenenfalls durch eine oder mehrere Hydroxylgruppen substituiert ist, einen Indolylrest, der gegebenenfalls durch eine oder mehrere Hydroxylgruppen substituiert ist, einen Imidazolylrest, einen Pyrrolinylrest, der gegebenenfalls durch eine $C_1$-$C_2$-Alkylgruppe substituiert ist;
  steht;

- **X** für ein Schwefel- oder Stickstoffatom steht;

◦ **Aminverbindungen der allgemeinen Formel (Vd):**

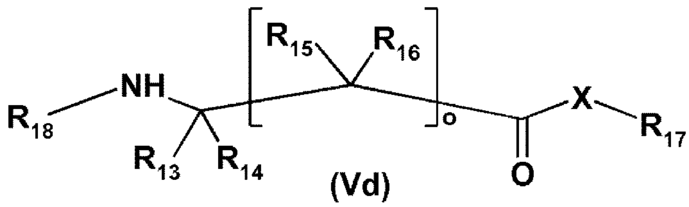

**(Vd)**

wobei in der Formel **(Vd):**

- $\mathbf{R_{13}}$, $\mathbf{R_{14}}$, $\mathbf{R_{15}}$ und $\mathbf{R_{16}}$ unabhängig voneinander für:

  * ein Wasserstoffatom;
  * einen linearen, verzweigten und/oder cyclischen, gesättigten und/oder ungesättigten $C_1$-$C_{20}$-Kohlenwasserstoffrest, der 1 bis 5 Kohlenstoff-Kohlenstoff-Doppelbindungen enthalten kann, gegebenenfalls aromatisch ist, gegebenenfalls wie oben angegeben substituiert ist, gegebenenfalls durch ein oder mehrere Heteroatome und/oder durch eine oder mehrere Gruppen, die mindestens ein Heteroatom umfassen, vorzugsweise ausgewählt aus Sauerstoff, Stickstoff, Schwefel, C=O, C=S, S(O), $S(O)_2$ oder Kombinationen davon, unterbrochen ist und gegebenenfalls mindestens eine Hydroxyl- oder $C_1$-$C_2$-Alkoxygruppe trägt, wobei die Alkylreste $R_{13}$ und $R_{14}$ oder $R_{14}$ und $R_{15}$ oder $R_{15}$ und $R_{16}$ gegebenenfalls mit dem Kohlenstoffatom, an das sie jeweils gebunden sind, einen gesättigten oder ungesättigten 5- oder 7-gliedrigen Heterocyclus bilden können, der gegebenenfalls wie oben angegeben substituiert

ist, gegebenenfalls aromatisch ist und gegebenenfalls ein anderes Heteroatom, das mit Stickstoff identisch oder davon verschieden ist, umfasst; wobei der Alkylrest keine Nitro-, Nitroso-, Peroxo- oder Diazofunktionen umfasst; spezieller einen gegebenenfalls substituierten $C_1$-$C_{10}$-Alkylrest und vorzugsweise einen linearen oder verzweigten $C_1$-$C_8$-Alkylrest, der gegebenenfalls durch mindestens eine Hydroxylgruppe, vorzugsweise 1 bis 2 Hydroxylgruppen, einen Ureidorest, einen ($C_1$-$C_4$)-Alkoxycarbonylrest substituiert ist; einen unsubstituierten Phenylrest
stehen;

- **X** für ein Stickstoff-, Sauerstoff- oder Schwefelatom steht;
- $\mathbf{R_{17}}$ für:

  * ein Wasserstoffatom;
  * einen linearen oder verzweigten, gesättigten oder ungesättigten $C_1$-$C_{18}$-Kohlenwasserstoffrest, der gegebenenfalls eine bis 5 konjugierte oder nichtkonjugierte Kohlenstoff-Kohlenstoff-Doppelbindungen umfasst, gegebenenfalls wie oben angegeben substituiert ist und gegebenenfalls durch ein oder mehrere Heteroatome und/oder durch eine oder mehrere Gruppen, die mindestens ein Heteroatom umfassen, vorzugsweise ausgewählt aus Sauerstoff, Stickstoff, Schwefel, C=O, C=S, S(O), $S(O)_2$ oder Kombinationen davon, unterbrochen ist; wobei der Alkylrest keine Nitro-, Nitroso-, Peroxo- oder Diazofunktion umfasst; steht; spezieller $R_{17}$ für Wasserstoff, einen linearen oder verzweigten $C_1$-$C_{10}$-Alkylrest, der gegebenenfalls substituiert ist; und vorzugsweise Wasserstoff, einen linearen oder verzweigten $C_1$-$C_4$-Alkylrest, der gegebenenfalls durch mindestens eine Hydroxylgruppe, vorzugsweise 1 bis 2 Hydroxylgruppen, substituiert ist; steht;

- $\mathbf{R_{18}}$ für:

  * ein Wasserstoffatom;
  * einen linearen oder verzweigten $C_1$-$C_8$-Alkylrest, der gegebenenfalls wie oben angegeben substituiert ist, gegebenenfalls durch ein oder mehrere Heteroatome und/oder durch ein oder mehrere Heteroatome, die mindestens ein Heteroatom umfassen, vorzugsweise ausgewählt aus Sauerstoff, Stickstoff, Schwefel, CO, C=S, S(O), $S(O)_2$ oder Kombinationen davon, unterbrochen ist und gegebenenfalls mindestens eine Hydroxyl- oder $C_1$-$C_2$-Alkoxygruppe trägt; wobei der Alkylrest keine Nitro-, Nitroso-, Peroxo- oder Diazofunktion umfasst;
  steht;

- **o** für eine ganze Zahl zwischen 0 und 5 inklusive steht;

◦ **Aminverbindungen der allgemeinen Formel (Ve):**

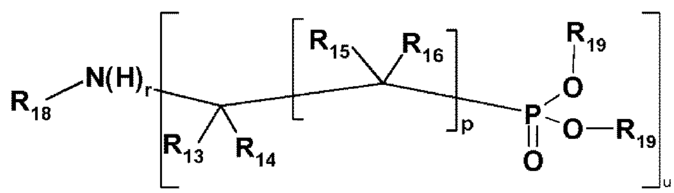

**(Ve)**

wobei in der Formel **(Ve):**

- $\mathbf{R_{13}}$, $\mathbf{R_{14}}$, $\mathbf{R_{15}}$, $\mathbf{R_{16}}$ and $\mathbf{R_{18}}$ die gleiche Bedeutung wie oben für Formel (Vd) haben;
- $\mathbf{R_{19}}$ für:

  * ein Wasserstoffatom;
  * einen linearen oder verzweigten $C_1$-$C_8$-Alkylrest, der gegebenenfalls wie oben angegeben substituiert ist, gegebenenfalls durch ein oder mehrere Heteroatome und/oder durch ein oder mehrere Heteroatome, die mindestens ein Heteroatom umfassen, vorzugsweise ausgewählt aus Sauerstoff, Stickstoff, Schwefel, C=O, C=S, S(O), $S(O)_2$ oder Kombinationen davon, unterbrochen ist und gegebenenfalls mindestens eine Hydroxyl- oder $C_1$-$C_2$-Alkoxygruppe trägt; wobei der Alkylrest keine Nitro-, Nitroso-,

Peroxo- oder Diazofunktion umfasst;
steht;

- **p** für eine ganze Zahl zwischen Ziffern null und 7 inklusive steht;
- **u** für eine ganze Zahl mit einem Wert von 1 oder 2 steht, mit der Maßgabe, dass dann, wenn u für 2 steht, der Rest $R_{18}$ für ein Wasserstoffatom steht;
- **r** für 0 oder 1 steht, mit der Maßgabe, dass dann, wenn u für 1 steht, r für 1 steht, und dann, wenn u für 2 steht, r für 0 steht;

◦ **Aminverbindungen der allgemeinen Formel (Vf):**

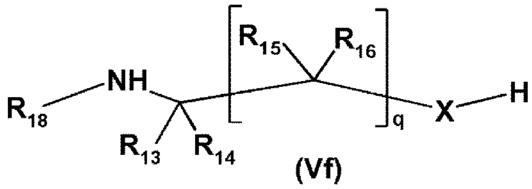

**(Vf)**

wobei in der Formel **(Vf):**

- $\mathbf{R_{13}}$, $\mathbf{R_{14}}$, $\mathbf{R_{15}}$, $\mathbf{R_{16}}$ and $\mathbf{R_{18}}$ die gleiche Bedeutung wie oben haben; außerdem die Reste $\mathbf{R_{13}}$, $\mathbf{R_{14}}$, $\mathbf{R_{15}}$ und $\mathbf{R_{16}}$ unabhängig voneinander auch für einen Hydroxyrest, einen ($C_1$-$C_4$) Alkoxycarbonylrest, einen Carboxaldehydrest, ein ($C_1$-$C_3$)Alkoxy stehen können;
- **q** für eine ganze Zahl zwischen 1 und 18 inklusive steht;
- **X** für ein Sauerstoff- oder Schwefelatom, eine Methylengruppe, die gegebenenfalls durch einen Hydroxyrest substituiert ist, steht; vorzugsweise X für ein Sauerstoffatom steht;
- mit der Maßgabe, dass dann, wenn **X** für ein Sauerstoffatom steht, $\mathbf{R_{18}}$ einen 5- oder 6-gliedrigen Ring bildet, der gegebenenfalls durch eine oder mehrere Hydroxy(methyl)gruppen, vorzugsweise 1 bis 4 Hydroxy(methyl)gruppen, substituiert ist;

◦ **Aminverbindungen der allgemeinen Formel (Vg):**

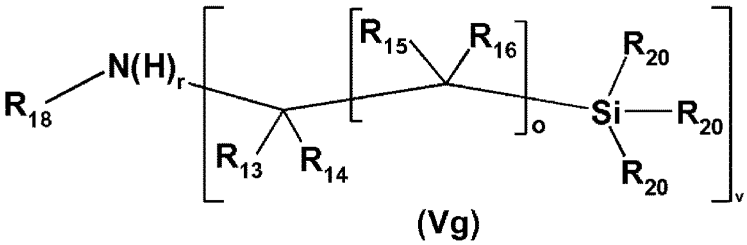

**(Vg)**

wobei in der Formel (Vg):

- $\mathbf{R_{13}}$, $\mathbf{R_{14}}$, $\mathbf{R_{15}}$, $\mathbf{R_{16}}$ und $\mathbf{R_{18}}$ die gleiche Bedeutung wie oben haben;
- $\mathbf{R_{20}}$ für:

  * einen linearen $C_1$-$C_4$-Alkylrest,
  * einen linearen $C_1$-$C_4$-Alkoxyrest

steht;

- **o** für eine ganze Zahl zwischen Ziffern 0 und 5 inklusive steht;
- **v** für eine ganze Zahl mit einem Wert von 1 oder 2 steht, mit der Maßgabe, dass dann, wenn v für 2 steht, $R_{18}$ für Wasserstoff steht;
- **r** für 0 oder 1 steht, mit der Maßgabe, dass dann, wenn v für 1 steht, r für 1 steht, und dann, wenn v für 2 steht, r für 0 steht;

◦ **Aminverbindungen der allgemeinen Formel (Vh):**

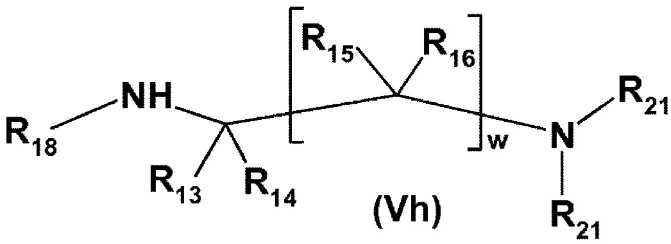

**(Vh)**

wobei in der Formel **(Vh):**

- $\mathbf{R_{13}}$, $\mathbf{R_{14}}$, $\mathbf{R_{15}}$, $\mathbf{R_{16}}$ und $\mathbf{R_{18}}$ die gleiche Bedeutung wie oben haben; außerdem die Reste $\mathbf{R_{13}}$, $\mathbf{R_{14}}$, $\mathbf{R_{15}}$ und $\mathbf{R_{16}}$ unabhängig voneinander auch für einen Hydroxyrest, einen ($C_1$-$C_4$)Alkoxycarbonylrest, einen Carboxaldehydrest, einen ($C_1$-$C_3$)Alkoxyrest stehen können;
- $\mathbf{R_{21}}$ und $\mathbf{R_{22}}$ unabhängig voneinander für:

  *ein Wasserstoffatom;
  * einen linearen, verzweigten und oder cyclischen, gesättigten und/oder ungesättigten $C_1$-$C_{20}$-Kohlenwasserstoffrest, der 1 bis 5 Kohlenstoff-Kohlenstoff-Doppelbindungen enthalten kann, gegebenenfalls wie oben angegeben substituiert ist, gegebenenfalls durch ein oder mehrere Heteroatome und/oder durch eine oder mehrere Gruppen, die mindestens ein Heteroatom umfassen, vorzugsweise ausgewählt aus Sauerstoff, Stickstoff, Schwefel, C=O, C=S, S(O) und $S(O)_2$ oder Kombinationen davon, unterbrochen ist und gegebenenfalls mindestens eine Hydroxyl- oder $C_1$-$C_2$-Alkoxygruppe trägt; spezieller für einen linearen oder verzweigten $C_1$-$C_{10}$-Alkylrest, der gegebenenfalls substituiert ist; und vorzugsweise Wasserstoff, einen linearen oder verzweigten $C_1$-$C_4$-Alkylrest, der gegebenenfalls durch mindestens eine Hydroxylgruppe, vorzugsweise 1 bis 2 Hydroxylgruppen, substituiert ist;
  steht;

- $\mathbf{R_{21}}$ und $\mathbf{R_{22}}$ gegebenenfalls mit dem Stickstoffatom, das sie gebunden sind, einen gesättigten oder ungesättigten 5- oder 7-gliedrigen Heterocyclus bilden, der gegebenenfalls wie oben angegeben substituiert ist, gegebenenfalls aromatisch ist und gegebenenfalls ein anderes Heteroatom, das mit Stickstoff identisch oder davon verschieden ist, umfasst; wobei der Alkylrest keine Nitro-, Nitroso-, Peroxo- oder Diazofunktion umfasst; - **w** für eine ganze Zahl zwischen 1 und 10 steht;

◦ **Aminverbindungen der allgemeinen Formel (Vi) und/oder (Vi'):**

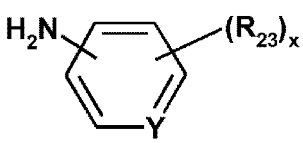

**(Vi)**

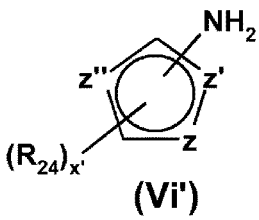

**(Vi')**

wobei in den Formeln **(Vi)** und/oder **(Vi'):**

- $\mathbf{R_{23}}$ und $\mathbf{R_{24}}$ unabhängig voneinander für:

  * einen $C_1$-$C_6$-Alkylrest, der gegebenenfalls substituiert ist und gegebenenfalls durch ein oder mehrere Heteroatome und/oder durch eine oder mehrere Gruppen, die mindestens ein Heteroatom umfassen, vorzugsweise ausgewählt aus Sauerstoff, Stickstoff, Schwefel, C(O), S(O) und $S(O)_2$ oder Kombinationen davon, unterbrochen ist; wobei der Alkylrest keine Nitro-, Nitroso-, Peroxo- oder Diazofunktion umfasst;
  * einen Alkylcarbonylrest (R-C(O)-), wobei R für einen $C_1$-$C_4$-Alkylrest steht;
  * einen Alkylsulfonylrest (R-S(O)$_2$-), wobei R für einen $C_1$-$C_4$-Alkylrest steht;

* einen (Di) (alkyl)aminosulfonylrest ($R_2N-S(O)_2-$), wobei die Reste R unabhängig für Wasserstoff oder einen $C_1$-$C_4$-Alkylrest stehen;
* einen (Di) (alkyl)aminocarbonylrest ($R_2N-C(O)-$), wobei die Reste R unabhängig für Wasserstoff oder einen $C_1$-$C_4$-Alkylrest stehen;
* ein Halogenatom, vorzugsweise ausgewählt aus Brom, Chlor und Fluor;
* eine $C_1$-$C_4$-Alkoxygruppe;
* eine $C_2$-$C_4$-(Poly)hydroxyalkoxygruppe;
* eine Hydroxycarbonyl- oder Carboxyl group (HO-C(O)-) ;
* eine Alkoxycarbonylgruppe (R-O-C(O)-), wobei R für einen $C_1$-$C_4$-Alkylrest steht;
* eine Alkylcarbonylaminogruppe (R-C(O)-NR'-), wobei der Rest R für einen $C_1$-$C_4$-Alkylrest steht und der Rest R' für ein Wasserstoffatom oder einen $C_1$-$C_4$-Alkylrest steht;
* einen Alkylsulfonylrest ($R-S(O)_2-$), wobei der Rest R für einen $C_1$-$C_4$-Alkylrest steht;

stehen;

- **Y** für ein Kohlenstoff- oder Stickstoffatom steht;
- **z, z'** und **z''** unabhängig voneinander für ein Kohlenstoffatom, ein Stickstoffatom oder ein durch Wasserstoff substituiertes Stickstoffatom stehen;
- **x** für eine ganze Zahl zwischen 0 und 2 steht; dann, wenn **x** kleiner als 2 ist, das oder die unsubstituierten Kohlenstoffatome ein Wasserstoffatom tragen;
- **x'** für eine ganze Zahl mit einem Wert von 0 oder 1 steht; dann, wenn **x'** <1 ist, das oder die unsubstituierten Kohlenstoffatome ein Wasserstoffatom tragen;

es sich vorzugsweise bei dem oder den Aminen um Aminoalkohole oder Aminothiole und insbesondere einen β- oder γ-Aminoalkohol oder ein β- oder γ-Aminothiol, vorzugsweise einen Aminoalkohol, spezieller der Formel **(Vf), (Vf')** oder **(Vf")** sowie die Salze davon mit einer mineralischen oder organischen Säure, die Solvate davon und die optischen Isomere davon handelt:

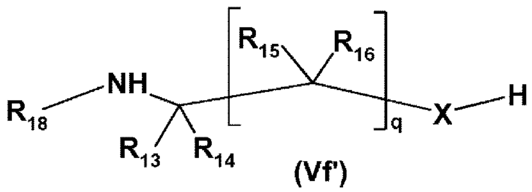

**(Vf')**

wobei in der Formel (Vf'):

- $\mathbf{R_{13}}$ und $\mathbf{R_{14}}$ gleich oder verschieden sind und für ein Wasserstoffatom, ii) eine lineare oder verzweigte $C_1$-$C_6$-Alkylgruppe, die gegebenenfalls durch eine Hydroxylgruppe substituiert ist, iii) einen Benzylrest, iv) einen Hydroxycarbonylrest -C(O)OH, v) einen -C(O)-O-($C_1$-$C_{12}$)Alkylrest stehen;
- $\mathbf{R_{15}}$ und $\mathbf{R_{16}}$ unabhängig voneinander für ii) einen linearen oder verzwigten $C_1$-$C_4$-Alkylrest, iii) einen Benzylrest stehen;
- $\mathbf{R_{18}}$ für ein Wasserstoffatom steht;
- **X** für ein Sauerstoff- oder Schwefelatom oder einem -NH-Rest steht; vorzugsweise X für ein Sauerstoffatom steht;
- **q** wie oben definiert ist, insbesondere q für eine ganze Zahl zwischen 1 und 10, spezieller zwischen 1 und 5, inklusive, bevorzugt 1 oder 2, steht;

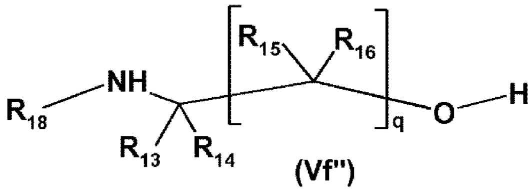

**(Vf")**

wobei in der Formel **(Vf") :**

- **$R_{13}$, $R_{14}$, $R_{15}$** und **$R_{16}$** für ein Wasserstoffatom oder eine ($C_1$-$C_4$)Alkylgruppe, vorzugsweise ein Wasserstoffatom, stehen;
- **$R_{18}$** für ein Wasserstoffatom oder eine ($C_1$-$C_6$)Alkylgruppe stehen, vorzugsweise **$R_{18}$** für ein Wasserstoffatom steht; und
- **q** wie oben definiert ist, insbesondere q für eine ganze Zahl zwischen 1 und 10, spezieller zwischen 1 und 5, bevorzugt zwischen 1 und 3, inklusive, wie 2, steht.

**5.** Färbeverfahren nach einem der vorhergehenden Ansprüche, bei dem außerdem iv) eine oder mehrere (Thio)carbonylverbindungen verwendet werden, die aus denjenigen der Formel **(i), (i'), (ii), (a), (b), (c)** und **(d),** den optischen oder geometrischen Isomeren davon, den Tautomeren davon und den Solvaten davon, den Salzen davon mit einer minderalischen oder organischen Säure oder Base sowie oxidierten Oligo- oder Polysacchariden ausgewählt sind:

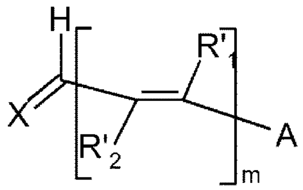

**(i)**

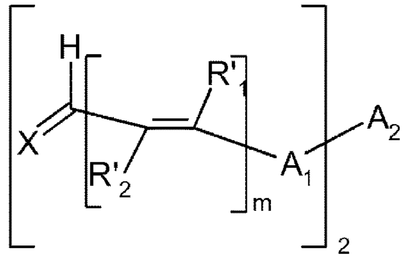

**(ii)**

wobei in den Formeln **(i)** and **(ii):**

- **m** für eine ganze Zahl zwischen 0 und 2, vorzugsweise mit einem Wert von 0 oder 1, steht;
- **X** für ein Sauerstoff- oder Schwefelatom oder NR''$_1$ steht, wobei R''$_1$ für ein Wasserstoffatom oder einen $C_1$-$C_{10}$-Alkylrest steht;
- dann, wenn **X** für ein Sauerstoff- oder Schwefelatom steht, vorzugsweise X = O, die Verbindungen auch:
  - in Form eines 5- oder 6-gliedrigen cyclischen oder acyclischen (Thio)acetal, vorzugsweise Acetal, das sich aus der Kondensation eines primären Monoalkohols (R''$_3$OH), wobei R'$_3$ für einen $C_1$-$C_5$-Alkylrest steht, oder eines symmetrischen 1,2- oder 1,3-Diols mit einer $C_2$-$C_3$-Alkylkette ergibt;
  - in Form eines (Thio)hemiacetals, vorzugsweise Acetals, das sich aus der Kondensation einer an A oder $A_1$ vorliegenden Hydroxylgruppe ergibt, wenn A oder $A_1$ für einen Alkylrest steht und wenn n für 0 steht;

  vorliegen können;
- **R'$_1$** und **R'$_2$** unabhängig voneinander für ein Wasserstoffatom oder einen unsubstituierten linearen $C_1$-$C_6$-Alkylrest stehen;
- **A** für einen einwertigen Rest steht und **$A_1$** für einen zweiwertigen Rest steht, ausgewählt aus:
  - * einer Hydroxycarbonylgruppe (-C(O)-OH);
  - * einer Arylgruppe, vorzugsweise einer $C_6$-Arylgruppe, die gegebenenfalls durch mindestens eine Gruppe, die aus:
    - einer Hydroxylgruppe;
    - einem $C_1$-$C_4$-Alkylrest;
    - einem Arylethylenylrest, wobei die Arylgruppe eine $C_6$-Arylgruppe ist und gegebenenfalls durch mindestens einen $C_1$-$C_2$-Alkyl- oder $C_1$-$C_2$-Alkoxyrest substituiert ist;
    - einer Gruppe -C(O)-OR'$_4$ oder -O-C(O)-R'$_4$ oder -O-C(O)-R'$_4$, in der $R_4$ für einen $C_1$-$C_2$-Alkylrest oder eine Phenylgruppe steht;
    - einer Gruppe -C(O)-OH in Säureform oder versalzter Form;
    - einer Gruppe -OR'$_5$, in der R'$_5$ für einen $C_1$-$C_8$-Alkylrest, der gegebenenfalls durch mindestens eine Hydroxylgruppe, eine Ammoniumgruppe -N$^+$R''$_6$, wobei die Reste R''$_6$ gleich oder verschieden sind und für einen $C_1$-$C_2$-Alkylrest stehen, eine Gruppe -SiR'$_7$, wobei die REste R'$_7$ gleich oder verschieden sind und für einen $C_1$-$C_2$-Alkylrest stehen, substituiert ist; oder einen Benzylrest (-$CH_2$-Phenyl) steht;
    - gemäß einer besonderen Variante zwei Reste -OR'$_5$ in ortho-Position der $C_6$-Arylgruppe über die

beiden Reste R'$_5$ einen 5- oder 6-gliedrigen Heterocyclus bilden können;
- einer Gruppe -N(R'$_8$)$_2$, in der die Reste R'$_8$ gleich oder verschieden sind und für einen $C_1$-$C_6$-Alkylrest, der gegebenenfalls durch eine Hydroxylgruppe, eine Carboxylgruppe (-C(O)-OH) in Säureform oder versalzter Form oder eine Sulfonsäuregruppe (-$SO_3$H) in Säureform oder versalzter Form substituiert ist, stehen; wobei die Reste R'$_8$ mit dem Stickstoffatom, das sie trägt, einen gesättigten oder ungesättigten 5- oder 6-gliedrigen Heterocyclus bilden können, der gegebenenfalls ein weiteres Heteroatom, das aus O, N und NR'$_9$ ausgewählt ist, wobei R'$_9$ für einen $C_1$-$C_2$-Alkylrest steht; wobei der Heterocyclus gegebenenfalls durch eine Hydroxylgruppe substituiert ist;
- einer Gruppe -COR'$_{10}$, wobei R'$_{10}$ für eine $C_6$-Arylgruppe, die mit einem 6-gliedrigen Kohlenwasserstoffring, der gegebenenfalls durch mindestens einen $C_1$-$C_2$-Alkylrest substituiert ist, kondensiert ist, steht;
- einem -SR'$_{11}$, wobei R'$_{11}$ für einen $C_1$-$C_2$-Alkylrest steht;
- einem Halogenatom, das vorzugsweise aus Chlor und Brom ausgewählt ist,
- einer Gruppe -O-S(O)$_2$-Phenyl;
- einer Gruppe -$SO_3$H;
- einem ungesättigten kationischen oder nichtkationischen 5- oder 6-gliedrigen Heterocyclus mit einem oder zwei Heteroatomen, die aus O, N und NR'$_{12}$ ausgewählt sind, wobei R'$_{12}$ für eine $C_1$-$C_2$-Alkylgruppe steht, der gegebenenfalls mit einem gesättigten oder ungesättigten, aromatischen oder nichtaromatischen 5- oder 6-gliedrigen Ring kondensiert ist, wobei eines der Heteroatome gegebenenfalls in beiden Ringen enthalten sein kann; wobei der Heterocyclus oder der kondensierte Ring gegebenenfalls durch mindestens einen $C_1$-$C_2$-Alkoxyreste substituiert sein kann;
ausgewählt ist, substituiert ist;
- wobei die Arylgruppe gegebenenfalls mit einer 5- oder 6-gliedrigen heterocyclischen Gruppe mit einem oder zwei Heteroatomen, die aus O, N und NR'$_{13}$ ausgewählt sind, wobei R'$_{13}$ für einen $C_1$-$C_4$-Alkylrest steht, kondensiert ist, wobei der Heterocyclus gegebenenfalls mit einer $C_6$-Arylgruppe kondensiert ist;
- wobei die Arylgruppe gegebenenfalls an eine $C_6$-Arylgruppe, die gegebenenfalls durch mindestens eine $C_1$-$C_2$-Alkoxygruppe substituiert ist, kondensiert ist;

* einer kationischen oder nichtkationischen, gesättigten oder ungesättigten, aromatischen oder nichtaromatischen 5- oder 6-gliedrigen heterocyclischen Gruppe mit einem oder zwei gleichen oder verschiedenen Heteroatomen, die vorzugsweise aus O, N und NR'$_{14}$ ausgewählt sind, wobei R'$_{14}$ für ein Wasserstoffatom, einen $C_1$-$C_6$-Alkylrest oder einen $C_6$-Arylrest, der gegebenenfalls durch eine Gruppe (R'$_{15}$)$_2$N-C(O)- oder R'$_{15}$-C(O)-N(H)- substituiert ist, wobei die Reste R'$_{15}$ gleich oder verschieden sind und für einen $C_1$-$C_2$-Alkylrest stehen, steht;

- wobei die heterocyclische Gruppe gegebenenfalls mit einer 6-gliedrigen Arylgruppe kondensiert ist, die ihrerseits gegebenenfalls durch mindestens eine $C_1$-$C_2$-Alkyl-, $C_1$-$C_4$-Alkoxy- oder Phenoxygruppe substituiert ist;
- wobei die heterocyclische Gruppe gegebenenfalls durch mindestens:

◦ eine Hydroxylgruppe;
◦ einen $C_1$-$C_2$-Alkylrest, der gegebenenfalls durch einen Hydroxylrest substituiert ist;
◦ einen Aminorest -N(R'$_{16}$)$_2$, wobei die Reste R'$_{16}$ gleich oder verschieden sind und für einen $C_1$-$C_4$-Alkylrest, der gegebenenfalls durch eine Hydroxylgruppe substituiert ist, stehen, wobei die Reste R'$_{16}$ gegebenenfalls mit dem Stickstoffatom, das sie trägt, einen gesättigten oder ungesättigten 5- oder 6-gliedrigen Heterocyclus bilden können, der gegebenenfalls ein weiteres Heteroatom, das aus O, N und NR'$_{17}$ ausgewählt ist, umfasst, wobei R'$_{17}$ für einen $C_1$-$C_2$-Alkylrest steht;
◦ einen $C_6$-Arylrest, der gegebenenfalls durch mindestens eine Carboxygruppe

- C(O)-OH oder Amidogruppe R'$_{18}$-C(O)-N(H)-, wobei R'$_{18}$ für einen $C_1$-$C_2$-Alkylrest steht, substituiert ist; wobei der $C_6$-Arylrest gegebenenfalls an ein Stickstoffatom der heterocyclischen Gruppe gebunden ist;

substituiert ist;
mit der Maßgabe, dass dann, wenn der Arylrest nicht durch einen oben angegebenen Rest substituiert ist, die Kohlenstoffatome durch ein Wasserstoffatom substituiert sind;

* einen linearen oder verzweigten $C_1$-$C_{10}$-Alkylrest oder einem $C_2$-$C_{10}$-Alkenylrest mit einer oder mehreren konjugierten oder nichtkonjugierten Kohlenstoff-Kohlenstoff-Ungesättigtheiten; wobei der Alkyl- oder Alkenylrest gegebenenfalls durch mindestens eine Hydroxylgruppe substituiert ist;

• **$A_2$** für einen linearen zweiwertigen $C_1$-$C_{10}$-Alkylenrest steht, der die beiden Reste **$A_1$** über ein Kohlenstoff-, Sauerstoff- oder Stickstoffatom verbindet;
• wobei die Verbindungen der Formeln **(i)** und **(ii)** gegebenenfalls ein kosmetisch unbedenkliches Anion An oder eine Mischung von kosmetisch unbedenklichen Anionen, das bzw. die die Elektroneutralität der Formeln gewährleistet, umfassen;

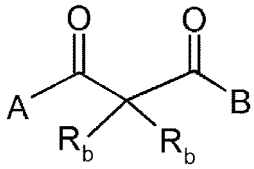
**(a)**

wobei in der Formel **(a):**

• **A** für eine Gruppe steht, die aus:

| | | |
|---|---|---|
| 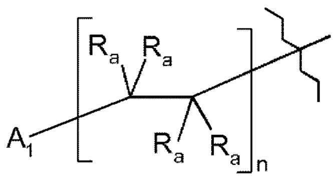 | 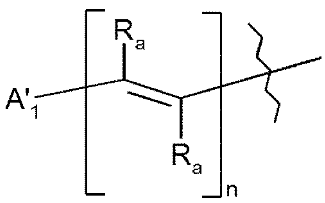 | 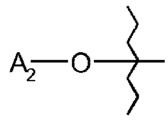 |
| (iiiA1) | (iiiA2) | (iiiA3) |

ausgewählt ist, wobei  für den Punkt der Verknüpfung der Bindung mit dem Rest des Moleküls (a) steht;
• **B** für eine Gruppe steht, die aus:

| | | |
|---|---|---|
| 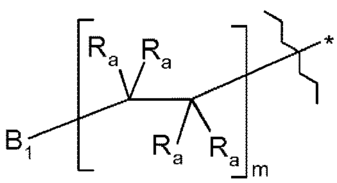 | 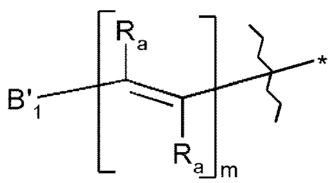 | 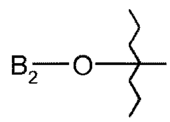 |
| (iiiB1) | (iiiB2) | (iiiB3) |

ausgewählt ist, wobei  für den Punkt der Verknüpfung der Bindung mit dem Rest des Moleküls (a) steht;
• **n** und **m** gleich oder verschieden sind und für 0 oder 1 stehen;
• die Reste **$R_a$** gleich oder verschieden sind und für ein Wasserstoffatom oder einen $C_1$-$C_4$-Alkylrest stehen;
• die Reste **$R_b$** gleich oder verschieden sind und für ein Wasserstoffatom, eine Acetylgruppe ($CH_3$-C(O)-) stehen; wobei einer der beiden Reste $R_b$ für ein Wasserstoffatom steht, oder auch zwei Reste $R_b$ zusammen für eine Phenylmethylenylgruppe Phenyl-C(H)= stehen, wobei der Phenylrest gegebenenfalls durch mindestens einen Hydroxyl- oder $C_1$-$C_4$-Alkoxyrest substituiert ist;
• **$A_1$, $B_1$, $A'_1$** und **$B'_1$** gleich oder verschieden sind und für:

◦ eine $C_6$-Arylgruppe, die gegebenenfalls durch mindestens

- einen linearen oder verzweigten $C_1$-$C_{20}$-Alkylrest;
- einen Hydroxylrest;

- einen Alkoxyrest -O-$R_1$, wobei $R_1$ für einen $C_1$-$C_4$-Alkylrest steht, der gegebenenfalls durch eine Hydroxylgruppe oder eine Gruppe -Si $(R_2)(OSi(R_3)_3)_3$ substituiert ist, wobei $R_2$ und $R_3$ gleich oder verschieden sind und für einen $C_1$-$C_4$-Alkylrest, vorzugsweise Methyl, stehen;
- eine Estergruppe -O-C(O)-$R_4$, wobei $R_4$ für einen Phenylrest steht;
- eine Ammoniumgruppe -$N^+(R_5)_3$, wobei die Reste $R_5$ gleich oder verschieden sind und für einen $C_1$-$C_4$-Alkylrest, der gegebenenfalls mindestens eine Hydroxylgruppe trägt, stehen;
- ein Halogenatom, vorzugsweise Chlor;

substituiert ist und

• gegebenenfalls mit einem ungesättigten oder aromatischen 5- oder 6-gliedrigen Heterocyclus mit mindestens einem Heteroatom, vorzugsweise Sauerstoff, kondensiert ist;

◦ eine Pyridyl- oder Pyridiniumgruppe; wobei das quaternisierte Stickstoffatom durch einen $C_1$-$C_4$-Alkylrest, der gegebenenfalls eine Hydroxylgruppe trägt, substituiert ist;
◦ eine $C_1$-$C_4$-Alkylgruppe;
stehen;
◦ wobei die Gruppen A und B zusammen einen 5- oder 6-gliedrigen Ring oder Heterocyclus bilden können, der gegebenenfalls ein Heteroatom, vorzugsweise Sauerstoff, umfasst; wobei der Ring oder Heterocyclus gegebenenfalls mit einem Phenylrest, der gegebenenfalls durch mindestens eine Acetyl- ($CH_3$-C(O)-), Ester--C(O)-O-$R_6$- oder -O-C(O)-$R_6$-Gruppe substituiert sein kann, kondensiert ist, wobei $R_6$ für einen $C_1$-$C_4$-Alkylrest steht;

• **$A_2$** und **$B_2$** gleich oder verschieden sind und für:

◦ einen linearen oder verzweigten $C_1$-$C_{10}$-Alkylrest, der gegebenenfalls eine oder mehrere ungesättigte Seiten aufweist und gegebenenfalls durch eine Gruppe -$SiR_3$ substituiert ist, wobei die Reste R gleich oder verschieden sind und für einen $C_1$-$C_4$-Alkylrest stehen;
◦ einen $C_3$-$C_6$-Alkenylrest;
◦ ein Wasserstoffatom, ein Alkalimetall oder Erdalkalimetall oder eine Ammoniumgruppe stehen;
◦ wobei die Reste $A_2$ und $B_2$ gegebenenfalls zusammen einen 6-gliedrigen Heterocyclus bilden können;
◦ die Reste $R_b$ und $A'_1$ in dem Fall, dass n für 1 steht, zusammen einen 6-gliedrigen Kohlenwasserstoffring bilden können, der gegebenenfalls durch eine $C_1$-$C_2$-Alkylgruppe oder eine Hydroxylgruppe substituiert ist;

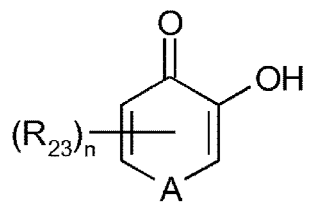
**(b)**
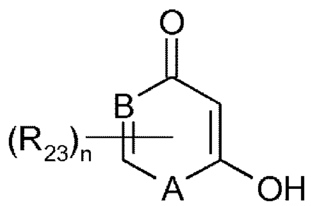
**(c)**

wobei in der Formel **(b)** oder **(c)** sowie den Salzen davon mit organischen oder mineralischen Säuren oder Basen und den Tautomeren davon:

* **n** für eine ganze Zahl von 0 bis 3 steht; die unsubstituierten Kohlenstoffatome ein Wasserstoffatom tragen;
* **A** für -$C(R_{24})(R_{23})$-, -$C(R_{24})_2$-, O, -C(O)- oder -$C(R_{24})=C(R_{24})$- steht;
* **B** für -$C(R'_{23})$- oder O steht; $R'_{23}$ für ein Wasserstoffatom oder $R_{23}$ steht;
* die Reste **$R_{23}$** gleich oder verschieden sind und für eine Hydroxylgruppe oder einen $C_1$-$C_4$-Alkylrest, der gegebenenfalls durch mindestens eine Hydroxylgruppe substituiert ist, stehen;
* zwei Reste $R_{23}$ an zwei benachbarten Kohlenstoffatomen zusammen einen kondensierten aromatischen Ring bilden können, der gegebenenfalls durch mindestens einen $C_1$-$C_4$-Alkoxyrest oder eine Hydroxylgruppe substituiert ist;
* die Reste **$R_{24}$** gleich oder verschieden sind und für ein Wasserstoffatom oder einen linearen oder verzweigten $C_1$-$C_4$-Alkylrest stehen;

mit der Maßgabe, dass die Verbindungen der Formeln **(a)** bis **(c)** gegebenenfalls ein kosmetisch unbedenkliches Anion $An^-$ oder eine Mischung von kosmetisch unbedenklichen Anionen, das bzw. die die Elektroneutralität der Formeln gewährleistet, umfasst;

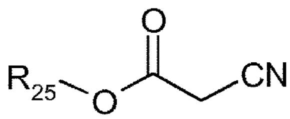

**(d)**

wobei in der Verbindung der Formel **(d)** $R_{25}$ für einen linearen oder verzweigten $C_1$-$C_6$-Alkylrest, der gegebenenfalls durch eine Gruppe $-SO_3^-M^+$, wobei $M^+$ für ein kationisches Gegenion wie Ammonium, ein Alkalimetall oder ein Erdalkalimetall, vorzugsweise $Na^+$, substituiert ist, oder $-C(NHR_{26})=NR'_{26}$, wobei $R_{26}$ und $R'_{26}$ gleich oder verschieden sind und für einen cyclischen $C_4$-$C_8$-Alkylrest stehen, steht;

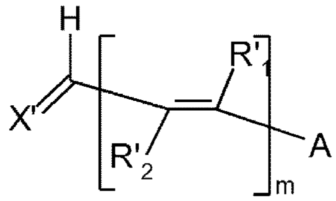

**(i')**

den optischen oder geometrischen Isomere davon, den Salzen davon mit einer organischen oder mineralischen Säuren, den Solvaten davon;
wobei in der Verbindung der Formel **(i') X'** für ein Sauerstoff- oder Schwefelatom, vorzugsweise Sauerstoff, steht, **$R'_1$** und **$R'_2$** für ein Wasserstoffatom oder eine $(C_1$-$C_4)$Alkylgruppe stehen, **A** für eine Arylgruppe, die gegebenenfalls durch die gleichen Gruppen wie oben für **(i)** definiert substituiert ist, wie Phenyl, steht, **m** für eine ganze Zahl zwischen 0 und 2, vorzugsweise zwischen 0 und 1, inklusive, wie 1, steht;
vorzugsweise die (Thio)carbonylverbindung bzw. die (Thio)carbonylverbindungwn aus **(i)** und spezieller **(i')** ausgewählt ist bzw. sind.

**6.** Färbeverfahren nach einem der Ansprüche 3 bis 5, wobei sich das Amin bzw. die Amine iii) und/oder die (Thio)carbonylverbindung bzw. die (Thio)-carbonylverbindungen iv) in Zusammensetzung **(A)** befindet bzw. befinden oder sich das Amin bzw. die Amine iii) gemäß Anspruch 3 oder 4 in einer weiteren Zusammensetzung **(C)** befindet bzw. befinden und sich die (Thio)carbonylverbindung bzw. die (Thio)carbonylverbindungen iv) in einer Zusammensetzung **(D)** befindet bzw. befinden oder auch sich das Amin bzw. die Amine iii) und die (Thio)carbonylverbindung bzw. die (Thio)carbonylverbindungen iv) zusammen in derselben Zusammensetzung **(E)** befinden;

- wobei die Zusammensetzungen **(A), (B), (C), (D)** und **(E)** gleichzeitig aufgebracht werden können oder
- nacheinander aufgebracht werden können, indem man zunächst die Zusammensetzung **(A),** dann gegebenenfalls **(C), (D)** und schließlich **(B)** aufbringt; vorzugsweise die Zusammensetzungen nacheinander aufgebracht werden.

**7.** Färbeverfahren nach dem vorhergehenden Anspruch, wobei der pH-Wert der Zusammensetzung **(A), (C), (D)** und/oder **(E)** zwischen 7 und 9,5 inklusive liegt, insbesondere die Zusammensetzung **(A)** einen pH-Wert zwischen 7 und 9,5 inklusive besitzt und vorzugsweise der pH-Wert von **(A)** neutral ist.

**8.** Färbeverfahren nach einem der vorhergehenden Ansprüche, wobei das chemische Oxidationsmittel bzw. die chemischen Oxidationsmittel der Zusammensetzung **(B)** aus Wasserstoffperoxid, Harnstoffperoxid, Alkalimetallbromaten oder -ferricyaniden, peroxygenierten Salzen, beispielsweise Persulfaten, Perboraten und Percarbonaten von Alkali- oder Erdalkalimetallen, wie Natrium, Kalium oder Magnesium, Übergangsmetallsalze, die aus Zink-, Kupfer-, Mangan- und Eisensalzen ausgewählt sind, wobei diese Salze organischer oder mineralischer Natur sein können, Oxidationsmitteln vom enzymatischen Typ wie Laccasen, Peroxidasen und 2-Elektronen-Oxidoreduktasen wie Uricase, gegebenenfalls in Gegenwart ihres jeweiligen Donors oder Cofaktors ausgewählt ist bzw. sind; es sich vorzugsweise bei dem chemischen Oxidationsmittel um Wasserstoffperoxid handelt; Zusammensetzung **(B)** vorteilhafterweise Wasserstoffperoxid in wässriger Lösung umfasst, deren Titer 1 bis 40 Volumina und weiter bevorzugt 5 bis 40 Volumina betragen kann.

**9.** Färbeverfahren nach einem der vorhergehenden Ansprüche mit den folgenden Schritten:
entweder:

1) Aufbringen der Zusammensetzung **(A),** die mindestens eine Vorstufe der Formel **(I)** gemäß Anspruch 1 oder 2 umfasst, auf die Keratinfasern mit einer Wartezeit zwischen 10 Minuten und 1 Stunde inklusive bei einer Temperatur zwischen 20 und 50 °C inklusive; dann
2) Aufbringen einer Zusammensetzung **(C),** die mindestens ein Amin der Formel **(V)** oder der Formel **(Va)** bis **(Vi')** gemäß den Ansprüchen 3 und 4 umfasst, auf die Keratinfasern, wobei die Zusammensetzung **(C)** einen pH-Wert zwischen 7 und 9,5 aufweist, mit einer Wartezeit zwischen 30 Minuten und 1 Stunde inklusive bei einer Temperatur zwischen 20 und 50 °C inklusive, dann
3) Entwickeln der Farbe durch Aufbringen der Zusammensetzung **(B)** gemäß Anspruch 1 oder 8 auf die Fasern;

oder:

1) Aufbringen der Zusammensetzung **(A),** die

- mindestens einer Vorstufe der Formel **(I)** gemäß Anspruch 1 oder 2 und
- mindestens ein Amin der Formel **(V)** oder der Formel **(Va)** bis **(Vi')** gemäß den Ansprüchen 3 und 4 umfasst, auf die Keratinfasern, wobei die Zusammensetzung **(A)** einen pH-Wert zwischen 7 und 9,5 aufweist,

umfasst, auf die Keratinfasern mit einer Wartezeit zwischen 10 Minuten und 1 Stunde inklusive bei einer Temperatur zwischen 20 und 50 °C inklusive; dann
2) Aufbringen einer Zusammensetzung **(D),** die mindestens eine (Thio)carbonylverbindung der Formel **(i), (i'), (ii), (a), (b), (c)** and **(d)** gemäß Anspruch 5 umfasst, auf die Keratinfasern, wobei die Zusammensetzung **(D)** einen pH-Wert zwischen 7 und 9,5 aufweist, mit einer Wartezeit zwischen 30 Minuten und 1 Stunde inklusive bei einer Temperatur zwischen 20 und 50 °C inklusive, dann
3) Entwickeln der Farbe durch Aufbringen der Zusammensetzung **(B)** gemäß Anspruch 1 oder 8 auf die Fasern;

oder:

1) Aufbringen der Zusammensetzung **(A),** die mindestens eine Vorstufe der Formel **(I)** oder **(I')** gemäß Anspruch 1 oder 2 umfasst und einen pH-Wert zwischen 7 und 9,5 aufweist, auf die Keratinfasern mit einer Wartezeit zwischen 10 Minuten und 1 Stunde inklusive bei einer Temperatur zwischen 20 und 50 °C inklusive; dann
2) Aufbringen einer Zusammensetzung **(C),** die mindestens ein Amin der Formel **(V)** oder der Formel **(Va)** bis **(Vi')** gemäß den Ansprüchen 3 und 4 umfasst, auf die Keratinfasern, wobei die Zusammensetzung **(C)** einen pH-Wert zwischen 7 und 9,5 aufweist, mit einer Wartezeit zwischen 30 Minuten und 1 Stunde inklusive bei einer Temperatur zwischen 20 und 50 °C inklusive, dann
3) Aufbringen einer Zusammensetzung **(D),** die mindestens eine (Thio)carbonylverbindung der Formel **(i), (i'), (ii), (a), (b), (c)** and **(d)** gemäß Anspruch 5 umfasst und einen ptt-Wertzswischen 7 und 9,5 aufweist, auf die Keratinfasern, wobei die Zusammensetzung **(D)** einen pH-Wert zwischen 7 und 9,5 aufweist, mit einer Wartezeit zwischen 30 Minuten und 1 Stunde inklusive bei einer Temperatur zwischen 20 und 50 °C inklusive, dann
4) Entwickeln der Farbe durch Aufbringen der Zusammensetzung **(B)** gemäß Anspruch 1 oder 8 auf die Fasern;

oder:

1) Aufbringen der Zusammensetzung **(A),** die mindestens eine Vorstufe der Formel **(I)** gemäß Anspruch 1 oder 2 umfasst und einen pH-Wert zwischen 7 und 9,5 aufweist, auf die Keratinfasern mit einer Wartezeit zwischen 10 Minuten und 1 Stunde inklusive bei einer Temperatur zwischen 20 und 50 °C inklusive; dann
2) Aufbringen einer Zusammensetzung **(E),** die

- mindestens ein Amin der Formel **(V)** oder der Formel **(Va)** bis **(Vi')** gemäß den Ansprüchen 3 und 4 und
- mindestens eine (Thio)carbonylverbindung der Formel **(i), (i'), (ii), (a), (b), (c)** and **(d)** gemäß Anspruch 5 umfasst, auf die Keratinfasern, wobei die Zusammensetzung **(E)** einen pH-Wert zwischen 7 und 9,5 aufweist, mit einer Wartezeit zwischen 30 Minuten und 1 Stunde inklusive bei einer Temperatur zwischen 20 und 50 °C inklusive, dann

3) Entwickeln der Farbe durch Aufbringen der Zusammensetzung **(B)** gemäß Anspruch 1 oder 8 auf die Fasern;

oder:

1) Aufbringen der Zusammensetzung **(A),** die

- mindestens eine Vorstufe der Formel **(I)** gemäß Anspruch 5;
- mindestens ein Amin der Formel **(V)** oder der Formel **(Va)** bis **(Vi')** gemäß den Ansprüchen 3 und 4 und
- mindestens eine (Thio)carbonylverbindung der Formel **(i), (i'), (ii), (a), (b), (c)** and **(d)** gemäß Anspruch 5

umfasst, auf die Keratinfasern, wobei die Zusammensetzung **(A)** einen pH-Wert zwischen 7 und 9,5 aufweist, mit einer Wartezeit zwischen 10 Minuten und 1 Stunde inklusive bei einer Temperatur zwischen 20 und 50 °C inklusive, dann

2) Entwickeln der Farbe durch Aufbringen der Zusammensetzung **(B)** gemäß Anspruch 1 oder 8 auf die Fasern.

**10.** Zusammensetzung, umfassend:

- eine oder mehrere Verbindungen der Formel **(I)** gemäß einem der Ansprüche 1 oder 2;
- gegebenenfalls ein oder mehrere Amine der Formel **(V)** oder der Formel **(Va)** bis **(Vi')** gemäß den Ansprüchen 3 und 4;
- gegebenenfalls ein oder mehrere Aminpolymere;
- gegebenenfalls eine oder mehrere (Thio)-carbonylverbindungen der Formel **(i), (i'), (ii), (a), (b), (c)** and **(d)** gemäß Anspruch 5; und
- gegebenenfalls ein oder mehrere chemische Oxidationsmittel gemäß Anspruch 1 oder 8.

**11.** Verbindung der Formel **(I)** nach einem der Ansprüche 1 oder 2.

**12.** Verfahren zur Herstellung von Verbindungen der Formel **(I),** erhalten durch Reaktion zwischen:

- mindestens einem nichtglykosylierten Iridoid der Formel **(II)** oder **(IV)** gemäß nachstehender Definition, wobei spezieller das nichtglykosylierte Iridoid die Formel **(II)** gemäß nachstehender Definition aufweist;
- mindestens einer nucleophilen Verbindung, die aus den Aminen der Formel **(V)** oder der Formel **(Va)** bis **(Vi')** gemäß den Ansprüchen 3 und 4 ausgewählt ist; und
- mindestens einer elektrophilen Verbindung, die aus den (Thio)carbonylverbindungen der Formel **(i), (i'), (ii), (a), (b), (c)** and **(d)** gemäß Anspruch 5 ausgewählt ist:

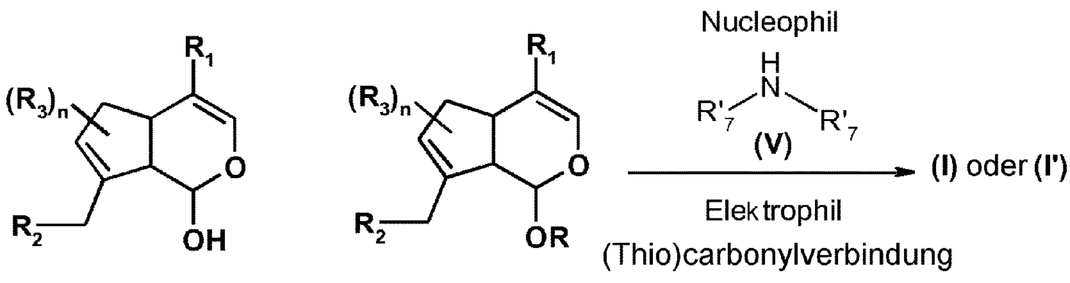


(II) (IV)

wobei in der Formel **(II):**

- **$R_1$** für ein Wasserstoffatom, einen Methylrest, einen Hydroxymethylrest, eine Aldehydgruppe; eine Gruppe -C(O)-O-$R_4$, in der $R_4$ für ein Wasserstoffatom oder einen linearen oder verzweigten ($C_1$-$C_6$)Alkylrest, vorzugsweise $C_1$-$C_2$-Alkylrest, steht; oder eine Gruppe -$CH_2$-Glucose steht; vorzugsweise **$R_1$** für ein Wasserstoffatom, eine Aldehydgruppe; eine Gruppe -C(O)-O-$R_4$, in der $R_4$ für ein Wasserstoffatom oder einen linearen oder verzweigten ($C_1$-$C_6$)Alkylrest, vorzugsweise $C_1$-$C_2$-Alkylrest, steht; vorzugsweise eine Gruppe -C(O)-O-$R_4$; steht;
- **$R_2$** für ein Wasserstoffatom, einen Hydroxylrest, einen Glucoserest steht; vorzugsweise **$R_2$** für ein Wasserstoffatom, einen Hydroxylrest, einen Glucoserest; vorzugsweise einen Hydroxylrest; steht;

• die Reste **$R_3$** gleich oder verschieden sind und für ein Wasserstoffatom, einen Hydroxylrest, einen ($C_1$-$C_4$)Alkyloxyrest stehen; wobei die Zahl der Hydroxylgruppen nicht größer als 2 ist;
• **n** für eine ganze Zahl zwischen 1 und 5 steht;

oder ein Pflanzenextrakt, das die Verbindung der Formel **(II)** umfasst; und
wobei in der Formel **(IV):**

• **$R_1$** für einen Hydroxymethylrest, eine Gruppe -C(O)-O-$R_4$, in der $R_4$ für ein Wasserstoffatom oder einen $C_1$-$C_2$-Alkylrest steht; einen Zuckerrest steht;
• **$R_2$** für ein Wasserstoffatom, einen Hydroxylrest, einen Zuckerrest steht;
• die Reste **$R_3$** gleich oder verschieden sind und für ein Wasserstoffatom, einen Hydroxylrest, einen ($C_1$-$C_4$)Alkyloxyrest stehen; wobei die Zahl der Hydroxylgruppen nicht größer als 2 ist;
• **R** für einen Zuckerrest steht;
• **n** für eine ganze Zahl zwischen 1 und 5 steht;
• der Zuckerrest ein Derivat einer Aldose oder eines Aldosederivats ist;

oder ein Pflanzenextrakt, das die Verbindung der Formel **(IV)** umfasst.

**13.** Verfahren zur Herstellung einer Verbindung der Formel **(I)** nach dem vorhergehenden Anspruch, bei dem die Ausgangsverbindungen **(II)** oder **(IV)** gemäß dem vorhergehenden Anspruch in Lösung, die vorzugsweise auf einen neutralen pH-Wert gepuffert ist, gebracht werden und zu einer nucleophilen Verbindung, die aus den Aminen der Formel **(V)** oder der Formel **(Va)** bis **(Vi')** gemäß den Ansprüchen 3 und 4 ausgewählt ist, und einer elektrophilen Verbindung, die aus den (Thio)carbonylverbindungen der Formel **(i), (i'), (ii), (a), (b), (c)** und **(d)** gemäß Anspruch 5 ausgewählt ist, gegeben werden, wobei diese Bestandteile über einen Zeitraum zwischen 1 Minute und 24 Stunden, vorzugsweise zwischen 20 Minuten und 6 Stunden, spezieller zwischen 1 Stunde und 4 Stunden, wie 2 Stunden, bei einer Temperatur zwischen 15 °C und 60 °C, vorzugsweise zwischen Umgebungstemperatur (25 °C) und 50 °C, wie 40 °C, gehalten werden; das Reaktionsprodukt dann nach herkömmlichen Methoden, die dem Fachmann bekannt sind, gereinigt werden kann, wie durch Extraktion in ein nicht mit Wasser mischbares organisches Lösungsmittel, das vorzugsweise halogeniert ist, wie Dichlormethan, oder Essigsäureethylester, gegebenenfalls mit anschließender präparativer Chromatographie wie an einer Siliciumdioxidsäule mit einem Elutionsmittel vom Typ halogeniertes organisches Lösungsmittel wie Dichlormethan oder Essigsäureethylester, gegebenenfalls im Gemisch mit einem polaren protischen Lösungsmittel wie Ethanol oder Methanol.

**14.** Vorrichtung mit mehreren Kompartimenten, umfassend:

- in einem ersten Kompartiment mindestens eine Verbindung der Formel **(I)** gemäß einem der Ansprüche 1 oder 2; und
- in einem zweiten Kompartiment mindestens ein chemisches Oxidationsmittel gemäß den Ansprüchen 1 oder 8;

mit der Maßgabe, dass die Verbindungen des ersten Kompartiment vorzugsweise in Pulverform, insbesondere wasserfrei, vorliegen.

**15.** Vorrichtung mit mehreren Kompartimenten gemäß obiger Definition, außerdem umfassend:

- in einem weiteren Kompartiment mindestens eine nucleophile Verbindung, die aus den Aminen der Formel **(V)** oder der Formel **(Va)** bis **(Vi')** gemäß den Ansprüchen 3 und 4 ausgewählt ist, und
- in einem weiteren Kompartiment mindestens eine elektrophile Verbindung, die aus den (Thio)-carbonylverbindungen der Formel **(i), (i'), (ii), (a), (b), (c)** und **(d)** gemäß Anspruch 5 ausgewählt ist; und

mit der Maßgabe, dass die Amine und die (Thio)-carbonylverbindungen vorzugsweise in Pulverform, insbesondere wasserfrei, vorliegen.

## Claims

**1.** Process for dyeing keratin fibres, preferably human keratin fibres such as the hair, comprising the application to said fibres:

i) of at least one composition (A) comprising at least one compound of formula (I) below:

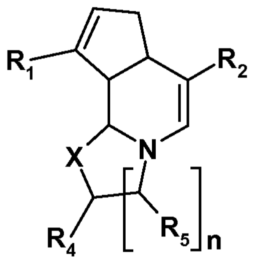

formula (I)

and also the optical or geometrical isomers thereof, the tautomers thereof, the mineral or organic acid or base salts thereof, and the solvates thereof such as hydrates;
in which formula (I):

- $R_1$ represents i) a linear or branched ($C_1$-$C_6$) alkyl group, such as methyl -$CH_3$, ii) -($C_1$-$C_6$) alk-O-H, preferably -$CH_2$-O-H with ($C_1$-$C_6$)alk representing a linear or branched $C_1$-$C_6$ alkylene group;
- $R_2$ represents an atom, ii) a group -C(O)-R with R representing a hydrogen atom or a ($C_1$-$C_4$) alkyl group such as -C(O)-$CH_3$, iii) carboxyl -C(O)-OH, iv) -C(O)-O-($C_1$-$C_6$) alkyl, v) (di) ($C_1$-$C_6$) (alkyl) aminocarbonyl such as -C(O)-$NH_2$ or -C(O)-N(H)-($C_1$-$C_6$)alkyl; preferably -C(O)-O-($C_1$-$C_6$)alkyl;
- $R_4$ represents a hydrogen atom, ii) a linear or branched $C_1$-$C_4$ alkyl radical, or iii) a benzyl radical; and
- $R_5$ represents i) a hydrogen atom, ii) a linear or branched $C_1$-$C_6$ alkyl group optionally substituted with a hydroxyl group, iii) a benzyl radical, iv) a hydroxycarbonyl radical -C(O)OH, v) a -C(O)-O-($C_1$-$C_{12}$) alkyl radical; in particular $R_4$ and $R_5$ represent a hydrogen atom;
- X represents an oxygen atom;
- n is 1 or 2;

ii) at least one composition (B) comprising at least one chemical oxidizing agent;

the compositions (A) and (B) possibly being applied simultaneously, or sequentially by first applying (A) and then applying (B); preferably composition (A) is applied first, followed by applying to said fibres composition (B), after a waiting time of at least 5 minutes after applying composition (A).

**2.** Dyeing process according to the preceding claim, in which the compound of formula (I) is chosen from:

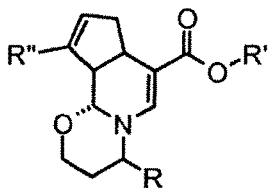

(A)

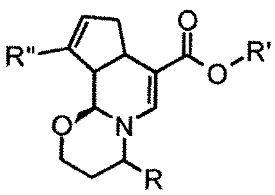

(B)

and

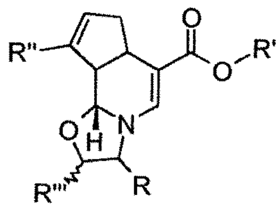

(C)

and also the optical or geometrical isomers thereof, the tautomers thereof, the mineral or organic acid or base salts thereof, and the solvates thereof such as hydrates;
in which formula (A), (B) or (C) :

R represents a hydrogen atom or a carboxyl group;
R' represents a hydrogen atom or a ($C_1$-$C_6$)alkyl group;
R" represents a ($C_1$-$C_6$) alkyl group optionally substituted with at least one hydroxyl group; and
R"' represents a hydrogen atom or a ($C_1$-$C_6$)alkyl group;
preferably, the compounds of formula (I) or (I') are chosen from:

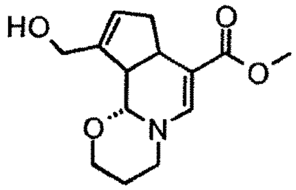

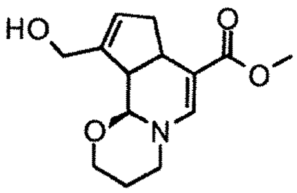

and

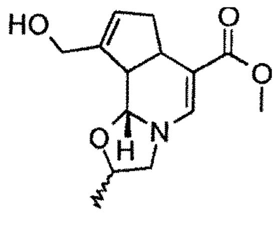

.

**3.** Dyeing process according to either of the preceding claims, which also uses iii) one or more amines chosen from the amines of formula (V):

$R'_7R'_8NH$ (V)

in which formula (V)

- $R'_7$ and $R'_8$ represent, independently of each other,
- a hydrogen atom;
- a linear, branched and/or cyclic, saturated and/or unsaturated, aromatic or non-aromatic $C_1$-$C_{20}$ hydrocarbon-based radical, which may contain from 1 to 5 carbon-carbon double bonds and/or may be optionally substituted, optionally interrupted with one or more heteroatoms and/or with one or more groups comprising at least one heteroatom or group comprising at least one heteroatom (preferably chosen from oxygen, nitrogen, sulfur, C=O, C=S, SO and $SO_2$ or combinations thereof); said hydrocarbon-based radicals $R'_7$ and $R'_8$ possibly forming, with the nitrogen atom to which each is attached, a saturated or unsaturated 5- or 7-membered heterocycle, which is optionally substituted, optionally aromatic, optionally fused to a 6-membered aromatic or heteroaromatic nucleus, optionally comprising another nitrogen or non-nitrogen heteroatom; the hydrocarbon-based radical not comprising any nitro, nitroso, peroxo or diazo functions.

**4.** Dyeing process according to any one of the preceding claims, which also uses iii) one or more amines chosen from the amines of formulae (Va) to (Vi') below, and also the mineral or organic acid-addition or base-addition salts thereof:

◦ amino acids of general formula (Va):

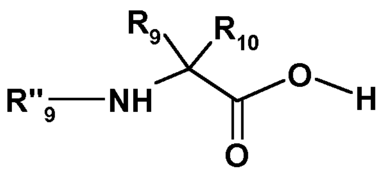

**(Va)**

in which formula (Va):

- $R_9$ represents a hydrogen atom, a linear or branched $C_1$-$C_6$ alkyl radical, preferably substituted with one or more hydroxyl, hydroxycarbonyl, thiol, ($C_1$-$C_4$)alkylthio, amido, amino or guanidine groups, a phenyl radical, optionally substituted with one or more hydroxyl, an indolyl radical optionally substituted with one or more hydroxyl, an imidazolyl radical, a pyrrolinyl radical optionally substituted with a $C_1$-$C_4$ alkyl group; or an unsubstituted phenyl radical;
- $R''_9$ represents a hydrogen, a $C_1$-$C_4$ alkyl radical or an unsubstituted phenyl radical;
- $R_{10}$ represents a hydrogen or a $C_1$-$C_4$ alkyl radical;
- $R''_9$ and $R_9$ possibly form, together with the nitrogen atom to which they are attached, a saturated 5- or 6-membered heterocycle;

◦ esters derived from amino acids and/or derivatives of general formula (Vb):

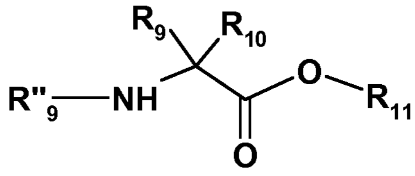

**(Vb)**

in which formula (Vb):

- $R_9$ represents a hydrogen atom, a linear or branched $C_1$-$C_6$ alkyl radical, preferably substituted with one or more hydroxyl, hydroxycarbonyl, ($C_1$-$C_4$)alkoxycarbonyl, thiol, ($C_1$-$C_4$) alkylthio, amido, amino or guanidine groups, a phenyl radical, optionally substituted with one or more hydroxyl, an indolyl radical optionally substituted with one or more hydroxyl, an imidazolyl radical, a pyrrolinyl radical optionally substituted with a $C_1$-$C_2$ alkyl group; or an unsubstituted phenyl radical;
- $R''_9$ represents a hydrogen, a $C_1$-$C_4$ alkyl radical or an unsubstituted phenyl radical;
- $R_{10}$ represents a hydrogen or a $C_1$-$C_4$ alkyl radical;
- $R''_9$ and $R_9$ possibly form, together with the nitrogen atom to which they are attached, a saturated 5- or 6-membered heterocycle;
- $R_{11}$ represents:

  ▪ a linear or branched, saturated or unsaturated $C_1$-$C_{18}$ hydrocarbon-based radical optionally comprising from 1 to 5 conjugated or unconjugated carbon-carbon double bonds, optionally substituted as indicated previously, optionally interrupted with one or more heteroatoms and/or with one or more groups comprising at least one heteroatom, preferably chosen from oxygen, nitrogen, sulfur, C=O, C=S, SO and $SO_2$ or combinations thereof; the alkyl radical not comprising any nitro, nitroso, peroxo or diazo functions;
  ▪ an unsubstituted benzyl radical;

◦ amides and thioesters derived from amino acids and/or derivatives of general formula (Vc):

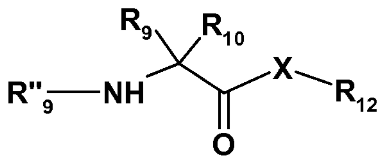

**(Vc)**

in which formula (Vc):

- $R_9$ represents a hydrogen atom, a linear or branched $C_1$-$C_6$ alkyl radical, preferably substituted with one or more hydroxyl, ($C_1$-$C_4$)alkoxycarbonyl, hydroxycarbonyl, thiol, ($C_1$-$C_4$)alkylthio, amido, amino or guanidine groups, a phenyl radical, optionally substituted with one or more hydroxyl, an indolyl radical optionally substituted with one or more hydroxyl, an imidazolyl radical, a pyrrolinyl radical optionally substituted with a $C_1$-$C_2$ alkyl group;
- $R''_9$ represents a hydrogen or a $C_1$-$C_4$ alkyl radical optionally substituted with a hydroxysulfonyl radical;
- $R_{10}$ represents a hydrogen or a $C_1$-$C_4$ alkyl radical;
- $R''_9$ and $R_9$ possibly form, together with the nitrogen atom to which they are attached, a saturated 5-membered heterocycle;
- $R_{12}$ represents:

  * a hydrogen atom;
  * a $C_1$-$C_6$ alkyl radical, preferably substituted with one or more hydroxyl, thiol, ($C_1$-$C_4$) alkylthio, amido or amino groups, a phenyl radical, optionally substituted with one or more hydroxyl, an indolyl radical optionally substituted with one or more hydroxyl, an imidazolyl radical, a pyrrolinyl radical optionally

substituted with a $C_1$-$C_2$ alkyl group;

- X represents a sulfur or nitrogen atom;

◦ amine compounds of general formula (Vd):

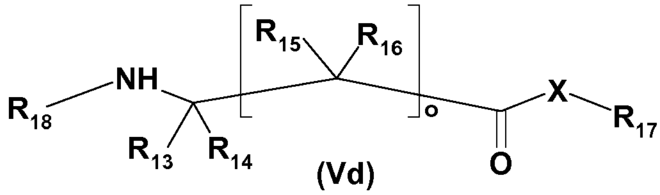

(Vd)

in which formula (Vd):

- $R_{13}$, $R_{14}$, $R_{15}$ and $R_{16}$ represent, independently of each other:

  * a hydrogen atom;
  * a linear, branched and/or cyclic, saturated and/or unsaturated $C_1$-$C_{20}$ hydrocarbon-based radical, which may contain from 1 to 5 optionally aromatic carbon-carbon double bonds, optionally substituted as indicated previously, optionally interrupted with one or more heteroatoms and/or with one or more groups comprising at least one heteroatom, preferably chosen from oxygen, nitrogen, sulfur, C=O, C=S, SO and $SO_2$ or combinations thereof, optionally bearing at least one hydroxyl or $C_1$-$C_2$ alkoxy group, said alkyl radicals $R_{13}$ and $R_{14}$ or $R_{14}$ and $R_{15}$ or $R_{15}$ and $R_{16}$ possibly forming, with the carbon atom to which each is attached, a saturated or unsaturated 5- or 7-membered heterocycle, optionally substituted as indicated previously, which is optionally aromatic, optionally comprising another nitrogen or non-nitrogen heteroatom; the alkyl radical not comprising any nitro, nitroso, peroxo or diazo functions; more particularly an optionally substituted $C_1$-$C_{10}$ alkyl radical; and preferably a linear or branched $C_1$-$C_8$ alkyl radical optionally substituted with at least one hydroxyl group, preferably from 1 to 2 hydroxyl groups, a hydroxycarbonyl radical, a ureido radical, a ($C_1$-$C_4$) alkoxycarbonyl radical; an unsubstituted phenyl radical;

- X represents a nitrogen, oxygen or sulfur atom;
- $R_{17}$ represents:

  * a hydrogen atom;
  * a linear or branched, saturated or unsaturated $C_1$-$C_{18}$ hydrocarbon-based radical, optionally comprising from 1 to 5 conjugated or non-conjugated carbon-carbon double bonds, optionally substituted as indicated previously, optionally interrupted with one or more heteroatoms and/or with one or more groups comprising at least one heteroatom, preferably chosen from oxygen, nitrogen, sulfur, C=O, C=S, SO and $SO_2$ or combinations thereof; the alkyl radical not comprising any nitro, nitroso, peroxo or diazo functions; more particularly, $R_{17}$ represents a hydrogen, an optionally substituted linear or branched $C_1$-$C_{10}$ alkyl radical; and preferably a hydrogen, a linear or branched $C_1$-$C_4$ alkyl radical optionally substituted with at least one hydroxyl group, preferably from 1 to 2 hydroxyl groups;

- $R_{18}$ represents:

  * a hydrogen atom;
  * a linear or branched $C_1$-$C_8$ alkyl radical, optionally substituted as indicated previously, optionally interrupted with one or more heteroatoms and/or with one or more groups comprising at least one heteroatom, preferably chosen from oxygen, nitrogen, sulfur, CO, C=S, SO and $SO_2$ or combinations thereof, optionally bearing at least one hydroxyl or $C_1$-$C_2$ alkoxy group; the alkyl radical not comprising any nitro, nitroso, peroxo or diazo functions;

- o is an integer between 0 and 5 inclusive;

◦ amine compounds of general formula (Ve):

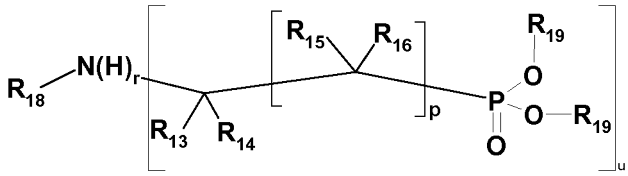

(Ve)

in which formula (Ve):

- $R_{13}$, $R_{14}$, $R_{15}$, $R_{16}$ and $R_{18}$ have the same meaning as previously for formula (Vd);
- $R_{19}$ represents:

    * a hydrogen atom;
    * a linear or branched $C_1$-$C_8$ alkyl radical, optionally substituted as indicated previously, optionally interrupted with one or more heteroatoms and/or with one or more groups comprising at least one heteroatom, preferably chosen from oxygen, nitrogen, sulfur, C=O, C=S, SO and $SO_2$ or combinations thereof, optionally bearing at least one hydroxyl or $C_1$-$C_2$ alkoxy group; the alkyl radical not comprising any nitro, nitroso, peroxo or diazo functions;

- p is an integer between 0 and 7 inclusive;
- u is an integer equal to 1 or 2, it being understood that when u is 2, then the radical $R_{18}$ represents a hydrogen atom;
- r is 0 or 1, it being understood that when u is 1, then r is 1 and when u is 2, then r is 0;

◦ amine compounds of general formula (Vf):

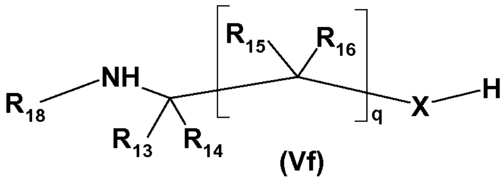

(Vf)

in which formula (Vf):

- $R_{13}$, $R_{14}$, $R_{15}$, $R_{16}$ and $R_{18}$ have the same meaning as previously. In addition, the radicals $R_{13}$, $R_{14}$, $R_{15}$ and $R_{16}$, independently of each other, may also represent a hydroxyl radical, a ($C_1$-$C_4$)alkoxycarbonyl radical, a carboxaldehyde radical, a ($C_1$-$C_3$)alkoxy;
- q is an integer between 1 and 18;
- X represents an oxygen or sulfur atom, a methylene group optionally substituted with a hydroxyl radical; preferably, X represents an oxygen atom;
- it being understood that when X represents an oxygen atom, then $R_{18}$ forms a 5- or 6-membered ring optionally substituted with one or more hydroxy(methyl), preferably from 1 to 4 hydroxy(methyl) groups;

◦ amine compounds of general formula (Vg):

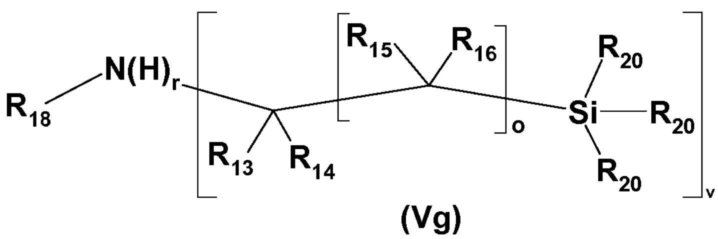

(Vg)

in which formula (Vg):

- $R_{13}$, $R_{14}$, $R_{15}$, $R_{16}$ and $R_{18}$ have the same meaning as previously;
- $R_{20}$ represents:

    * a linear $C_1$-$C_4$ alkyl radical,
    * a linear $C_1$-$C_4$ alkoxy radical;

- o is an integer between 0 and 5 inclusive;
- v is an integer equal to 1 or 2, it being understood that when v is 2, then $R_{18}$ represents a hydrogen;
- r is 0 or 1, it being understood that when v is 1, then r is 1 and when v is 2, then r is 0;

◦ amine compounds of general formula (Vh):

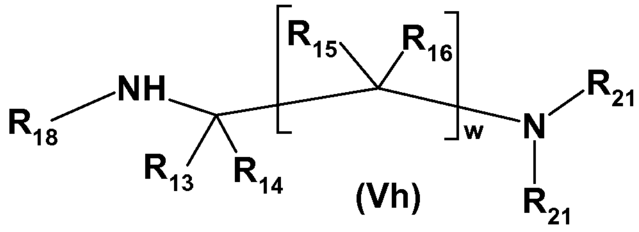

(Vh)

in which formula (Vh):

- $R_{13}$, $R_{14}$, $R_{15}$, $R_{16}$ and $R_{18}$ have the same meaning as previously. In addition, the radicals $R_{13}$, $R_{14}$, $R_{15}$ and $R_{16}$, independently of each other, may also represent a hydroxyl radical, a $(C_1$-$C_4)$alkoxycarbonyl radical, a carboxaldehyde radical, a $(C_1$-$C_3)$alkoxy radical;
- $R_{21}$ and $R_{22}$ represent, independently of each other:

    * a hydrogen atom;
    * a linear, branched and/or cyclic, saturated and/or unsaturated $C_1$-$C_{20}$ hydrocarbon-based radical, which may contain from 1 to 5 carbon-carbon double bonds, optionally substituted as indicated previously, optionally interrupted with one or more heteroatoms and/or with one or more groups comprising at least one heteroatom, preferably chosen from oxygen, nitrogen, sulfur, C=O, C=S, SO and $SO_2$ or combinations thereof, optionally bearing at least one hydroxyl or $C_1$-$C_2$ alkoxy group, more particularly an optionally substituted $C_1$-$C_{10}$ alkyl radical; and preferably a linear or branched $C_1$-$C_4$ alkyl radical optionally substituted with at least one hydroxyl group, preferably from 1 to 2 hydroxyl groups;

- $R_{21}$ and $R_{22}$ possibly forming, with the nitrogen atom to which they are attached, a saturated or unsaturated 5- or 7-membered heterocycle, optionally substituted as indicated previously, optionally aromatic, optionally comprising another nitrogen or non-nitrogen heteroatom;
the alkyl radical not comprising any nitro, nitroso, peroxo or diazo functions;
- w is an integer between 1 and 10;

◦ amine compounds of general formulae (Vi) and/or (Vi'):

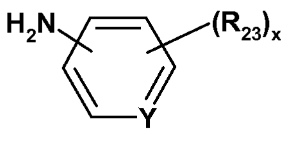

(Vi)

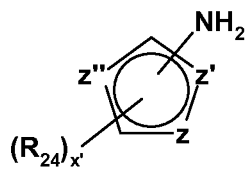

(Vi')

in which formulae (Vi) and/or (Vi'):

- $R_{23}$ and $R_{24}$ represent, independently of each other:

    * an optionally substituted $C_1$-$C_6$ alkyl radical, optionally interrupted with one or more heteroatoms

and/or with one or more groups comprising at least one heteroatom, preferably chosen from oxygen, nitrogen, sulfur, CO, SO and $SO_2$ or combinations thereof; the alkyl radical not comprising any nitro, nitroso, peroxo or diazo functions;

* an alkylcarbonyl radical (R-C(O)-) in which R represents a $C_1$-$C_4$ alkyl radical;
* an alkylsulfonyl radical (R-S(O)$_2$-) in which R represents a $C_1$-$C_4$ alkyl radical;
* a (di)(alkyl)aminosulfonyl radical ($R_2$N-S(O)$_2$-) in which the radicals R independently represent a hydrogen or a $C_1$-$C_4$ alkyl radical;
* a (di)(alkyl)aminocarbonyl radical ($R_2$N-C(O)-) in which the radicals R independently represent a hydrogen or a $C_1$-$C_4$ alkyl radical;
* a halogen atom preferably chosen from bromine, chlorine and fluorine;
* a $C_1$-$C_4$ alkoxy group,
* a $C_2$-$C_4$ (poly)hydroxyalkoxy group;
* a hydroxycarbonyl or carboxyl group (HO-C(O)-);
* an alkoxycarbonyl group (R-O-C(O)-) in which R represents a $C_1$-$C_4$ alkyl radical;
* an alkylcarbonylamino group (R-C(O)-NR'-) in which the radical R represents a $C_1$-$C_4$ alkyl radical and the radical R' represents a hydrogen atom or a $C_1$-$C_4$ alkyl radical;
* an alkylsulfonyl radical (R-S(O)$_2$-) in which the radical R represents a $C_1$-$C_4$ alkyl radical;

- Y represents a carbon or nitrogen atom;
- z, z' and z" represent, independently of each other, a carbon atom, a nitrogen atom or a nitrogen atom substituted with a hydrogen;
- x is an integer between 0 and 2; when x is less than 2, the unsubstituted carbon atom(s) bear a hydrogen atom;
- x' is an integer equal to 0 or 1; when x' is less than 1, the unsubstituted carbon atom(s) bear a hydrogen atom;

preferably, the amine(s) are amino alcohols or amino thiols, and in particular a β- or γ-amino alcohol or amino thiol, preferably an amino alcohol, more particularly of formula (Vf), (Vf') or (Vf"), and also the mineral or organic acid salts thereof, the solvates thereof and the optical isomers thereof:

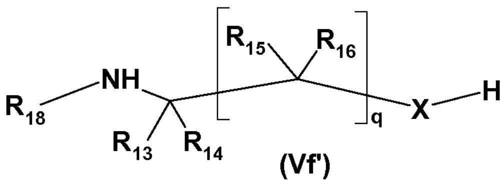
(Vf')

in which formula (Vf'):

- $R_{13}$ and $R_{14}$, which may be identical or different, represent a hydrogen atom, ii) a linear or branched $C_1$-$C_6$ alkyl group optionally substituted with a hydroxyl group, iii) a benzyl radical, iv) a hydroxycarbonyl radical -C(O)-OH, v) a -C(O)-O-($C_1$-$C_{12}$)alkyl radical;
- $R_{15}$ and $R_{16}$, independently of each other, represent ii) a linear or branched $C_1$-$C_4$ alkyl radical, iii) a benzyl radical;
- $R_{18}$ represents a hydrogen atom;
- X represents an oxygen or sulfur atom, an -NH-radical; preferably, X represents an oxygen atom;
- q is as defined previously, in particular q is an integer inclusively between 1 and 10, more particularly between 1 and 5, preferentially 1 or 2;

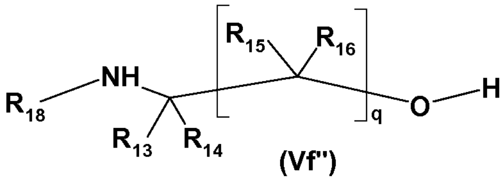
(Vf")

in which formula (Vf"):

- $R_{13}$, $R_{14}$, $R_{15}$ and $R_{16}$ represent a hydrogen atom or a ($C_1$-$C_4$)alkyl group, preferably a hydrogen atom;

- $R_{18}$ represents a hydrogen atom or a ($C_1$-$C_6$) alkyl group, preferably $R_{18}$ represents a hydrogen atom; and
- q is as defined previously, in particular q is an integer inclusively between 1 and 10, more particularly between 1 and 5, preferentially between 1 and 3, such as 2.

**5.** Dyeing process according to any one of the preceding claims, which also uses iv) one or more (thio)carbonyl compounds chosen from those of formulae (i), (i'), (ii), (a), (b), (c) and (d), the optical or geometrical isomers thereof, the tautomers thereof, and the solvates thereof, the organic or mineral acid salts thereof, the solvates thereof, and also oxidized oligo- or polysaccharides:

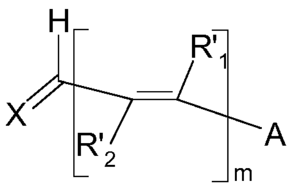

(i)

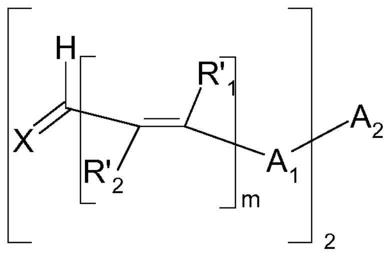

(ii)

in which formulae (i) and (ii):

- m is an integer between 0 and 2, preferably equal to 0 or 1;
- X represents an oxygen or sulfur atom, NR"$_1$ with R"$_1$ representing a hydrogen atom or a $C_1$-$C_{10}$ alkyl radical;
- when X represents an oxygen or sulfur atom, preferably X = O, the compounds may also be:

  ◦ in the form of a 5- or 6-membered cyclic or acyclic (thio)acetal, preferably acetal, resulting from the condensation of an additional primary monoalcohol (R'$_3$OH) in which R'$_3$ represents a $C_1$-$C_5$ alkyl radical or a symmetrical 1,2- or 1,3-diol containing a $C_2$-$C_3$ alkyl chain;
  ◦ in the form of a (thio)hemiacetal, preferably acetal, resulting from the condensation of a hydroxyl group present on A or $A_1$ when A or $A_1$ represents an alkyl radical and when n is equal to 0;

- R'$_1$ et R'$_2$, independently of each other, represent a hydrogen atom or an unsubstituted linear $C_1$-$C_6$ alkyl radical;
- A represents a monovalent radical and $A_1$ represents a divalent radical chosen from:

  * a hydroxycarbonyl group (-C(O)-OH):
  * an aryl group, preferably of $C_6$ optionally substituted with at least one group chosen from:

    - a hydroxyl group;
    - a $C_1$-$C_4$ alkyl radical;
    - an aryl-ethylenyl radical, the aryl group being $C_6$ and optionally substituted with at least one $C_1$-$C_2$ alkyl or $C_1$-$C_2$ alkoxy radical;
    - a group -C(O)-OR'$_4$ or -O-C(O)-R'$_4$ or -O-C(O)-R'$_4$ in which $R_4$ represents a $C_1$-$C_2$ alkyl radical, or a phenyl group;
    - a group -C(O)-OH, in acid or salified form;
    - a group -OR'$_5$ in which R'$_5$ represents a $C_1$-$C_8$ alkyl radical optionally substituted with at least one hydroxyl group, an ammonium group -$N^+$R"$_6$ with R"$_6$, which may be identical or different, representing a $C_1$-$C_2$ alkyl radical, a group -SiR'$_7$ with R'$_7$, which may be identical or different, representing a $C_1$-$C_2$ alkyl radical; a benzyl radical (-$CH_2$-phenyl);
    - according to one particular variant, two radicals
    - OR'$_5$ located ortho to the $C_6$ aryl group may form a 5- or 6-membered heterocycle by means of the two radicals R'$_5$ located ortho to the $C_6$ aryl group may form a 5- or 6-membered heterocycle by means of the two radicals R'$_5$;
    - a group -N(R'$_8$)$_2$ in which R'$_8$, which may be identical or different, represent a $C_1$-$C_6$ alkyl radical optionally substituted with a hydroxyl group, a carboxylic group (-C(O)-OH) in acid or salified form, or a sulfonic group (-$SO_3$H) in acid or salified form;

the radicals $R'_8$ possibly forming a saturated or unsaturated 5- or 6-membered heterocycle with the nitrogen atom that bears them, optionally comprising another heteroatom chosen from O, N and $NR'_9$ in which $R'_9$ represents a $C_1$-$C_2$ alkyl radical; the heterocycle being optionally substituted with a hydroxyl group;

- a group $-COR'_{10}$ in which $R'_{10}$ represents a $C_6$ aryl group fused to a 6-membered hydrocarbon-based ring optionally substituted with at least one $C_1$-$C_2$ alkyl radical;
- an $-SR'_{11}$ in which $R'_{11}$ represents a $C_1$-$C_2$ alkyl radical;
- a halogen atom preferably chosen from chlorine and bromine,
- a group $-O-S(O)_2$-phenyl;
- a group $-SO_3H$;
- an unsaturated cationic or non-cationic 5- or 6-membered heterocycle comprising one or two heteroatoms chosen from O, N and $NR'_{12}$ in which $R'_{12}$ represents a $C_1$-$C_2$ alkyl group optionally fused to a saturated or unsaturated, aromatic or non-aromatic 5- or 6-membered ring, one of the heteroatoms possibly being included in the two rings; the heterocycle or the fused ring possibly being substituted with at least one $C_1$-$C_2$ alkoxy radical;
- said aryl group being optionally fused to a 5- or 6-membered heterocyclic group, comprising one or two heteroatoms chosen from O, N and $NR'_{13}$ in which $R'_{13}$ represents a $C_1$-$C_4$ alkyl radical, the heterocycle being optionally fused to a $C_6$ aryl group;
- said aryl group being optionally fused to a $C_6$ aryl group optionally substituted with at least one $C_1$-$C_2$ alkoxy group;

* a cationic or non-cationic, saturated or unsaturated, aromatic or non-aromatic 5- or 6-membered heterocyclic group, comprising one or two identical or different heteroatoms preferably chosen from O, N and $NR'_{14}$ with $R'_{14}$ representing a hydrogen atom, a $C_1$-$C_6$ alkyl radical or a $C_6$ aryl radical optionally substituted with a group $(R'_{15})_2N-C(O)-$ or $R'_{15}-C(O)-N(H)-$ in which $R'_{15}$, which may be identical or different, represent a $C_1$-$C_2$ alkyl radical;
- said heterocyclic group being optionally fused to a 6-membered aryl group which is itself optionally substituted with at least one $C_1$-$C_2$ alkyl, $C_1$-$C_4$ alkoxy or phenoxy group;
- said heterocyclic group being optionally substituted with at least:

◦ a hydroxyl group;
◦ a $C_1$-$C_2$ alkyl radical optionally substituted with a hydroxyl radical;
◦ an amino radical $-N(R'_{16})_2$ in which $R'_{16}$, which may be identical or different, represent a $C_1$-$C_4$ alkyl radical optionally substituted with a hydroxyl group, the radicals $R'_{16}$ possibly forming a saturated or unsaturated 5- or 6-membered heterocycle with the nitrogen atom that bears them, optionally comprising another heteroatom chosen from O, N and $NR'_{17}$ in which $R'_{17}$ represents a $C_1$-$C_2$ alkyl radical;
◦ a $C_6$ aryl radical optionally substituted with at least one carboxyl group $-C(O)-OH$, amido group $R'_{18}-C(O)-N(H)-$ with $R'_{18}$ representing a $C_1$-$C_2$ alkyl radical; said $C_6$ aryl radical being optionally linked to a nitrogen atom of the heterocyclic group;

it being understood that when the aryl radical is not substituted with a radical mentioned previously, the carbon atoms are substituted with a hydrogen atom; * a linear or branched $C_1$-$C_{10}$ alkyl radical, or a $C_2$-$C_{10}$ alkenyl radical, comprising one or more conjugated or non-conjugated carbon-carbon unsaturations; said alkyl or alkenyl radical being optionally substituted with at least one hydroxyl group;

• $A_2$ represents a linear divalent $C_1$-$C_{10}$ alkylene radical connecting two radicals $A_1$ via a carbon, oxygen or nitrogen atom;
• the compounds of formulae (i) and (ii) comprising, where appropriate, a cosmetically acceptable anionic counterion An or a mixture of anions, ensuring the electrical neutrality of the formulations;

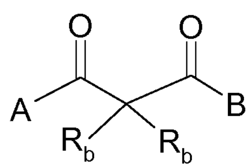
(a)

in which formula (a):

• A represents a group chosen from:

| 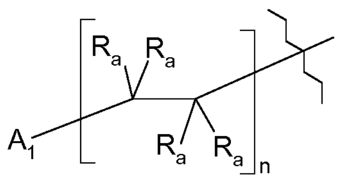 | 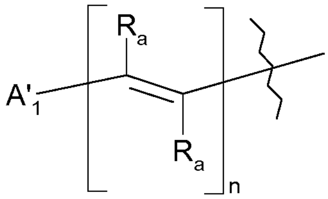 | 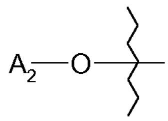 |
|---|---|---|
| (iiiA1) | (iiiA2) | (iiiA3) |

with  representing the point of attachment of the bond to the rest of the molecule (a);

• B represents a group chosen from:

| 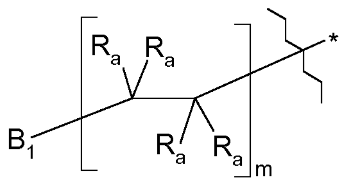 | 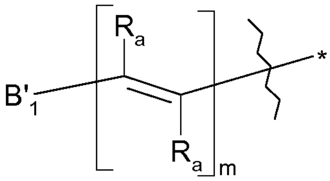 | 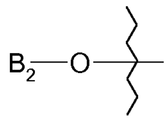 |
|---|---|---|
| (iiiB1) | (iiiB2) | (iiiB3) |

with  representing the point of attachment of the bond to the rest of the molecule (a);

• n and m, which may be identical or different, are equal to 0 or 1;

• $R_a$, which may be identical or different, represent a hydrogen atom or a $C_1$-$C_4$ alkyl radical;

• $R_b$, which may be identical or different, represents a hydrogen atom, an acetyl group ($CH_3$-C(O)-); one of the two radicals $R_b$ representing a hydrogen atom, or alternatively two radicals $R_b$ together representing a phenylmethylenyl group phenyl-C(H)=, said phenyl radical being optionally substituted with at least one hydroxyl or $C_1$-$C_4$ alkoxy radical;

• $A_1$, $B_1$, $A'_1$ and $B'_1$, which may be identical or different, represent:

◦ a $C_6$ aryl group optionally substituted with at least

- a linear or branched $C_1$-$C_{20}$ alkyl radical;
- a hydroxyl radical;
- an alkoxy radical -O-$R_1$ in which $R_1$ represents a $C_1$-$C_4$ alkyl radical optionally substituted with a hydroxyl group, a group -Si($R_2$)(OSi($R_3$)$_3$)$_3$ in which $R_2$ and $R_3$, which may be identical or different, represent a $C_1$-$C_4$ alkyl radical, preferably methyl;
- an ester group -O-C(O)-$R_4$ in which $R_4$ represents a phenyl radical;
- an ammonium group -$N^+$($R_5$)$_3$ in which $R_5$, which may be identical or different, represent a $C_1$-$C_4$ alkyl radical optionally bearing at least one hydroxyl group;
- a halogen atom, preferably chlorine;

• optionally fused to an unsaturated or aromatic 5- or 6-membered heterocycle, comprising at least one heteroatom, preferably oxygen;

◦ a pyridyl or pyridinium group; the quaternized nitrogen atom being substituted with a $C_1$-$C_4$ alkyl radical optionally bearing a hydroxyl group;

◦ a $C_1$-$C_4$ alkyl group;

◦ the groups A and B possibly forming together a 5- or 6-membered ring or heterocycle optionally comprising a heteroatom, preferably oxygen; said ring or heterocycle being optionally fused to a phenyl radical optionally substituted with at least one acetyl ($CH_3$-C(O)-), ester -C(O)-O-$R_6$ or -O-C(O)-$R_6$ group with $R_6$ representing a $C_1$-$C_4$ alkyl radical;

• $A_2$ and $B_2$, which may be identical or different, represent:

◦ a linear or branched $C_1$-$C_{10}$ alkyl radical, possibly bearing one or more unsaturations, optionally substituted with a group $-SiR_3$, the radicals R, which may be identical or different, representing a $C_1$-$C_4$ alkyl radical;
◦ a $C_3$-$C_6$ alkenyl radical;
◦ a hydrogen atom, an alkali metal or alkaline-earth metal or an ammonium group;
◦ the radicals $A_2$ and $B_2$ possibly together forming a 6-membered heterocycle;
◦ the radicals $R_b$ and $A'_1$, in the case where n is equal to 1, may together form a 6-membered hydrocarbon-based ring, optionally substituted with a $C_1$-$C_2$ alkyl group or a hydroxyl group;

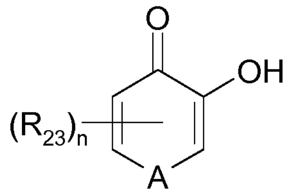

(b)

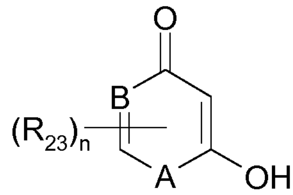

(c)

formula (b) or (c), and also the organic or mineral acid or base salts thereof, and the tautomers thereof, in which:

* n is an integer from 0 to 3; the unsubstituted carbon atoms bear a hydrogen atom;
* A represents $-C(R_{24})(R_{23})-$, $-C(R_{24})_2-$, O, -C(O)-,$-C(R_{24})=C(R_{24})-$;
* B represents $-C(R'_{23})-$, O; $R'_{23}$ represents a hydrogen atom or $R_{23}$;
* $R_{23}$, which may be identical or different, represent a hydroxyl group or a $C_1$-$C_4$ alkyl radical optionally substituted with at least one hydroxyl group;
* two radicals $R_{23}$ borne by two adjacent carbon atoms may together form a fused aromatic ring optionally substituted with at least one $C_1$-$C_4$ alkoxy radical or a hydroxyl group;
* $R_{24}$, which may be identical or different, represent a hydrogen atom or a linear or branched $C_1$-$C_4$ alkyl radical;

it being understood that the compounds of formulae (a) to (c) comprise, where appropriate, a cosmetically acceptable anionic counterion $An^-$ or a mixture of anions, ensuring the electrical neutrality of the formulations;

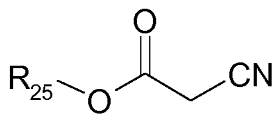

(d)

in which compound of formula (d) $R_{25}$ represents a linear or branched $C_1$-$C_6$ alkyl radical optionally substituted with a group $-SO_3^-M^+$ with $M^+$ representing a cationic counterion such as ammonium, alkali metal or alkaline-earth metal, preferably $Na^+$, $-C(NHR_{26})=NR'_{26}$ in which $R_{26}$ and $R'_{26}$, which may be identical or different, represent a $C_4$-$C_8$ cyclic alkyl radical;

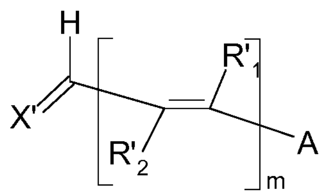

(i')

the optical or geometrical isomers thereof, the organic or mineral acid salts thereof, the solvates thereof; in which compound of formula (i') x' represents an oxygen or sulfur atom, preferably oxygen, $R'_1$ and $R'_2$ represent a hydrogen atom or a $(C_1$-$C_4)$ alkyl group, A represents an aryl group optionally substituted with the same groups as defined for (i) previously, such as phenyl, m is an integer between 0 and 2 inclusive, preferably between 0 and 1 such as 1;

preferentially, the (thio)carbonyl compound(s) are chosen from (i), and more particularly (i').

**6.** Dyeing process according to any one of Claims 3 to 5, in which the amine(s) iii) and/or the (thio)carbonyl compound(s) iv) are in composition (A), or the amine(s) iii) as defined in Claim 3 or 4 are in another composition (C), and the (thio)carbonyl compound(s) iv) as defined in the preceding claim are in a composition (D), or alternatively the amine(s) iii) and the (thio)carbonyl compound(s) iv) are together in the same composition (E);

- the compositions (A), (B), (C), (D) and (E) possibly being applied simultaneously, or
- sequentially by first applying composition (A), optionally followed by (C), (D) and finally by applying (B); preferably the compositions are applied sequentially.

**7.** Dyeing process according to the preceding claim, in which the pH of composition (A), (C), (D) and/or (E) is between 7 and 9.5 inclusive, in particular composition (A) has a pH of between 7 and 9.5 inclusive and preferentially the pH of (A) is neutral.

**8.** Dyeing process according to any one of the preceding claims, in which the chemical oxidizing agent(s) of composition (B) are chosen from hydrogen peroxide, urea peroxide, alkali metal bromates or ferricyanides, peroxygenated salts, for instance alkali metal or alkaline-earth metal persulfates, perborates and percarbonates, such as sodium, potassium or magnesium, transition metal salts chosen from zinc, copper, manganese and iron salts, these salts possibly being of organic or mineral nature, oxidizing agents of enzymatic type such as laccases, peroxidases and 2-electron oxidoreductases such as uricase, where appropriate in the presence of the respective donor or cofactor thereof; preferably, the chemical oxidizing agent is hydrogen peroxide; advantageously, composition (B) comprises aqueous hydrogen peroxide solution, the titer of which may be from 1 to 40 volumes and more preferentially from 5 to 40 volumes.

**9.** Dyeing process according to any one of the preceding claims, which uses the following steps: either:

1) applying to the keratin fibres composition (A) comprising at least one precursor of formula (I) as defined in Claim 1 or 2 with a waiting time of between 10 minutes and 1 hour inclusive at a temperature of between 20 and 50°C inclusive; followed by
2) applying to said fibres a composition (C) comprising at least one amine of formula (V) or of formulae (Va) to (Vi') as defined in Claims 3 and 4, said composition (C) having a pH of between 7 and 9.5, with a waiting time of between 30 minutes and 1 hour inclusive at a temperature of between 20 and 50°C inclusive, followed by
3) revelation of the colour by applying to said fibres composition (B) as defined in Claim 1 or 8; or:

1) applying to the keratin fibres composition (A) comprising:

- at least one precursor of formula (I) as defined in Claim 1 or 2, and
- at least one amine of formula (V) or of formulae (Va) to (Vi') as defined in Claim 3 or 4, said composition (A) having a pH of between 7 and 9.5, with a waiting time of between 10 minutes and 1 hour inclusive at a temperature of between 20 and 50°C inclusive, followed by

2) applying to said fibres a composition (D) comprising at least one (thio)carbonyl compound of formulae (i), (i'), (ii), (a), (b), (c) and (d) as defined in Claim 5, said composition (D) having a pH of between 7 and 9.5, with a waiting time of between 30 minutes and 1 hour inclusive at a temperature of between 20 and 50°C inclusive, followed by
3) revealing the colour by applying to said fibres composition (B) as defined in Claim 1 or 8; or:

1) applying to the keratin fibres composition (A) comprising at least one precursor of formula (I) or (I') as defined in Claim 1 or 2 having a pH of between 7 and 9.5, with a waiting time of between 10 minutes and 1 hour inclusive at a temperature of between 20 and 50°C inclusive; followed by
2) applying to said fibres a composition (C) comprising at least one amine of formula (V) or of formulae (Va) to (Vi') as defined in Claims 3 and 4, said composition (C) having a pH of between 7 and 9.5, with a waiting time of between 30 minutes and 1 hour inclusive at a temperature of between 20 and 50°C inclusive, followed by
3) applying to said fibres a composition (D) comprising at least one (thio)carbonyl compound of formulae (i), (i'), (ii), (a), (b), (c) and (d) as defined in Claim 5, said composition (D) having a pH of between 7 and 9.5, with a waiting time of between 30 minutes and 1 hour inclusive at a temperature of between

20 and 50°C inclusive, followed by
4) revealing the colour by applying to said fibres composition (B) as defined in Claim 1 or 8; or:

1) applying to the keratin fibres composition (A) comprising at least one precursor of formula (I) as defined in Claim 1 or 2 having a pH of between 7 and 9.5, with a waiting time of between 10 minutes and 1 hour inclusive at a temperature of between 20 and 50°C inclusive; followed by
2) applying to said fibres a composition (E) comprising:

- at least one amine of formula (V) or of formulae (Va) to (Vi') as defined in Claims 3 and 4;
- at least one (thio)carbonyl compound of formulae (i), (i'), (ii), (a), (c) and (d) as defined in Claim 5, said composition (E) having a pH of between 7 and 9.5, with a waiting time of between 30 minutes and 1 hour inclusive at a temperature of between 20 and 50°C inclusive, followed by

3) revealing the colour by applying to said fibres composition (B) as defined in Claim 1 or 8; or:

1) applying to the keratin fibres composition (A) comprising:

- at least one precursor of formula (I) as defined in Claim 5;
- at least one amine of formula (V) or of formulae (Va) to (Vi') as defined in Claims 3 and 4; and
- at least one (thio)carbonyl compound of formulae (i), (i'), (ii), (a), (c) and (d) as defined in Claim 5;

said composition (A) having a pH of between 7 and 9.5, with a waiting time of between 10 minutes and 1 hour inclusive at a temperature of between 20 and 50°C inclusive, followed by
2) revealing the colour by applying to said fibres composition (B) as defined in Claim 1 or 8.

**10.** Composition comprising:

- one or more compounds of formula (I) as defined in either of Claims 1 and 2;
- optionally one or more amines of formula (V) or of formulae (Va) to (Vi') as defined in Claims 3 and 4;
- optionally one or more amine polymers;
- optionally one or more (thio)carbonyl compounds of formula (i), (i'), (ii), (a), (b), (c) and (d) as defined in Claim 5; and
- optionally one or more chemical oxidizing agents as defined in Claim 1 or 8.

**11.** Compound of formula (I) according to either of Claims 1 and 2.

**12.** Process for preparing compounds of formula (I) obtained by reaction between:

- at least one non-glycosyl iridoid of formula (II) or (IV) as defined below; more particularly, the non-glycosyl iridoid is of formula (II) as defined below;
- at least one nucleophilic compound chosen from the amines of formula (V) or of formulae (Va) to (Vi') as defined in Claims 3 and 4; and
- at least one electrophilic compound chosen from the (thio)carbonyl compounds of formulae (i), (i'), (ii), (a), (b), (c) and (d) as defined in Claim 5:

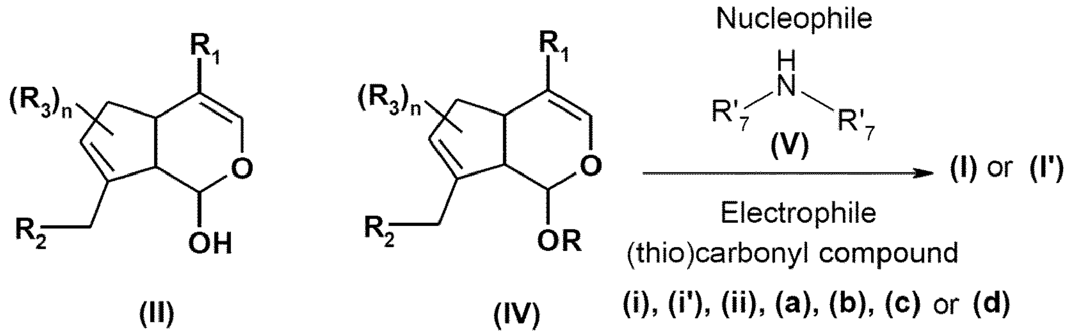

(II) (IV)

with formula (II) in which:

- $R_1$ represents a hydrogen atom, a methyl radical, a hydroxymethyl radical, an aldehyde group; a group-C(O)-O-$R_4$ in which $R_4$ represents a hydrogen atom or a linear or branched ($C_1$-$C_6$) alkyl, preferably $C_1$-$C_2$ alkyl radical; or a group -$CH_2$-glucose; preferably, $R_1$ represents a hydrogen atom, an aldehyde group; a group -C(O)-O-$R_4$ in which $R_4$ represents a hydrogen atom or a linear or branched ($C_1$-$C_6$) alkyl, preferably $C_1$-$C_2$ alkyl radical; preferably a group -C(O)-O-$R_4$;
- $R_2$ represents a hydrogen atom, a hydroxyl radical, a glucose radical; preferably, $R_2$ represents a hydrogen atom, a hydroxyl radical, a glucose radical; preferably a hydroxyl radical;
- $R_3$, which may be identical or different, represent a hydrogen atom, a hydroxyl radical, a ($C_1$-$C_4$)alkyloxy radical; the number of hydroxyl groups not being greater than 2;
- n is an integer between 1 and 5;

or a plant extract comprising said compound of formula (II); and
in which formula (IV):

- $R_1$ represents a hydroxymethyl radical, a group-C(O)-O-$R_4$ in which $R_4$ represents a hydrogen atom or a $C_1$-$C_2$ alkyl radical; a sugar radical;
- $R_2$ represents a hydrogen atom, a hydroxyl radical or a sugar radical;
- $R_3$, which may be identical or different, represent a hydrogen atom, a hydroxyl radical, a ($C_1$-$C_4$)alkyloxy radical; the number of hydroxyl groups not being greater than 2;
- R represents a sugar radical;
- n is an integer between 1 and 5;
- the sugar radical is a derivative obtained from an aldose or an aldose derivative;

or a plant extract comprising said compound of formula (IV) .

**13.** Process for preparing a compound of formula (I) according to the preceding claim, in which the starting compound (II) or (IV) as defined in the preceding claim is dissolved preferably buffered at neutral pH, and added thereto are a nucleophilic compound chosen from the amines of formula (V) or of formulae (Va) to (Vi') as defined in Claims 3 and 4, and an electrophilic compound chosen from the (thio)carbonyl compounds of formulae (i), (i'), (ii), (a), (b), (c) and (d) as defined in Claim 5, these ingredients being left at a temperature of between 15°C and 60°C, preferably between room temperature (25°C) and 50°C such as 40°C, for a time between 1 minute and 24 hours, preferentially between 20 minutes and 6 hours, more particularly between 1 hour and 4 hours such as 2 hours; the reaction product may then be purified via standard methods known to those skilled in the art, such as by extraction, into a water-immiscible organic solvent, which is preferably halogenated, such as dichloromethane, or ethyl acetate, optionally followed by preparative chromatography such as on a column of silica with an eluent of halogenated organic solvent type such as dichloromethane, or ethyl acetate optionally mixed with a polar protic solvent such as ethanol or methanol.

**14.** Multi-compartment device comprising:

- in a first compartment, at least one compound of formula (I) as defined in either of Claims 1 and 2; and
- in a second compartment, at least one chemical oxidizing agent as defined in Claim 1 or 8;

it being understood that the compounds of the first compartment are preferentially in powder form, particularly anhydrous.

**15.** Multi-compartment device as defined previously, also comprising:

- in another compartment, at least one nucleophilic compound chosen from the amines of formula (V) or of formulae (Va) to (Vi') as defined in Claims 3 and 4; and
- in another compartment, at least one electrophilic compound chosen from the (thio)carbonyl compounds of formulae (i), (i'), (ii), (a), (b), (c) and (d) as defined in Claim 5; and

it being understood that the amines and the (thio)carbonyl compounds are preferentially in powder form, preferably anhydrous.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 440494 A
- WO 2005105020 A
- FR 2842200
- FR 2854161
- US 4444928 A


### Littérature non-brevet citée dans la description


- *Tetrahedron Letters,* 1969, 2347-2350
- **E. FREDON et al.** Hydrophobic films from maize bran hemicelluloses. *Carbohydrate Polymers,* 2002, vol. 49, 1-12
- **R. E. WING ; J. L. WILLET.** water soluble oxidized starches by peroxide reaction extrusion. *Industril Crops and Products,* 1997, vol. 75, 45-52